(19) **Europäisches Patentamt / European Patent Office / Office européen des brevets**

(11) **EP 2 664 612 B1**

(12) **EUROPEAN PATENT SPECIFICATION**

(45) Date of publication and mention of the grant of the patent:
**26.12.2018 Bulletin 2018/52**

(51) Int Cl.:
**C07C 253/24** *(2006.01)*      **B01J 23/88** *(2006.01)*
**C07C 255/08** *(2006.01)*      **C07B 61/00** *(2006.01)*

(21) Application number: **12734459.6**

(22) Date of filing: **13.01.2012**

(86) International application number:
**PCT/JP2012/050561**

(87) International publication number:
**WO 2012/096367 (19.07.2012 Gazette 2012/29)**

(54) **METHOD FOR PRODUCING UNSATURATED NITRILE**

VERFAHREN ZUR HERSTELLUNG VON UNGESÄTTIGTEN NITRILEN

PROCÉDÉ POUR PRODUIRE DU NITRILE INSATURÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.01.2011   JP 2011005048**
**01.02.2011   JP 2011020017**
**23.02.2011   JP 2011037471**

(43) Date of publication of application:
**20.11.2013   Bulletin 2013/47**

(73) Proprietor: **ASAHI KASEI KABUSHIKI KAISHA Tokyo 101-8101 (JP)**

(72) Inventors:
 • **TATENO, Eri**
   **Tokyo 101-8101 (JP)**
 • **TAMURA, Sho**
   **Tokyo 101-8101 (JP)**
 • **KATO, Takaaki**
   **Tokyo 101-8101 (JP)**
 • **SHOJI, Sadao**
   **Tokyo 101-8101 (JP)**

(74) Representative: **dompatent von Kreisler Selting Werner -
Partnerschaft von Patent- und Rechtsanwälten mbB
Deichmannhaus am Dom
Bahnhofsvorplatz 1
50667 Köln (DE)**

(56) References cited:
   **JP-A- 9 208 550       JP-A- 2003 170 044
   JP-A- 2007 216 081   US-A1- 2006 235 238**

**Description**

BACKGROUND OF THE INVENTION

Field of the Invention

The present invention relates to a method for producing acrylonitrile by propane ammoxidation.

Description of the Related Art

Conventionally, a method for subjecting propylene to a vapor-phase catalytic ammoxidation to produce a corresponding unsaturated nitrile has been well known. Recently, attention has been directed to a method for subjecting propane instead of propylene to a vapor-phase catalytic ammoxidation to produce a corresponding unsaturated nitrile. Various proposals have been made for the catalyst used during that process.

Many composite metal oxides obtained by mixing a plurality of metal salts and calcining the resultant mixture are used as an ammoxidation reaction catalyst. The type and composition of the metals included in the catalyst are optimized based on catalytic activity and target compound selectivity. However, it is known that during the reaction the Mo and Te in the catalyst escape, so that the composition can deviate from the designed values. As a measure to prevent this, Patent Literature 1 describes a method in which, when performing a propane or an isobutane vapor-phase catalytic oxidation reaction or vapor-phase catalytic ammoxidation reaction in a fluidized bed reactor using an oxide catalyst containing at least Mo, a powder of a molybdenum compound is added to the catalyst dense layer in the reactor during the reaction. The utilization rate of the added Mo is confirmed by extracting the catalyst before and after the addition, and determining the Mo content in the catalyst by XRF, for example.

US 2006/0235238 relates to the provision of a catalyst which can maintain the yield of the desired product over a long reaction time which is accomplished by adjusting the reduction ratio of the catalyst of the specific range controlled during the preparation of the catalyst. The described concept is directed to preventing the change of the performance of the catalyst and it is taught that the yield, conversion rate or the like can be used as an index when evaluating the reaction results of an industrial catalyst.

Citation List

Patent Literature

Patent Literature 1: Japanese Patent Laid-Open No. 2007-308423

SUMMARY OF THE INVENTION

Technical Problem

As described in Patent Literature 1, when reaction results deteriorate during an ammoxidation reaction, there are certainly some cases in which the reaction results recover due to the addition of a molybdenum compound into the reactor. However, based on research by the present inventors, it was learned that even when a molybdenum compound is periodically or intermittently added, in some cases the reaction results do not improve. Solution to Problem

Concerning the reason for this, the present inventors performed diligent research focusing on the possibility that some other factor than the molybdenum content in the catalyst was greatly influencing the reaction results. Consequently, the present inventors discovered that unsaturated nitrile yield could be maintained by measuring changes in specific physical properties of the catalyst, and appropriately adjusting the reaction conditions based on those measurement results.

Specifically, the present invention is as follows:

[1] A method for producing acrylonitrile by a propane ammoxidation reaction, using a composite oxide catalyst represented by the following formula:

$$Mo_1V_aNb_bA_cX_dZ_eO_n$$

wherein component A represents Sb; component X represents at least one element selected from the group consisting of W, Bi and Mn; component Z represents at least one element selected from the group consisting of La, Ce, Pr, Yb, Y, Sc, Sr, and Ba; a, b, c, d, e, and n each represent an atomic ratio of the corresponding element per Mo atom; a is in the range of $0.01 \leqq a \leqq 1$; b is in the range of $0.01 \leqq b \leqq 1$; c is in the range of $0.01 \leqq c \leqq 1$; d is in the range of $0 \leqq d \leqq 1$; e is in the range of $0 \leqq e \leqq 1$; and n represents the number determined by a valence of component elements,
said method comprising:

(1) a step of extracting a part of the catalyst from the reactor in a non-steady state of the ammoxidation reaction, wherein a steady-state refers to a state in which, when no change has been made to the temperature of the catalyst layer in the reactor or the composition of the raw material gas supplied to the reactor for 1 or more days, temperature fluctuations of the catalyst layer in the reactor over 1 day are no greater than 10 °C;
(2) a step of measuring at least one catalyst physical property value selected from the group consisting of the normalized UV value of the catalyst and the reduction ratio of the catalyst contained in a reactor; and
(3) a step of maintaining or changing a reaction condition based on the measured physical property value so that fluctuation in the physical property value of the catalyst is maintained within ±30% of the physical property value of the catalyst prior to packing into the reactor,
wherein in the step (3) of maintaining or changing the reaction condition comprises performing at least one selected from the group consisting of maintaining or changing of the temperature of the catalyst layer in the reactor, and maintaining or changing the composition of raw material gases supplied to the reactor, and maintaining or changing a contact time between the catalyst and the raw material gases, and
wherein the normalized UV value acts as an index of the catalyst redox state which is determined based on the absorbance at 400 nm, 580 nm, and 700 nm of the absorption and/or reflection spectrum obtained by measuring by a diffuse reflection method using a visible-ultraviolet spectrophotometer and calculated based on the following equation (1):

$$\text{Normalized UV value} = \{(580 \text{ nm absorbance}) - (400 \text{ nm absorbance})\} / \{(700 \text{ nm absorbance}) - (400 \text{ nm absorbance})\} \qquad (1)$$

and
wherein the reduction ratio of the catalyst is represented by the following equation (2):

$$\text{Reduction ratio (\%)} = ((n_0 - n) / n_0 \times 100 \qquad (2)$$

wherein n denotes the number of oxygen atoms satisfying the valence of the constituent elements other than oxygen in the catalyst; and no denotes the number of oxygen atoms required when the constituent elements other than oxygen in the catalyst each have their maximum oxidation number.

[2] The method for producing acrylonitrile according to the above-described [1], wherein in the step of maintaining or changing the reaction condition, so that an oxygen concentration in a production gas at an outlet of the reactor during the reaction when the ammoxidation reaction is in a steady state is at a target concentration set to be between 1.5 and 6.0 vol.%, at least one condition selected from the group consisting of (1) a molar ratio of oxygen to propane in the raw material gases, (2) a temperature of the reactor, and (3) a contact time between the catalyst and the raw material gases is adjusted, and
when adjusting the condition (1) and/or (3), change in the temperature of the reactor is set to be within ±5 °C of the temperature prior to adjusting the condition, and when adjusting the condition (2), change in the temperature of the reactor is set to be within ±5 °C of a target temperature.

[3] The method for producing acrylonitrile according to the above-described [1], wherein in the step of maintaining or changing the reaction condition, so that an outlet ammonia concentration calculated based on a propane concentration in a raw material gases when the ammoxidation reaction is in a steady state is at a target concentration set to be more than 0 vol.% to 18 vol.% or less depending on change in an outlet ammonia amount obtained by measuring the outlet ammonia amount of the reactor, at least one condition selected from the group consisting of (1) a molar ratio of ammonia to propane in the raw material gases, (2) a temperature of the reactor, and (3) a contact time between the catalyst and the raw material gases is adjusted, and
when adjusting the condition (1) and/or (3), change in the temperature of the reactor is set to be within ±5 °C of the temperature prior to adjusting the condition, and when adjusting the condition (2), change in the temperature of the reactor is set to be within ±5 °C of a target temperature.

[4] The method for producing acrylonitrile according to the above-described [2], wherein when the oxygen concentration is higher than the target concentration, the molar ratio of oxygen to propane in the raw material gases is decreased, and when the oxygen concentration is lower than the target concentration, the molar ratio of oxygen to propane in the raw material gases is increased.

[5] The method for producing acrylonitrile according to the above-described [4], wherein the rate of increasing or decreasing the oxygen amount in the raw material gases is 10 % or less based on the oxygen amount included in the raw material gases per minute.

[6] The method for producing acrylonitrile according to the above-described [3], wherein when the outlet ammonia concentration is higher than the target concentration, the molar ratio of ammonia to propane in the raw material gases is decreased, and when the outlet ammonia concentration is lower than the target concentration, the molar ratio of ammonia to propane in the raw material gases is increased.

[7] The method for producing acrylonitrile according to the above-described [6], wherein the rate of increasing or decreasing the ammonia amount in the raw material gases is 15% or less based on the ammonia amount included in the raw material gases per minute.

[8] The method for producing acrylonitrile according to any one of the above-described [2] to [7], wherein the rate of change in the temperature of the reactor is 10°C or less per hour.

[9] The method for producing acrylonitrile according to any one of [2] to [8], wherein the rate of change in the contact time between the composite oxide catalyst and the raw material gases is 1.0 sec or less per hour.

Advantageous Effect of Invention

According to the production method of the present invention, the yield of acrylonitrile can be maintained.

DETAILED DESCRIPTION OF THE PREFERRED EMBODIMENTS

An embodiment for carrying out the present invention (hereinafter, referred to as "present embodiment") will now be described in detail. In the following description, acrylonitrile is referred to as the unsaturated nitrile.

[Method for producing unsaturated nitrile]

As the reaction process in the method for producing the unsaturated nitrile according to the present embodiment, typically, a conventional method can be employed, such as a fixed bed reaction, a fluidized bed reaction, and a moving bed reaction. However, a fluidized bed reaction is preferred, because heat removal in the reactor is easy and the extraction and/or addition of the catalyst and the addition of the catalyst constituent elements are easy.

(1) Catalyst extraction step

In the production method according to the present embodiment, to measure the physical property values of the catalyst, a step of extracting a part of the catalyst from the reactor is included. Extracting a part of the catalyst from the reactor, and measuring the below-described physical property values of the catalyst, can help in grasping changes in the physical property values of the catalyst and in the appropriate reaction conditions due to changes in the reaction apparatus to set the appropriate reaction conditions. If the physical property values of the catalyst can be measured without extracting the catalyst from the reactor, this step does not have to be carried out. The timing and frequency for extracting the catalyst from the reactor are not especially limited. However, it is preferred to extract the catalyst when the ammoxidation reaction starts, during the reaction, and when the reaction finishes, one or more times within 3 hours, one or more times within 30 days, and one or more times within 3 hours, respectively. More preferred is one or more times within 2 hours, one or more times within 15 days, and one or more times within 2 hours. Here, "when the reaction starts" refers to the period from when starting to pack the catalyst into the reactor to when the reaction conditions are set to their steady state, "when the reaction finishes" refers to the period from when the reaction conditions are in their steady state to when the gas flow is stopped and the reactor temperature is decreased, and "during the reaction" refers to the period other than these. However, if the reaction conditions are not stable even during the reaction, the time for changing the reaction conditions and the like are not limited to the above. The frequency for extracting the catalyst can be further increased in consideration of the fluctuation frequency, the method for changing the reaction conditions, and how much the reaction conditions are changed. In this case, it is preferred to extract one or more times within 1 day.

The concept of the ammoxidation reaction in the present embodiment being in a "steady state" refers to a state in which, when no change has been made to the temperature of the catalyst layer in the reactor or the composition of the raw material gas supplied to the reactor for 1 or more days, temperature fluctuations of the catalyst layer in the reactor over 1 day are no greater than 10°C. A "non-steady state" refers to periods excluding a steady state from starting catalyst packing until the reaction is finished. Representative non-steady states include the period after production in a new reactor is started until reaching a steady state, and the period after temporarily stopping production for a regular inspection or repair, for example, until restarting the production and reaching a steady state.

Although the method for measuring the temperature of the catalyst layer in the reactor is not especially limited, it is preferred to obtain the temperature of the catalyst layer in the reactor by measuring at a point indicating an average temperature in the catalyst layer in a state in which the catalyst is uniformly flowing in the reactor interior. The temperature of the catalyst layer in the reactor may be the average of temperatures measured at a plurality of locations, or the temperature of the catalyst layer in the reactor at a representative location indicating the average temperature.

Although the method for extracting the catalyst is not especially limited as long as the catalyst in the reactor can be uniformly extracted, for a fluidized bed reaction, examples that can be used include the following.

(1) Connecting a vessel to a nozzle coming from the reactor, and (a) transferring the catalyst in the reactor to the vessel utilizing a pressure difference by setting the pressure in the vessel to be lower than that in the reactor, or (b) conveying the catalyst into the vessel by introducing a gas for catalyst extraction from outside the reactor and applying a gas flow from the reactor to the vessel.

(2) Attaching a vessel to a lower portion of the reactor, and utilizing gravity to extract the catalyst.

If the pressure in the reactor is equal to or more than atmospheric pressure, the above-described method (a) of utilizing a pressure difference is preferred due to its simplicity. The method of setting the pressure in the vessel to be lower may be an ordinary method. As long as the pressure in the reactor is sufficiently higher than atmospheric pressure, the vessel interior may be maintained in an atmospheric pressure as is, or the pressure in the vessel interior may be reduced by causing gas to flow out by an ejector method.

In all of these methods, so that the physical properties of the extracted catalyst do not change, it is preferred that locations in contact with the catalyst, such as the vessel interior and the insides of the connected pipes, have been sufficiently purged in advance with an inert gas, such as nitrogen. Further, so that impurities or catalyst extracted in the past do not become mixed in the extracted catalyst, it is preferred to purge and clean the vessel interior and the insides of the pipes in advance with a suitable gas, an inert gas and the like.

The location for extracting the catalyst may be a single location or a plurality of locations. Although the extraction location is not especially limited as long as the catalyst in the reactor can be uniformly extracted, for a fluidized bed reaction, it is preferred to extract from a location where the flow state of the catalyst in the reactor is good and the catalyst density is thick, as representative physical properties of the catalyst in the reactor can be obtained, and the catalyst can be extracted efficiently in a short time. In the reactor, a raw material gas distribution and a temperature distribution exist, so that how the catalyst changes can depend on the location. In such a case, the catalyst can be extracted from a location thought to have average conditions, or the catalyst can be extracted from a plurality of locations based on the distribution. Further, a cyclone, pipes, shelves and the like are placed in the reactor. If there are locations where the catalyst flow state is thought to be different, in addition to extracting the catalyst from a location where the catalyst is flowing averagely and the flow state is good, the catalyst can also be extracted from a specific location.

The extracted amount of catalyst is not especially limited, as long as the extracted catalyst is in an amount that is sufficient to represent the catalyst in the reactor, is within a range that does not have an influence on the state in the reactor or on the reaction results, and is sufficient for the physical properties to be measured. The extracted amount can be appropriately adjusted based on the size and type of the reactor, the amount of the catalyst, pressure, and the flow state in the reactor, the type of the catalyst physical property to be measured, the number of times measurement is performed, the extraction location, and the number of locations.

The catalyst extraction rate is not especially limited, as long as the extracted catalyst represents the catalyst in the reactor, and does not have an influence on the state in the reactor or on the reaction results. The extraction rate can be appropriately adjusted based on the size and type of the reactor, the amount of the catalyst, pressure, and the flow state in the reactor, and the extraction location. If the extraction rate is too slow, there is a concern for a bias toward extraction of small catalyst particles. Consequently, it is preferred to extract at a sufficient rate. Further, if the extraction rate is too fast, this can cause a pressure change in the reactor, which can have an adverse impact on the catalyst flow state or the reaction results. For example, when about 600 kg of catalyst is packed into a fluidized bed reactor that is made from SUS and has an inner diameter of 600 mm, it is preferred that the catalyst extraction rate is 30 to 2000 g/min, and more preferred is 70 to 1000 g/min. The catalyst extraction rate can be adjusted by adjusting the pressure in the extraction vessel, adjusting the opening level of the valve attached to the vessel when starting to introduce the catalyst into the vessel, adjusting how quickly the valve is opened and the like.

Although the catalyst extraction frequency is not especially limited, it is preferred to extract at a frequency that enables the physical properties of the catalyst to be measured while allowing the yield to be stably maintained, and that does not have an influence on the state in the reactor or on the reaction results. The catalyst extraction frequency can be appropriately adjusted based on the size and type of the reactor, the amount of the catalyst, pressure, and the flow state in the reactor, the type of the catalyst physical property to be measured, the number of times measurement is performed, the extraction location, the number of locations, and the state and stage of the reaction. The time from extracting the catalyst until measuring the physical property values of the catalyst and/or starting a pre-treatment that is required for the measurement is not especially limited. Extraction can be started immediately, or started after a predetermined period has elapsed. However, from the perspectives of maintaining a preferred catalyst state by maintaining or changing the reaction conditions based on the measured physical property values, and maintaining the yield of the target product, it is preferred to start as immediately as possible.

(2) Catalyst physical property measurement step

The production method according to the present embodiment includes a step of measuring at least one physical property value selected from the group consisting of a normalized UV value and a reduction ratio of the catalyst contained in the reactor. Performing the above-described step of extracting a part of the catalyst from the reactor tends to enable at least one physical property value selected from the group consisting of a normalized UV value and a reduction ratio of the catalyst to be easily measured. By continuously monitoring these physical property values, changes in the state of the reactor can be known, so that appropriate reaction conditions can be set. The physical property value to be monitored may be appropriately selected based on the reaction type, reaction conditions, catalyst type and the like. Obviously, a plurality of physical properties can be continuously monitored. Further, if there are large changes immediately after reaction start or during the reaction, the number of items to be measured can be increased.

(Normalized UV value)

In an unsaturated nitrile production reaction, the reaction results are influenced by the redox state of the catalyst. Continuously monitoring changes in the redox state of the catalyst during the reaction is important in maintaining the unsaturated nitrile yield over the long term. The catalyst redox state is measured based on an absorption and/or reflection spectrum. For example, if the catalyst includes Mo and V, the valence state of the Mo and V is thought to be reflected in the redox state. As a result of diligent research performed by the present inventors, it was learned that, especially for a catalyst including Mo and V that is used in a propane ammoxidation reaction, the catalyst redox state can be simply and accurately determined based on the absorbance of the catalyst measured using a visible-ultraviolet spectrophotometer.

**More specifically, the normalized UV value is determined using the following equation (1)** based on the absorbance at 400 nm, 580 nm, and 700 nm of the absorption and/or reflection spectrum obtained by measuring by a diffuse reflection method using a visible-ultraviolet spectrophotometer.

$$\text{Normalized UV value} = \{(580\ \text{nm absorbance}) - (400\ \text{nm absorbance})\} / \{(700\ \text{nm absorbance}) - (400\ \text{nm absorbance})\} \quad (1)$$

The normalized UV value acts as an index of the catalyst redox state, because a larger value indicates that the catalyst is reduced and a smaller value indicates that the catalyst is oxidized.

If the catalyst normalized UV value depends on the particle size of the catalyst, the normalized UV value for a specific particle size range can be measured by performing a classifying operation using a sieve so that the particle size distribution of the catalyst extracted from the reactor does not affect the measurement value. This is also the case for the below-described reduction ratio and catalyst constituent element concentration.

(Reduction ratio)

The "reduction ratio" in the present embodiment refers to the amount of element that is oxidized by potassium permanganate among the catalyst constituent elements. The deficient amount of oxygen in the catalyst is thought to be reflected in the reduction ratio. Similar to the absorption and/or reflection spectrum, continuously measuring the reduction ratio of the catalyst is important in maintaining the unsaturated nitrile yield over the long term. The reduction ratio of the catalyst is represented by the following equation (2).

$$\text{Reduction ratio (\%)} = ((n_0 - n) / n_0 \times 100 \qquad (2)$$

(wherein n denotes the number of oxygen atoms satisfying the valence of the constituent elements other than oxygen in the catalyst; and $n_0$ denotes the number of oxygen atoms required when the constituent elements other than oxygen in the catalyst each have their maximum oxidation number).

To determine the reduction ratio, the value of $(n_0 - n)$ in the equation (2) can be obtained by redox titration of a sample with $KMnO_4$. An example of the measurement method will be described below.

About 200 mg of a sample is precisely weighed into a beaker. Further, an aqueous solution of $KMnO_4$ having a known concentration is added in excess. Then, 150 mL of purified water and 2 mL of 1:1 sulfuric acid (i.e., an aqueous solution of sulfuric acid obtained by mixing concentrated sulfuric acid and purified water in a 1/1 volumetric ratio) are added to the mixture. The beaker is then covered with a watch glass, and the sample is oxidized while stirring for 1 hour in a hot bath of 70°C $\pm$2°C. At this point, $KMnO_4$ is present in excess, so that unreacted $KMnO_4$ is present in the solution. Therefore, it is confirmed that the solution has a purple color. After oxidation is finished, the solution is filtered with filter paper, and all of the filtrate is recovered. An aqueous solution of sodium oxalate having a known concentration is added in excess based on the $KMnO_4$ present in the filtrate. The solution is heated to a temperature of 70°C, and stirred. It is confirmed that the solution has become colorless and is transparent, and then 2 mL of 1:1 sulfuric acid is added. Stirring is continued while maintaining the solution temperature at 70°C $\pm$2°C, and the solution is then titrated with an aqueous solution of $KMnO_4$ having a known concentration. The endpoint is taken as the point at which the solution has a faint pale peach color from the $KMnO_4$ that continues for about 30 seconds. The $KMnO_4$ amount consumed in the oxidation of the sample is determined based on the total amount of $KMnO_4$ and the total amount of $Na_2C_2O_4$. From this value, $(n_0 - n)$ is calculated, and based on the calculated result the reduction ratio is determined.

(Catalyst constituent element concentration)

In the production method according to the present embodiment, in addition to measuring the normalized UV value and reduction ratio of the catalyst extracted from the reactor, one of the preferred aspects is also measuring the concentration of the catalyst constituent elements. The catalyst includes elements that tend to escape under the reaction conditions (temperature, pressure, vapor pressure, etc.). Further, a concentration distribution of the catalyst constituent elements exists among the catalyst particles. If the concentration of the catalyst constituent elements changes due to the scattering of only catalyst particles having a specific catalyst constituent element concentration from the reactor, or the mixing in the catalyst of impurities in the reactor or the raw material gases, this can directly adversely impact the reaction results or the reaction results can deteriorate due to changes in the redox state of the catalyst. Therefore, from the perspective of maintaining the catalyst redox state in a preferred state, it is preferred to also continuously monitor changes in the concentration of the catalyst constituent elements during the reaction.

The method for measuring the concentration of the catalyst constituent elements is not especially limited. An ordinary method for measuring metal concentrations can be employed, such as X-ray fluorescence analysis (XRF), X-ray photoelectron spectroscopy (XPS), ICP emission spectrometry, atomic absorption spectrophotometry, and CHN analysis. When measuring the concentrations of the metals in a solid particle state catalyst, from perspectives such as measurement simplicity and quantitative accuracy, it is preferred to use XRF. When analyzing a trace amount of a metal, the catalyst can be dissolved in a suitable solution, and the concentration can be determined based on ICP or atomic absorption using that solution. Moreover, when trying to determine the concentrations of carbon, hydrogen, and nitrogen, CHN analysis can be preferably used.

Due to tiny differences, such as in the scale, type, or structure of the reaction apparatus, the appropriate reaction conditions up to supplying the ammoxidation reaction raw material gases, as well as during the subsequent reaction, can change. This can prevent the desired reaction results from being obtained because the reactor cannot be operated under the appropriate conditions when the reactor is scaled-up or remodeled. In contrast, in the production method according to the present embodiment, by measuring changes in the physical properties of the catalyst per se, the proper reaction conditions can be set without being influenced by the scale, type, or structure of the reactor.

(3) Reaction condition adjustment step

The production method according to the present embodiment includes a step of maintaining or changing the reaction conditions based on the above-described measured physical property values. In the present step, the reaction conditions are maintained or changed based on the measurement results of the catalyst physical property values. In the present embodiment, "maintaining or changing the reaction conditions based on the measured physical property values" means maintaining or changing the reaction conditions so that the physical properties of the catalyst are optimized within a preferred range of values. If the measured physical property values are within the preferred range, the reaction conditions are maintained, while if the measured physical property values are not within the preferred range, the reaction conditions are changed so that the physical property values are changed to be in the preferred range. To change the physical properties of the catalyst to preferred values by maintaining or changing the reaction conditions, before starting the reaction, it is preferred to grasp the relationship between changing the reaction conditions and the changes in the catalyst physical properties caused by such changes. More specifically, if the catalyst is excessively reduced, it is preferred to determine a so-called calibration curve between the catalyst physical properties and the reaction conditions that, for example, will return the catalyst to its original reduction ratio by determining how oxidative the reaction conditions should be set to be. Obviously, this calibration curve can also be determined by experimentation.

It is preferred to optimize the catalyst physical properties before starting the reaction, and to adjust the reaction conditions so that the catalyst physical properties are in the preferred range before, during, and after the reaction. According to the invention the reaction conditions are maintained or changed so that fluctuation of the catalyst physical property values is within $\pm 30\%$ of the physical property values of the catalyst prior to packing into the reactor, preferably $\pm 20\%$, and more preferably $\pm 10\%$. If the catalyst physical property values deviate by $\pm 30\%$ or more from the values properly adjusted prior to packing, it is difficult to subsequently return the physical property values to within the appropriate range even by trying to adjust these values by changing the temperature or atmosphere in the reactor, so that catalyst performance may deteriorate. Although the reason for the catalyst physical properties irreversibly changing is not clear, one possible reason is that there are limitations on the reactor conditions. If the catalyst redox state changes to the reduction side by 30% or more, for example (i) the oxygen concentration in the reactor may increase up to or beyond the explosion limit even when trying to increase the oxygen concentration in the raw material gases to adjust the redox state, which makes it difficult to expose to a sufficiently oxidative atmosphere. Conversely, if the catalyst redox state changes to the oxidation side by 30% or more, (ii) since a reduction state with an oxygen concentration of 0% or less cannot be realized in the reactor, it is difficult to sufficiently reduce the catalyst. Another reason is that when the redox level deviates by $\pm 30\%$, the crystals that exhibit catalytic activity are irreversibly degraded, which can prevent the original crystal planes from being rebuilt based on adjustments that change the temperature or atmosphere in the reactor. During the period from the packing of the catalyst to the reaction start in a steady state (Step 1) described below, and during termination of the reaction (Step 3), since the temperature and atmosphere in the reactor greatly fluctuate, generally, fluctuations in the normalized UV value and/or the reduction ratio can be expected. In (Step 1), the temperature of the reactor fluctuates from room temperature to the steady state reaction temperature (in the below-described examples, 20 to 450°C), the ammonia supply rate fluctuates from 0 to the steady state rate (in the below-described examples, 0 to 80 Nm$^3$/hr), and the air supply rate fluctuates from 0 to the steady state rate (in the below-described examples, 0 to 400 Nm$^3$/hr). Due to these fluctuations, the composition ratio of the supply gases, the outlet oxygen concentration (in the below-described examples, 0 to 21 vol.%), and the outlet ammonia concentration greatly fluctuate. Especially, since the normalized UV value and/or the reduction ratio can deviate by $\pm 30\%$ or more from the appropriate values in (Step 1) unless they are consciously managed, it is much more effective to measure the normalized UV value and/or the reduction ratio during a non-steady state, and set the reaction conditions based thereon. During the reaction in a steady state (Step 2) described below, it is preferred to maintain the fluctuation of the physical property value range more narrowly.

The maintenance or change of the reaction conditions is not especially limited, as long as such maintenance or change of the reaction conditions allows the catalyst physical property values to be optimized. Examples of this action include one or more selected from the group consisting of addition of the catalyst in the reactor, removal of the catalyst in the reactor, addition of the catalyst constituent elements into the reactor, change of the temperature of the catalyst layer in the reactor, and change of the composition of the raw material gases supplied to the reactor. The maintenance or change of the reaction conditions may be carried out on one per time, or by combining a plurality of types. Further, the reaction conditions may be temporarily changed and then returned back to the pre-change conditions, or the changed conditions may be maintained as is.

As a preferred method for adjusting the reaction conditions based on fluctuations of the respective physical property values, an example will be specifically described of a propane ammoxidation reaction that uses an oxide catalyst including Mo and V.

(Normalized UV value and reduction ratio)

If the normalized UV value and/or reduction ratio calculated based on the absorption and/or reflection spectrum exceed the preferred range, the catalyst is thought to have been excessively reduced or oxidized. To adjust the catalyst redox level to the preferred range, it is preferred to subject the excessively reduced or oxidized catalyst to an oxidization or a reduction treatment.

If the catalyst has been excessively reduced, the catalyst can be oxidized by (i) increasing the oxygen concentration in the raw material gases supplied to the reactor by an appropriate range, (ii) decreasing the ammonia concentration in these raw material gases by an appropriate range, (iii) increasing the rate at which oxygen is incorporated in the catalyst by increasing the temperature of the catalyst layer in the reactor (hereinafter also referred to as "reaction temperature") by an appropriate range, (iv) decreasing the supply rate of the raw material gases by an appropriate range, (v) adding catalyst to the reactor, and (vi) adding a molybdenum compound to the reactor.

For example, in the propane ammoxidation reaction, if the normalized UV value and/or reduction ratio value have changed by 10% to the reduction side, when (i) increasing the oxygen concentration in the raw material gases supplied to the reactor by an appropriate range, it is preferred to increase the molar ratio of air/propane by 0.3 to 3, and more preferred to increase by 1 to 2. When (iii) increasing the reaction temperature, the reaction rate increases due to the increase in the reaction temperature, so that the amount of oxygen consumed in the reaction also increases. Consequently, the opposite effect of oxidation also occurs, in which the oxygen concentration at the reactor outlet decreases. This effect is determined based on the magnitude of the decrease in the oxygen concentration and the level of increase in the rate at which oxygen is incorporated in the catalyst. Therefore, it is more effective to increase the reaction temperature and increase the oxygen concentration in the supplied raw material gases. Similarly, in the propane ammoxidation reaction, for a change of 10% to the reduction side, it is preferred to increase the temperature by 1 to 20°C, and more preferably by 3 to 10°C. Further, when (iv) decreasing the supply rate of the raw material gases, the amount of propane and ammonia, which are reducing gases included in the raw material gases, decreases, which is thought to allow the catalyst to be oxidized by reducing the load on the catalyst per unit catalyst amount. Similarly, for a change of 10% to the reduction side, it is preferred to decrease the flow rate (WWH $[h^{-1}]$) of propane per catalyst amount by 0.01 to 3, and more preferably by 0.05 to 2. However, this is based on the assumption of not fluctuating the molar ratio of ammonia or air to propane. At this time, although the reaction pressure decreases, if the pressure is increased so as to keep it at a fixed level, the contact time increases due to the increase in pressure. In addition, due to the increase in the propane conversion rate, the opposite effect of oxidation also occurs, in which the oxygen concentration at the reactor outlet decreases. Therefore, it is preferred to let the reaction pressure naturally decrease. Here, the "contact time" is the catalyst amount in the reactor divided by the flow rate of all the gases supplied to the reactor. Further, it is thought that reducing the load on the catalyst per unit catalyst amount in the same manner as the method (iv) by (v) adding catalyst to the reactor allows the catalyst to be oxidized. In addition, by (vi) adding a molybdenum compound to the reactor, when the valence of the added molybdenum is higher than that of the molybdenum reduced in the catalyst and/or when the added molybdenum can obtain a higher valence due to being oxidized by the reaction atmosphere more quickly than the catalyst, the catalyst can be oxidized as a result of the added molybdenum and the catalyst coming into contact or forming a complex. This method is also effective when the below-described molybdenum concentration in the catalyst has decreased. Concerning oxidizing the catalyst, from perspectives such as operation simplicity, magnitude of effect, fast action, less impact on unsaturated nitrile productivity and the like, it is especially preferred to employ the above-described (i) and/or (iii) as the method for oxidizing the catalyst to adjust the redox level to the preferred range.

If the catalyst has been excessively oxidized, opposite to when the catalyst has been excessively reduced, the catalyst can be reduced by (i) decreasing the oxygen concentration in the raw material gases supplied to the reactor by an appropriate range, (ii) increasing the ammonia concentration in these raw material gases by an appropriate range, (iii) decreasing the rate at which oxygen is incorporated in the catalyst by decreasing the reaction temperature by an appropriate range, (iv) increasing the supply rate of the raw material gases by an appropriate range, (v) extracting catalyst from the reactor, and (vi) extracting a molybdenum compound from the reactor. Regarding the amount of change, roughly the opposite change to the above-described case of the catalyst being excessively reduced may be made.

When (iii) decreasing the reaction temperature, the reaction rate decreases due to the decrease in the reaction temperature, so that the amount of oxygen consumed in the reaction also decreases. Consequently, the opposite effect of reduction also occurs, in which the oxygen concentration at the reactor outlet increases. This effect is determined based on the magnitude of the increase in the oxygen concentration and the level of decrease in the rate at which oxygen is incorporated in the catalyst. Therefore, it is more effective to decrease the reaction temperature and decrease the oxygen concentration in the supplied raw material gases. When (iv) increasing the supply rate of the raw material gases, the amount of propane and ammonia, which are reducing gases included in the raw material gases, increases, which is thought to allow the catalyst to be reduced by increasing the load on the catalyst per unit catalyst amount. At this time, although the reaction pressure increases, if the pressure is decreased so as to keep it at a fixed level, the contact time decreases due to the decrease in pressure. In addition, due to the decrease in the propane conversion rate, the opposite effect of reduction also occurs, in which the oxygen concentration at the reactor outlet increases. Therefore, it is preferred to let the reaction pressure naturally increase. Further, it is thought that increasing the load on the catalyst per unit catalyst amount in the same manner as the method (iv) by (v) extracting catalyst from the reactor allows the catalyst to be reduced. In addition, by (vi) extracting a molybdenum compound from the reactor, when the valence of the added molybdenum is higher than that of the molybdenum oxidized in the catalyst and/or the added molybdenum can obtain a higher valence due to being oxidized by the reaction atmosphere more quickly than the catalyst, since the catalyst can be oxidized as a result of the added molybdenum and the catalyst coming into contact or forming a complex, it is also thought to be effective to extract the molybdenum compound. However, if the molybdenum compound that has been charged into the reactor adheres to the surface of the catalyst particles or forms a complex with the catalyst, or if the particle size of the molybdenum compound is the same as the catalyst and the like, it tends to be difficult to selectively extract only the molybdenum compound.

Concerning the method for reducing the catalyst to adjust the redox level to the preferred range, from perspectives such as operation simplicity, magnitude of effect, fast action, less impact on unsaturated nitrile productivity and the like, similar to when oxidizing, it is especially preferred to employ the above-described (i) and/or (iii).

Concerning (v) addition or extraction of the catalyst, from the perspectives of improving the fluidity and mixing properties with the catalyst to be added into the reactor, and adjusting the redox state of the catalyst, one of the preferred aspects is selectively adding catalyst having a specific particle size based on the state of the catalyst in the reactor. When the particle size of the extracted catalyst is measured and the particle size has changed from that of the catalyst before packed into the reactor, the particle size of the added catalyst can be made larger or smaller than the average particle size of the pre-packing catalyst. Generally, since a catalyst having smaller particles gradually scatters during the reaction, the average particle size of the catalyst after continuing the reaction for a certain time is often greater than that of the pre-packing catalyst. In such a case, it is preferred to sieve the catalyst to be added, and select those particles having a particle size of 30 to 95% of the particle size of the particles in the reactor. The average particle size of the catalyst in the reactor may be set to be an average particle size that is known to achieve a good fluidity state. For a propane ammoxidation reaction, the average particle size of the catalyst in the reactor is preferably 30 to 100 $\mu$m, and more preferably 40 to 65 $\mu$m. Further, when calcining a composite oxide catalyst that includes Mo, V, and Sb by a known method, since the reduction ratio tends to increase the smaller the particle size is, the particle size of the catalyst to be added can be adjusted based on the redox state of the catalyst in the reactor.

The average particle size of the catalyst can be determined by measuring the particle size distribution based on JIS R 1629-1997 "Particle size distribution measurement methods based on a laser diffraction/scattering method of fine ceramic materials", and averaging the obtained values by volume. For example, the measurement can be performed using the laser diffraction/scattering method particle size distribution measurement apparatus LS230 manufactured by Beckman Coulter, Inc. More specific average particle size measurement can be performed in the following manner based on the manual that comes with the laser diffraction/scattering method particle size distribution measurement apparatus LS230 manufactured by Beckman Coulter, Inc. After carrying out background measurement (run speed 60), 0.2 g of particles is weighed into a screw tube having an appropriate size, and 10 **cm$^3$** of water is added to the tube. Then, the screw tube is capped, and the tube is thoroughly shaken to disperse the particles in the water. Ultrasonic waves from the apparatus are applied at a power of 30 watts, and the screw tube is again thoroughly shaken. Then, the particles dispersed in the water are injected with a dropper into the apparatus main body to an appropriate concentration (concentration 10, PIDS 60). When the concentration display has stabilized, the ultrasonic waves are turned off, and after 10 seconds has passed, measurement is started (measurement time 90 seconds). The value of the median diameter in the measurement results is taken as the average particle size.

(Catalyst constituent element concentration)

If the concentration of a catalyst constituent element exceeds the preferred range, to adjust to the preferred range, the concentration can be returned to an appropriate concentration by adding or extracting to/from the reactor a compound containing the catalyst constituent element that exceeds the preferred range. For example, as the catalyst includes Mo, Mo is known to escape due to the water that is produced by the reaction. Therefore, during the reaction, since the Mo concentration can decrease over time, it is effective to add a molybdenum compound in the reactor. The kind of Mo compound to be added is not especially limited. Examples that can be used include oxides and ammonium salts such as molybdenum dioxide, molybdenum trioxide, molybdenum-containing composite oxide, ammonium heptamolybdate, ammonium dimolybdate, and ammonium polymolybdate. The added Mo compound breaks down in the reactor, and the Mo travels to the catalyst. Although the Mo compound breaks down in the reactor, whereby the Mo is supplied to the catalyst, from the perspective of easily breaking down, the Mo compound is preferably an ammonium salt. Especially preferred is ammonium heptamolybdate. Further, it is also preferred to newly add a Mo-containing catalyst to the reactor. Whether to add a molybdenum compound or a catalyst may be appropriately selected in consideration of the catalyst performance, reaction conditions, economic efficiency and the like. Further, both may be added.

Preferred reaction conditions for maintaining the physical property values of the catalyst in the preferred range will now be specifically illustrated in terms of each reaction step.

(Step 1) From catalyst packing until reaction start in a steady state.

First, the catalyst is packed into the reactor. The catalyst may be directly conveyed from a container such as a catalyst drum to the reactor, or may be temporarily stored in a hopper that is especially for the catalyst and then conveyed to the reactor. The catalyst may be conveyed to the reactor using a gas, although the method is not limited to this. Although the gas used for conveyance may be nitrogen, air, oxygen and the like, from the perspective of availability, economic efficiency, and ease of handleability, it is preferred to use air. Although the temperature of the air used for conveyance is not especially limited, being allowed to take its course, to prevent the catalyst from being oxidized by the air during conveyance, and from the perspective of the heat resistance of the pipes and reactor, it is preferred to set to 20 to 300°C. It is preferred that the catalyst is made to flow around the reactor by also introducing air during catalyst packing from a lower portion of the reactor. The temperature of the air introduced from a lower portion of the reactor is, from the perspective of increasing the temperature of the reactor after the catalyst has been conveyed, and from the perspectives of preventing the catalyst from being oxidized in the air and heat resistance of the reactor, preferably 100 to 650°C. It is preferred to adjust the temperature of the catalyst during conveyance so as to be, from the perspective of increasing the temperature of the reactor after the catalyst has been conveyed, and from the perspective of preventing the catalyst from being oxidized in the air, 50 to 450°C. The time required for catalyst conveyance is, from the same perspectives, if the catalyst temperature is 200°C for example, preferably not greater than 300 hours.

Next, to increase the temperature in the reactor and the temperature of the catalyst layer in the reactor to the reaction temperature of the ammoxidation reaction, heated air is further introduced into the reactor. The temperature of the catalyst layer in the reactor at this stage is, to prevent the catalyst from being oxidized by the heated air, preferably 200 to 450°C, and more preferably 250 to 400°C. The time until the temperature increases to the reaction temperature of the ammoxidation reaction is, from the same perspective, if increasing the temperature of the catalyst layer from 200°C to 350°C for example, preferably not greater than 100 hours. The rate of supplied air is not especially limited, and may be appropriately adjusted based on the size, shape, material, and heat retention properties of the reactor, and the amount of catalyst to be packed. For example, when packing about 600 kg of catalyst into a fluidized bed reactor that is made from carbon steel and has an inner diameter of 600 mm, this rate is preferably 100 to 600 Nm$^3$/hr, and more preferably 150 to 550 Nm$^3$/hr. Even when the size of the reactor and the catalyst amount are different, although the flow rate per catalyst amount can be set to approximately the same preferred range, it is preferred to appropriately adjust based on the catalyst structure and the magnitude of heat loss. The increase of the temperature of the catalyst layer can be carried out in the reactor as described above, or can be carried out in the above-described catalyst hopper.

Next, while supplying a gas containing molecular oxygen and, in the presence of the catalyst, a combustible gas that reacts with the molecular oxygen-containing gas and combusts, the temperature of the catalyst is increased until it reaches the temperature at which the ammoxidation reaction is carried out. In the presence of the catalyst, the combustible gas combusts because of the molecular oxygen-containing gas, thereby producing combustion heat. Consequently, the temperature of the catalyst can be increased by utilizing this combustion heat. The supply amount of the combustible gas is not especially limited, and may be set in consideration of the size and shape of the reactor, the amount of catalyst to be packed, the suppression effect of performance deterioration of the catalyst, and avoidance of the explosive range as a gas composition at the reactor outlet. Specifically, the lower limit of the supply amount can be set in the range of 0.1 vol.% or more based on the combustible gas included in the molecular oxygen-containing gas supplied to the reactor, preferably in the range of 0.5 vol.% or more, and more preferably in the range of 1 vol.% or more. Further, the upper limit of the supply amount can be set in consideration of the above factors, as well as economic disadvantages resulting from an increase in the supply amount. Specifically, the upper limit is preferably in the range of 30 vol.% or less based on the combustible gas included in the molecular oxygen-containing gas supplied to the reactor, and more preferably in the range of 25 vol.% or less. For example, when packing about 600 kg of catalyst into a fluidized bed reactor that is made from carbon steel and has an inner diameter of 600 mm, using air as the molecular oxygen-containing gas, the flow rate used to increase the temperature of the catalyst layer is continued. It is preferred to, using ammonia as the combustible gas, increase the ammonia supply rate to 20 to 80 Nm$^3$/hr in 1 hour at rate of 20 to 80 Nm$^3$/hr. When the temperature of the catalyst layer reaches about 300 to 450°C, it is preferred to decrease the supply rate of air to 150 to 400 Nm$^3$/hr over about 5 minutes to 1 hour. Even when the size of the reactor and the catalyst amount are different, although the flow rate per catalyst amount can be set to approximately the same preferred range, it is preferred to appropriately adjust based on the catalyst structure and the magnitude of heat loss.

It is preferred to start supplying the combustible gas when the temperature of the catalyst layer has reached 300°C or more, and more preferably 300 to 440°C. At this stage, the temperature of the molecular oxygen-containing gas is preferably 100 to 550°C. The combustible gas can be supplied to the reactor by a method such as including it in the molecular oxygen-containing gas supplied to the reactor, or supplying it to the reactor using a separated supply line.

Here, the molecular oxygen-containing gas supplied to the reactor is a mixed gas of molecular oxygen and a gas that is inactive in the combustion reaction. Specific examples include air, a gas having a reduced oxygen concentration obtained by introducing an inert gas, such as nitrogen, argon, water, and carbon dioxide, to air, a gas having an increased oxygen concentration obtained by introducing oxygen to air, and an enriched gas of nitrogen or oxygen obtained by a method such as membrane separation or PSA (pressure swing adsorption). However, among these, it is preferred to use air, which is the most economically advantageous.

After the temperature has been increased to the temperature at which the ammoxidation reaction is carried out, to start the ammoxidation reaction, if the combustible gas is ammonia and/or propane, the ammoxidation reaction can be carried out by adjusting the supply gas to the reactor to the raw material gas composition for the ammoxidation reaction while controlling the supply rate of that gas, and finally adjusting various conditions such as the temperature of the catalyst layer in the reactor, operation pressure, contact time, gas line velocity (LV), and catalyst amount. Further, if the combustible gas is a gas other than ammonia and/or propane, the ammoxidation reaction can be carried out by increasing the supply rate of ammonia and/or propane while gradually reducing the supply rate of that other gas to switch to the raw material gas composition for the ammoxidation reaction, and finally adjusting the various conditions. From the perspectives of operational properties, simplicity, economic efficiency, and sufficient heat produced from combustion, it is preferred to use ammonia as the combustible gas. After the temperature of the catalyst layer has been increased to 300°C or more, it is preferred to prevent the gas supplied to the reactor from becoming only the molecular oxygen-containing gas. However, it is permitted for the gas supplied to the reactor to be temporarily or intermittently only the molecular oxygen-containing gas within a range in which the physical properties of the catalyst can be adjusted to the preferred range, and the performance of the catalyst does not deteriorate.

After the temperature in the reactor has been increased, to start the ammoxidation reaction, the temperature for starting to switch the supply gas to the reactor from the above-described molecular oxygen-containing gas and combustible gas to the ammoxidation reaction raw material gases is not especially limited. However, the temperature for steadily performing the ammoxidation reaction, or a temperature in that vicinity, is preferred. Specifically, it is preferred to perform the switch of the supply gas to the reactor in the range of ±50°C, and more preferably ±30°C, based on the temperature for steadily performing the ammoxidation reaction. The time taken for the switch is, from the perspective of preventing excessive oxidation of the catalyst by the molecular oxygen-containing gas and/or excessive reduction of the catalyst by the combustible gas, if performing the switch at a temperature +10°C based on the temperature for steadily performing the ammoxidation reaction, preferably not more than 100 hours, and more preferably not more than 50 hours. Further, the oxygen concentration at the reactor outlet at this point is, from the perspectives of not exceeding the explosion limit, and adjusting the physical properties of the catalyst to be in the preferred range, preferably 0.1 to 10 vol.%, and more preferably 0.5 to 8 vol.%.

As an example of increasing the temperature, the catalyst may be conveyed to a fluidized bed reactor using 300°C air, and the temperature is increased from room temperature to 340°C by supplying from the bottom of the reactor externally-heated air via a heat exchanger by combustion of a hydrocarbon fuel. In addition, after the temperature of the catalyst layer reaches 340°C, the supply of ammonia is started via a sparger, which is a propane and ammonia supply line located at a bottom portion of the reactor, whereby the temperature of the catalyst is further increased by also utilizing the heat of combustion from the ammonia combustion reaction ($NH_3 + 3/4O_2 \rightarrow 1/2N_2 + 3/2H_2O$). The supply rate of ammonia is gradually increased with the increase in the temperature of the catalyst layer. Ultimately, the concentration of ammonia in the supply gas is increased to 15 to 25 vol.%, and the temperature of the catalyst layer is adjusted to 450°C. Then, the supply of propane is started. While gradually increasing the supply rate, the ammoxidation reaction is finally started under steady conditions by, in addition to adjusting the supply rates of propane, ammonia, and air, adjusting various conditions such as the temperature of the catalyst layer in the reactor, operation pressure, contact time, gas line velocity, and catalyst amount to the predetermined values. The reaction is started by setting the ammonia, propane, and air supply rates so that, when packing about 600 kg of catalyst into a fluidized bed reactor that is made from carbon steel and has an inner diameter of 600 mm for example, the ammonia supply rate is increased to preferably 60 to 80 $Nm^3$/hr over about 30 minutes to 1 hour, then the propane supply is started, increased to 10 to 50 $Nm^3$/hr over 1 to 12 hours, simultaneously the supply rate of air is increased to 200 to 400 $Nm^3$/hr over 1 to 12 hours, and the ammonia supply rate is decreased to 10 to 50 $Nm^3$/hr.

In the present step, since the concentration and temperature of the gases in contact with the catalyst greatly change, the redox state of the catalyst also greatly changes. Therefore, it is preferred that especially the normalized UV value and reduction ratio, which indicate the redox state, are within the appropriate ranges during the below-described steady state by frequently measuring the normalized UV value and reduction ratio, and adjusting the reaction conditions to allow adjustment to the appropriate ranges. In the present embodiment, "frequently" means extracting the catalyst as often as possible at every stage of changing the concentration of the various raw material gases and the temperature of the catalyst layer in the reactor, at a frequency of preferably once every 3 hours or less, and more preferably once or more per hour or less. In the present step, if the physical property values of the extracted catalyst have fluctuated so that they exceed the physical property values of the catalyst prior to packing into the reactor by ±30%, and preferably ±10%, at that point, for example, it is preferred to adjust the catalyst physical property values to within the appropriate ranges by maintaining or changing the reaction conditions, for example by adjusting the air temperature of catalyst conveyance, increasing/decreasing the air, ammonia and/or propane supply rate, and adjusting the supply start temperature/time of air, ammonia/propane.

In a non-steady state, since the environment in the reactor greatly fluctuates, the catalyst redox state may also greatly fluctuate. Therefore, it is especially effective to monitor the physical property values of the catalyst, and control the environment so that these values are maintained in the appropriate ranges. It is also preferred to measure in advance the trend of the normalized UV value and reduction ratio in the present step using a small scale reactor, for example, and not only compare with the catalyst physical property values prior to packing into the reactor, but also determine the appropriate ranges by referring to the pre-measured physical property value trends.

(Step 2) During reaction in a steady state

Examples of the reaction conditions to be maintained or changed in order to adjust the catalyst physical property values to the preferred ranges after the reaction has started in a steady state include the oxygen concentration at the reactor outlet (hereinafter, also referred to as "outlet oxygen concentration"), the ammonia concentration at the reactor outlet (hereinafter, also referred to as "outlet ammonia concentration"), the temperature of the catalyst layer in the reactor, the supply gas rates, the contact time, and the reaction pressure. It is preferred that the oxygen concentration at the reactor outlet is 1.5 to 6 vol.%, and it is more preferred to keep it to 2 to 5 vol.%, and still more preferred to keep it to 2 to 4 vol.%. It is preferred to set a target concentration for the ammonia concentration at the reactor outlet to more than 0 vol.% to 18 vol.% or less. The target concentration for the outlet ammonia concentration can be appropriately set so that the unsaturated nitrile yield and/or selectivity are a desired value based on the composite oxide catalyst subjected to the ammoxidation reaction, the composition of the raw materials, the redox level of the composite oxide catalyst, the reaction process (uniflow or recycle), the reaction mode (fluidized bed or fixed bed) and the like. The target concentration can be set in a range. The below-described parameters are adjusted so that the ammonia concentration in the outlet production gas is within that range. Generally, it is more preferred to set the target concentration for the outlet ammonia concentration to 3 to 16 vol.%, and still more preferred to 5 to 13 vol.%. Maintaining the outlet ammonia concentration to 18 vol.% or less allows the cost of the sulfuric acid used to neutralize the ammonia included in the outlet production gas to be suppressed, and the propane ammoxidation reaction to proceed at an appropriate rate by performing the reaction so that even a little ammonia remains in the outlet production gas (is more than 0 vol.%). Further, although the mechanism is not clear, by maintaining the oxygen concentration and the ammonia concentration at the reactor outlet within the above-described ranges, it is inferred that the catalyst redox level is adjusted during a long-lasting reaction. This does not apply to the case where the reaction conditions are maintained or changed based on the physical property values of the catalyst, so that the catalyst may be subjected to a reduction treatment while temporarily ignoring the above-described target concentrations. Once the catalyst redox state has returned to a preferred range, the outlet concentrations may be set back to the above-described target concentrations.

The reaction process may be a recycle process, in which unreacted raw material gases are recovered and re-supplied to the reactor, or a uniflow process, in which recycling of the raw material gasses is not carried out. However, the preferred composition ratio of the raw material gases may depend on the reaction process.

The composition of the raw material gases supplied to the reactor is not especially limited. For example, when reacting by a uniflow process, since the propane conversion rate needs to be high, it is preferred that the molar ratio of air/propane is 3 to 21, more preferred is 7 to 19, and still more preferred is to adjust to 10 to 17. Further, it is preferred that the molar ratio of ammonia/propane is 0.5 to 1.5, more preferred is 0.65 to 1.3, and still more preferred is to adjust to 0.8 to 1.15.

From perspectives such as obtaining the preferred catalyst performance and catalyst life, and maintaining the physical property values of the catalyst in the preferred ranges, it is preferred that the reaction amount of propane per unit time based on the catalyst amount, which is represented by the following formula (3), is 0.03 to 0.20, and more preferred is 0.04 to 0.18.

```
Propane flow rate (kg/h) / Catalyst amount (kg) × Propane

conversion rate (%) / 100    (3)
```

When maintaining or changing the reaction conditions based on the physical property values of the catalyst, although the adjustment may be carried out so that the physical property values deviate from the above-described preferred numerical ranges, it is preferred to adjust so that the physical property values do not deviate from the explosion range.

When recycling the unreacted propane, since conditions that suppress the propane conversion rate to a low level are preferred in order to obtain a high selectivity from propane to the corresponding unsaturated nitrile, it is preferred that the molar ratio of air/propane is 1 to 16, more preferred is 3 to 13, and still more preferred is to adjust to 5 to 10. However, since the composition ratio of the raw material gases can affect the outlet oxygen concentration, for either reaction process, it is preferred to determine the composition ratio by also considering the setting of the outlet oxygen concentration to be a desired value. It is preferred that the molar ratio of ammonia/propane is 0.2 to 1.3, and more preferred is to adjust to 0.4 to 1.0. However, since the composition ratio of the raw material gases can affect the outlet ammonia concentration, for either reaction process, it is preferred to determine the composition ratio by also considering the setting of the outlet ammonia concentration to be a desired value. When maintaining or changing the reaction conditions based on the physical property values of the catalyst, although the adjustment may be carried out so that the physical property values deviate from the above-described preferred numerical ranges, it is preferred to adjust so that the physical property values do not deviate from the explosion range.

It is preferred that the temperature of the catalyst layer in the reactor during the ammoxidation reaction is 350 to 500°C, and more preferred is 380 to 470°C. Setting the temperature to be 350°C or more tends to enable the propane ammoxidation reaction to proceed at a practical rate. Setting the temperature to be 500°C or less tends to enable degradation of the target product to be suppressed. When maintaining or changing the reaction conditions based on the physical property values of the catalyst, although the adjustment may be carried out by deviating from this range so that the physical property values of the catalyst are in the above-described preferred numerical ranges when the reaction conditions are maintained or changed, in this case, it is preferred to adjust so that the oxygen and/or ammonia concentration at the reactor outlet do not exceed the explosion range due to an increased conversion rate caused by an increase in the temperature of the catalyst layer in the reactor.

The lower the reaction pressure is, the better the unsaturated nitrile selectivity tends to become. A reaction pressure of $5 \times 10^4$ to $5 \times 10^5$ Pa is preferred, and $0.3 \times 10^5$ to $3 \times 10^5$ is more preferred. It is preferred that the contact time between the raw material gases and the composite oxide catalyst is 0.1 to 10 (sec·g/cm$^3$), and more preferred is to adjust to 0.5 to 5 (sec·g/cm$^3$). Examples of method for changing the contact time include (1) increasing/decreasing the amount of the raw material gases, and (2) increasing/decreasing the amount of catalyst contained in the reactor. From the perspective of obtaining a fixed production amount of the unsaturated nitrile, the method (2) is preferred.

Next, the method for analyzing the oxygen concentration in the reactor outlet gas will be described. The reactor "outlet" does not have to be strictly at or near a portion where the production gas flows out from the reactor. It is sufficient if the outlet is within a region where the "outlet oxygen concentration" can be measured in a range in which the ratio of oxygen in the production gas does not change. Therefore, the "outlet oxygen concentration" can be measured in the gas over a region from downstream of the reactor or immediately before flowing out from the reactor until immediately before subjected to the purification operation. For example, when the production gas is rapidly cooled and then purified by extractive distillation by absorption in water, the production gas for measuring the outlet oxygen concentration can be sampled at the pipes between the reactor and the cooling tower provided downstream of the reactor.

In the production method according to the present embodiment, a gas containing an unsaturated nitrile is produced by bringing the raw material gases into contact with the composite oxide catalyst in the reactor, and causing an ammoxidation reaction to proceed. In addition to the unsaturated nitrile, the production gas can also contain unreacted raw materials, and water and byproducts produced by the reaction. The outlet oxygen concentration is analyzed without diluting the production gas. The method for analyzing the oxygen concentration in the production gas at the reactor outlet is not especially limited. Examples include a method in which analysis is performed by providing a sampling line at the reactor outlet, collecting gas from this sampling line in an SUS vessel heated to 180°C, and placing the collected gas into a gas chromatograph (GC-14B, Shimadzu Corporation) that uses the molecular sieve 5A for the filler and argon for the carrier gas, and a method in which analysis is performed by connecting a reactor outlet sampling line directly to the same gas chromatograph, and directly carrying the gas.

In the method for analyzing the ammonia concentration in the reactor outlet gas too, the reactor "outlet" does not have to be strictly at or near a portion where the production gas flows out from the reactor. It is sufficient if the "outlet ammonia concentration" can be measured in a range in which the ratio of ammonia in the production gas does not change. Therefore, the "outlet ammonia concentration" can be measured in the gas over a region from downstream of the reactor or immediately before flowing out from the reactor until immediately before subjected to the purification operation. For example, when the production gas is rapidly cooled and then purified by extractive distillation by absorption in water, the production gas for measuring the outlet ammonia concentration can be sampled at the pipes between the reactor and the cooling tower provided downstream of the reactor.

The outlet oxygen concentration and/or outlet ammonia concentration can be measured continuously, or can be measured intermittently as long as the frequency does not allow the deviation from the target concentration to become too large. When measuring intermittently, it is preferred to grasp the changing rate of the outlet oxygen concentration and/or outlet ammonia concentration, and set the frequency based thereon. Specifically, for a reaction system in which the outlet oxygen concentration and/or outlet ammonia concentration tend to increase and decrease, since the concentration can greatly deviate from the target concentration in a short period of time, it is preferred to measure at intervals of about a few minutes. For a reaction system in which the increase and decrease of the outlet oxygen concentration and/or outlet ammonia concentration is small, the measurement interval may be set at a few hours or more.

Although measurement of the outlet oxygen concentration and the outlet ammonia concentration can be carried out in parallel with measurement of the catalyst physical property values, from the perspective of confirming the explosion range, it is preferred to give priority to the measurement results of the outlet oxygen concentration and outlet ammonia concentration. From the perspective of maintaining a high yield, although it is preferred to determine the results of the catalyst physical property values and the measurement values of the outlet oxygen concentration and/or outlet ammonia concentration as a whole, it is more preferred to give priority to the measurement results of the physical property values of the catalyst itself that is involved with reaction activity and target product yield.

The gas produced by bringing the raw material gases into contact with the composite oxide catalyst in the reactor, and causing the ammoxidation reaction to proceed includes an unsaturated nitrile. However, in addition to the unsaturated nitrile, this gas can also contain unreacted raw materials, and water and byproducts produced by the reaction. The outlet ammonia concentration is analyzed without diluting this production gas. The method for analyzing the ammonia concentration in the outlet production gas in the reactor is not especially limited. For example, a sampling line is provided at the reactor outlet, reaction gas is absorbed in a 1/50 N aqueous nitric acid solution from this sampling line, and titration is performed with 1/50 N caustic soda. At that stage, if the absorbed gas amount is clear, the apparent ammonia concentration can be determined from that amount. If the absorbed gas amount is not clear, the apparent ammonia concentration can be determined based on the correlation between the main component amounts in the solution and the main component concentrations in the gas by, simultaneously with the absorption in a 1/50 aqueous nitric acid solution, collecting separate gas in an SUS vessel heated to 180°C, and placing the collected gas into a gas chromatograph (GC-14B, Shimadzu Corporation). Since the thus-obtained outlet ammonia concentration does not include the concentration of ammonia that reacted with byproduct organic acids, this concentration is referred to as the "apparent outlet ammonia concentration". To obtain the "total outlet ammonia concentration (the true ammonia concentration)" from this, the ammonia amount needs to be corrected by placing the same absorption solution into the gas chromatograph, quantifying the amount of organic acid in the absorption solution, and correcting the ammonia amount that reacted with the organic acid as shown in the following equation (4). In the present embodiment, the "outlet ammonia concentration" is a value obtained by calculating based on propane the true ammonia concentration which corrects for the ammonia amount that reacted with the organic acid, as shown in the following equation (5).

$$\text{True ammonia concentration} = \text{Apparent ammonia}$$
$$\text{concentration} + \text{Concentration of ammonia that reacted}$$
$$\text{with the organic acid} \quad (4)$$

$$\text{Outlet ammonia concentration} = \text{True ammonia}$$
$$\text{concentration} / \text{Propane concentration in raw material gas}$$
$$(5)$$

Although slight differences can occur in the measurement value of the outlet ammonia concentration due to differences in the analysis method, long-term operation can be realized by maintaining or changing the reaction conditions based on the changes in the outlet ammonia concentration. Therefore, such differences in the measurement value due to differences in the analysis method do not have a large effect on control of the reaction. It is sufficient to adjust the reaction conditions based on the changes in the measurement value obtained from the analysis method.

The "reactor outlet" refers to a location that is downstream of the reactor and upstream of the cooling tower. Further, "reactor outlet gas" is a gas that includes the unsaturated nitrile produced by performing a vapor-phase catalytic ammoxidation reaction by supplying the raw material gases to the composite oxide catalyst, and the oxygen/ammonia concentrations are obtained by analyzing without diluting this gas.

To adjust the outlet oxygen concentration when the reaction has become a steady state (the initial concentration) to a target concentration set between 1.5 and 6.0 vol.%, it is preferred to check in advance what level of oxygen concentration will be exhibited when the below-described conditions, such as the composition of the composite oxide catalyst, are adjusted to various ranges. If the initial concentration is in the range of 1.5 to 6.0 vol.% but not at the target concentration, from the perspective of maintaining the catalyst redox level in the desired range, it is preferred to increase/decrease the respective conditions based on the following outlet oxygen concentration control methods (1) to (3) so that outlet oxygen concentration is closer to the target concentration. If the initial concentration is at the target concentration, it is preferred to maintain the operation conditions for the moment, and when a deviation from the target concentration occurs, it is preferred to increase/decrease the respective conditions in the manner described below so that the deviation from the target concentration of the outlet oxygen concentration is within 1 vol.% (within $\pm$ 0.5%).

To adjust the ammonia concentration in the outlet production gas when the reaction has become a steady state (the initial concentration) to a target concentration set to be more than 0% to 18 vol.% or less, it is preferred to check in advance what level of outlet ammonia concentration will be exhibited when the below-described conditions, such as the composition of the composite oxide catalyst, are adjusted to various ranges. If the initial concentration is in the range of more than 0% to 18 vol.% or less but not at the target concentration, from the perspective of maintaining the catalyst redox level in the desired range, it is preferred to increase/decrease the respective conditions based on the following outlet ammonia concentration control methods (1) to (3) so that outlet ammonia concentration is closer to the target concentration. If the initial concentration is at the target concentration, it is preferred to maintain the operation conditions for the moment, and when a deviation from the target concentration occurs, it is preferred to increase/decrease the respective conditions in the manner described below so that the deviation from the target concentration of the outlet ammonia concentration is within 2 vol.% (within $\pm$ 1%).

Next, the method for adjusting the outlet oxygen concentration will be described.

In the production method according to the present embodiment, it is preferred to adjust at least one condition selected from the group consisting of:

(1) Molar ratio of oxygen to propane in the raw material gases,
(2) Temperature of the reactor, and
(3) Contact time between the composite oxide catalyst and the raw material gases,
so that the oxygen concentration in the production gas at the reactor outlet is at a target concentration set between 1.5 to 6.0 vol.%.

For example, if the oxygen concentration in the production gas at the reactor outlet is below the lower limit of the target concentration, the oxygen concentration in the outlet production gas can be increased by 1 vol.% by increasing (1) the molar ratio of oxygen to propane (oxygen/propane) in the raw material gases by 0.15 to 0.4. Further, the oxygen concentration in the outlet production gas can similarly be increased by 1 vol.% by decreasing the temperature of the reactor by 2 to 5°C or (3) the contact time between the composite oxide catalyst and the raw material gases by 0.10 to 0.20 sec. Conversely, if the oxygen concentration in the reactor outlet production gas exceeds the upper limit of the target concentration, the oxygen concentration in the reactor outlet gas can be decreased by 1 vol.% by decreasing (1) oxygen/propane by 0.15 to 0.4, increasing (2) the temperature of the reactor by 2 to 5°C, or increasing (3) the contact time by 0.10 to 0.20 sec. Obviously, it is also effective to get closer to the target oxygen concentration by combining these conditions. Especially, when the deviation from the target concentration is large, a preferred aspect is adjusting a plurality of conditions so as to return the oxygen concentration to the target concentration. However, from the perspective of maintaining the activity of the catalyst and preventing side reactions from proceeding, since it is preferred to adjust the outlet oxygen concentration so that the deviation from the target concentration is within 1 vol.% (within $\pm$0.5%), from the perspective of responding while the deviation from the target concentration is still small, it is preferred to perform the adjustment by increasing/decreasing (1) oxygen/propane since the responsiveness (time taken until the change of the condition is reflected in the outlet oxygen concentration) is comparatively fast.

To increase/decrease the temperature of the reactor, although this also depends on the reactor configuration and the reaction conditions, the supply rate of the cooling medium that passes through the heat removal pipes that are continuously used and/or the heat removal pipes for temperature adjustment can be adjusted at a rate of 0.1 FS/minute or more while monitoring the temperature. The temperature of the reactor can be measured with one or more temperature detectors provided in the catalyst layer in the reactor. If a plurality of temperature detectors are provided, one of those may be selected and used, or two or more detectors may be selected and the average value thereof may be used. The type of the temperature detector to be placed is not especially limited. For example, ordinarily used types, such as a thermocouple or a resistance temperature detector can be used.

The contact time between the composite oxide catalyst and the raw material gases is preferably 0.1 to 10 (sec·g/cm$^3$), and more preferably 0.5 to 5 (sec·g/cm$^3$). Examples of methods for adjusting the contact time include (3-1) increasing/decreasing the amount of the raw material gases, and (3-2) increasing/decreasing the catalyst amount contained in the reactor. However, from the perspective of obtaining a fixed production amount of the unsaturated nitrile, the method (3-2) which is increasing/decreasing the catalyst amount contained in the reactor is preferred. For example, to increase the current contact time by 10%, the catalyst amount contained in the reactor may be increased by 10%. The contact time between the composite oxide catalyst and the raw material gases can be calculated from the amount of flowing raw material gases and the catalyst amount packed into the reactor.

When adjusting the above-described conditions (1) and/or (3), it is preferred that the change in the temperature of the reactor is within ±5°C of the temperature prior to adjusting the conditions. More specifically, when bringing the oxygen concentration in the reactor outlet gas closer to the target concentration by conditions (1) and/or (3), it is preferred to set the rate of increase/decrease in the respective conditions so that the temperature change is within ±5°C or less of the temperature of the reactor prior to the increase/decrease. The propane ammoxidation reaction is a divergent system in which the preferred conversion rate is comparatively low and changes in the temperature of the reactor lead to more temperature change. Therefore, by increasing/decreasing the respective conditions to try to control the oxygen concentration in the reactor outlet gas, the temperature of the reactor can greatly fluctuate. It is thus preferred to bring the oxygen concentration closer to the target concentration while maintaining the change in the temperature of the reactor reactor within ±5°C or less of the temperature prior to adjusting the conditions.

When adjusting the above-described condition (2), it is preferred that the change in the temperature of the reactor is maintained within ±5°C of the target temperature. By maintaining the change in the temperature of the reactor within ±5°C of the target temperature, the reaction amount per unit time of the whole reaction system, and the calorific value greatly increase/decrease, which tends to allow prevention of the reaction temperature from getting out of control. For example, if the temperature of a reactor exhibiting a target concentration of oxygen concentration in the outlet production gas is 445°C, the reaction is allowed to proceed for the moment with that temperature as the target temperature. Generally, the control of the reactor for obtaining the required level of heat removal (e.g., operation of a cooling coil) is grasped before the reaction. Therefore, it is possible to control the temperature of the reactor to about ±0.5°C of the target temperature. Since the activity and the like of the catalyst changes while continuing the reaction for a long duration, even if operation is continued at the same temperature and with the same raw material composition ratio, the oxygen concentration in the outlet production gas can change. If the outlet oxygen concentration increases by 1 vol.% due to improvement in catalytic activity, to use the temperature of the reactor to decrease the outlet oxygen concentration, the temperature is decreased by (for example) 3°C from a target temperature of 445°C. In this case, the reaction is continued with a new target temperature of 442°C while monitoring the outlet oxygen concentration. The reaction is allowed to proceed further, and when a change occurs in the outlet oxygen concentration, a new reaction condition is set based on the reaction conditions at 442°C. Similarly, when decreasing the temperature by 3°C to decrease the outlet oxygen concentration by 1 vol.%, the reaction is continued with a new target temperature of 339°C. Although the above-described example is an aspect in which only the reaction temperature is changed each time a change occurs in the oxygen concentration in the outlet production gas, obviously, the outlet oxygen concentration can be controlled by additionally increasing/decreasing the molar ratio of oxygen/propane, for example. Further, the outlet oxygen concentration can also be controlled by changing the reaction temperature the first time, and changing the molar ratio of oxygen/propane the next time.

Although the outlet oxygen concentration can be increased/decreased by increasing/decreasing the molar ratio of oxygen/propane while measuring the temperature of the reactor, the reactor temperature measurement is not essential. This is because the rate of change in the oxygen/propane molar ratio capable of maintaining the temperature of the reactor in the range of ±5°C can be obtained by plotting a calibration curve by measuring prior to the reaction the changes in the temperature of the reactor caused by fluctuations in the oxygen/propane molar ratio.

To set the oxygen concentration in the reactor outlet production gas to be the target concentration, when adjusting (1) the molar ratio of oxygen to propane in the raw material gases, the rate of increase or decrease in the oxygen amount corresponding to that molar ratio is preferably 10% or less of the oxygen amount included in the raw material gases per minute, and more preferably 5% or less of the oxygen amount included in the raw material gases per minute. Setting the rate of change per minute to be 10% or less of the oxygen amount included in the raw material gases tends to allow the rate of change of the reaction temperature to be prevented from becoming too large. Further, when increasing/decreasing the oxygen amount included in the raw material gases, it is preferred to change to the desired amount by breaking into small stages. For example, when increasing by 60 $Nm^3$/hr over 10 minutes, it is preferred to increase by 1 $Nm^3$/hr per 10 seconds rather than by 6 $Nm^3$/hr per 1 minute.

To set the oxygen concentration in the reactor outlet production gas to be the target concentration, when adjusting (2) the temperature of the reactor, the rate of change in the temperature of the reactor is preferably 10°C or less per hour, and more preferably 5°C or less per hour. The rate of temperature change when increasing/decreasing the temperature of the reactor is set so that, for example, when increasing the temperature of the reactor, the temperature is no more than +5°C from the target temperature, while when decreasing the temperature of the reactor, the temperature is no less than -5°C from the target temperature. Similar to the above-described increase/decrease of the oxygen amount, when increasing or decreasing the temperature of the reactor, it is preferred to change to the desired temperature by breaking into small stages.

To set the oxygen concentration in the reactor outlet production gas to be the target concentration, when adjusting (3) the contact time between the composite oxide catalyst and the raw material gases, the rate of change in the contact time is preferably 1.0 sec or less per hour, and more preferably 0.5 sec or less per hour. To set the rate of change in the contact time to be 1.0 sec or less per hour, the rate of change in the catalyst amount per hour is changed

**EP 2 664 612 B1**

by Xg or less, wherein Xg is represented by the following equation.

$$Xg = \text{Current catalyst amount} - \text{Current catalyst amount} / \text{Current contact time} \times (\text{Current contact time} - 1.0)$$

Similar to the above-described increase or decrease of the oxygen amount and the temperature of the reactor, when increasing or decreasing the contact time, it is preferred to change to the desired time by breaking into small stages.

Next, the method for adjusting the outlet ammonia concentration will be described.

In the production method according to the present embodiment, it is preferred to adjust at least one condition selected from the group consisting of:

(1) Molar ratio of ammonia to propane in the raw material gases,
(2) Temperature of the reactor, and
(3) Contact time between the composite oxide catalyst and the raw material gases,
so that an outlet ammonia concentration calculated based on the propane concentration in the raw material gases is at a target concentration set to be more than 0 vol.% to 18 vol.% or less depending on change in an outlet ammonia amount obtained by measuring the outlet ammonia amount in the reactor.

For example, if the outlet ammonia concentration is below the lower limit of the target concentration, the outlet ammonia concentration can be increased by 1 vol.% by increasing (1) the molar ratio of ammonia to propane (ammonia/propane) in the raw material gases by 0.01 to 0.02. Further, the outlet ammonia concentration can similarly be increased by 1 vol.% by decreasing (2) the temperature of the reactor by 5 to 10°C or (3) the contact time between the composite oxide catalyst and the raw material gases by 0.40 to 0.70 sec. Conversely, if the outlet ammonia concentration exceeds the upper limit of the target concentration, the outlet ammonia concentration can be decreased by 1 vol.% by decreasing (1) the ammonia/propane molar ratio by 0.01 to 0.02, increasing (2) the temperature of the reactor by 5 to 10°C, or increasing (3) the contact time by 0.40 to 0.70 sec. Obviously, it is also effective to get closer to the target ammonia concentration by combining these conditions. Especially, when the deviation from the target concentration is large, a preferred aspect is adjusting a plurality of conditions so as to return the ammonia concentration to the target concentration. However, from the perspective of maintaining the activity of the catalyst and preventing side reactions from proceeding, since it is preferred to adjust the outlet ammonia concentration so that the deviation from the target concentration is within 2 vol.% (within $\pm 1.0\%$), from the perspective of responding while the deviation from the target concentration is still small, it is preferred to perform the adjustment by increasing/decreasing (1) ammonia/propane molar ratio since the responsiveness (time taken until the change of the condition is reflected in the outlet ammonia concentration) is comparatively fast.

To increase/decrease the temperature of the reactor, although this also depends on the reactor configuration and the reaction conditions, the supply rate of the cooling medium that passes through the heat removal pipes that are continuously used and/or the heat removal pipes for temperature adjustment can be adjusted at a rate of 0.1 FS/minute or more while monitoring the temperature. The temperature of the reactor can be measured with one or more temperature detectors provided in the catalyst layer in the reactor. If a plurality of temperature detectors are provided, one of those may be selected and used, or two or more detectors may be selected and the average value thereof may be used. The type of the temperature detector to be placed is not especially limited. For example, ordinarily used types, such as a thermocouple or a resistance temperature detector can be used.

The contact time between the raw material gases and the composite oxide catalyst is preferably 0.1 to 10 (sec·g/cm$^3$), and more preferably 0.5 to 5 (sec·g/cm$^3$). Examples of methods for adjusting the contact time include (3-1) increasing/decreasing the amount of the raw material gases, and (3-2) increasing/decreasing the catalyst amount contained in the reactor. However, from the perspective of obtaining a fixed production amount of the unsaturated nitrile, it is preferred to perform the adjustment by the above-described method (3-2). For example, to increase the current contact time by 10%, the catalyst amount may be increased by 10%.

The contact time between the composite oxide catalyst and the raw material gases can be calculated from the amount of flowing raw material gases and the catalyst amount packed into the reactor. Specifically, the contact time is determined based on the following equation.

$$Contact\ time\ (sec \cdot g/cm^3) = (W\ /\ F) \times 273\ /\ (273\ +\ T)$$

$$\times\ (0.1013\ +\ P)\ /\ 0.1013$$

Here, W, F, T, and P are defined as follows.

W = Packed catalyst amount (g)
F = Raw material gas flow rate (N $cm^3$/sec) in a standard state (0°C, $1.013 \times 10^5$ Pa)
T = Reaction temperature (°C).
P = Reaction pressure (MPa)

When adjusting the above-described conditions (1) and/or (3), it is preferred that the change in the temperature of the reactor is within $\pm 5°C$ of the temperature prior to adjusting the conditions. More specifically, when bringing the ammonia concentration in the reactor outlet gas closer to the target concentration by conditions (1) and/or (3), it is preferred to set the rate of increase/decrease in the respective conditions so that the temperature change is within $\pm 5°C$ or less of the temperature of the reactor prior to the increase/decrease. The propane ammoxidation reaction is a divergent system in which the preferred conversion rate is comparatively low and changes in the temperature of the reactor lead to more temperature change. Therefore, by increasing/decreasing the respective conditions to try to control the ammonia concentration in the reactor outlet gas, the temperature of the reactor can greatly fluctuate. It is thus preferred to bring the ammonia concentration closer to the target concentration while maintaining the change in the temperature of the reactor within $\pm 5°C$ or less of the temperature prior to adjusting the conditions.

When adjusting the above-described condition (2), it is preferred that the change in the temperature of the reactor is maintained within $\pm 5°C$ of the target temperature. By maintaining the change in the temperature of the reactor within $\pm 5°C$ of the target temperature, the reaction amount per unit time of the whole reaction system, and the calorific value greatly increase/decrease, which tends to allow prevention of the reaction temperature from getting out of control. For example, if the temperature of a reactor exhibiting a target concentration of ammonia concentration in the outlet production gas is 445°C, the reaction is allowed to proceed for the moment with that temperature as the target temperature. Generally, the control of the reactor for obtaining the required level of heat removal (e.g., operation of a cooling coil) is grasped before the reaction. Therefore, it is possible to control the temperature of the reactor to about $\pm 0.5°C$ of the target temperature. Since the activity and the like of the catalyst changes while continuing the reaction for a long duration, even if operation is continued at the same temperature and with the same raw material composition ratio, the ammonia concentration in the outlet production gas can change. If the outlet ammonia concentration decreases by 1 vol.% due to improvement in catalytic activity, to use the temperature of the reactor to increase the outlet ammonia concentration, the temperature is decreased by (for example) 5°C from a target temperature of 445°C. In this case, the reaction is continued with a new target temperature of 440°C while monitoring the outlet ammonia concentration. The reaction is allowed to proceed further, and when a change occurs in the outlet ammonia concentration, a new reaction condition is set based on the reaction conditions at 440°C. Similarly, when decreasing the temperature by 5°C to increase the outlet ammonia concentration by 1 vol.%, the reaction is continued with a new target temperature of 435°C. Although the above-described example is an aspect in which only the reaction temperature is changed each time a change occurs in the ammonia concentration in the outlet production gas, obviously, the outlet ammonia concentration can be controlled by additionally increasing/decreasing the molar ratio of ammonia/propane, for example. Further, the outlet ammonia concentration can also be controlled by changing the reaction temperature the first time, and changing the molar ratio of ammonia/propane the next time.

Although the outlet ammonia concentration can be increased/decreased by increasing/decreasing the molar ratio of ammonia/propane while measuring the temperature of the reactor, the reactor temperature measurement is not essential. This is because the rate of change in the ammonia/propane molar ratio capable of maintaining the temperature of the reactor in the range of $\pm 5°C$ can be obtained by plotting a calibration curve by measuring prior to the reaction the changes in the temperature of the reactor caused by fluctuations in the ammonia/propane molar ratio.

To set the ammonia concentration in the reactor outlet production gas to be the target concentration, when adjusting (1) the molar ratio of ammonia to propane in the raw material gases, the rate of increase or decrease in the ammonia amount corresponding to that molar ratio is preferably 15% or less of the ammonia amount included in the raw material gases per minute, more preferably 10% or less of the ammonia amount included in the raw material gases per minute, and still more preferably 5% or less of the ammonia amount included in the raw material gases per minute. Setting the rate of change per minute to be 15% or less of the ammonia amount included in the raw material gases allows the rate of change in the reaction temperature to be prevented from becoming too large. Further, when increasing/decreasing the ammonia amount included in the raw material gases, it is preferred to change to the desired amount by breaking into small stages. For example, when increasing by 60 $Nm^3$/hr over 10 minutes, it is preferred to increase

by 1 Nm$^3$/hr per 10 seconds rather than by 6 Nm$^3$/hr per 1 minute.

To set the ammonia concentration in the reactor outlet production gas to be the target concentration, when adjusting (2) the temperature of the reactor, it is preferred that the change in the temperature of the reactor is, when increasing the temperature of the reactor, no more than +5°C from the target temperature, while when decreasing the temperature of the reactor, is no less than -5°C from the target temperature. When adjusting the ammonia concentration by increasing or decreasing the temperature of the reactor, the rate of change in the temperature of the reactor is preferably 10°C or less per hour, and more preferably 5°C or less per hour. Similar to the above-described increase or decrease of the ammonia amount, when increasing/decreasing the temperature of the reactor, it is preferred to change to the desired temperature by breaking into small stages.

To set the ammonia concentration in the reactor outlet production gas to be the target concentration, when adjusting (3) the contact time between the composite oxide catalyst and the raw material gases, the rate of change in the contact time is preferably 1.0 sec or less per hour, and more preferably 0.5 sec or less per hour.

To set the rate of change in the contact time to be 1.0 sec or less per hour, the rate of change in the catalyst amount per hour is changed by Xg or less, wherein Xg is represented by the following equation.

$$\text{Xg = Current catalyst amount - Current catalyst}$$

$$\text{amount / Current contact time} \times \text{(Current contact time -}$$

$$\text{1.0)}$$

Similar to the increase or decrease of the oxygen amount and the temperature of the reactor, when increasing or decreasing the contact time, it is preferred to change to the desired time by breaking into small stages.

(Step 3) When terminating the reaction

When terminating the ammoxidation reaction, first, the supply of propane and ammonia to the reactor is terminated. The terminating method is not especially limited. The supply rates of both the propane and ammonia may be gradually reduced, or the supply of one of these may be terminated first and then the other terminated. From the perspective of adjusting within the range in which the gases in the reactor do not exceed the explosion limit, and ease of adjustment, it is preferred to first gradually reduce the supply rates of both the propane and ammonia, then gradually reduce the supply rate of only propane, and when the supply of propane has completely terminated, simultaneously increase the supply rate of ammonia in order to avoid the explosion limit.

It is preferred to reduce the supply rates of propane and ammonia to 60 to 90% preferably over 0.1 to 30 hours, and more preferably over 0.5 to 20 hours, then while continuing reducing the supply rate of propane over 2 to 30 hours, more preferably over 3 to 20 hours, increase the ammonia flow rate by 100 to 400%, and preferably 100 to 350%. Then, after the supply of propane is terminated, the catalyst temperature is decreased. It is also preferred to continue supplying ammonia while decreasing the catalyst temperature. Once the catalyst temperature is decreased preferably to 200 to 400°C, and more preferably 250 to 380°C, it is preferred to reduce the supply rate of ammonia over 0.3 to 10 hours, and then also terminated the supply of ammonia. Even in the reaction terminating step, it is preferred to maintain the physical properties of the catalyst, from the perspectives of maintaining the catalyst in a preferred state and re-starting the reaction in a good state.

For example, when packing about 600 kg of catalyst into a fluidized bed reactor that is made from carbon steel and has an inner diameter of 600 mm, it is preferred to increase the ammonia flow rate by from 10 to 50 Nm$^3$/hr to 20 to 200 Nm$^3$/hr while reducing the supply rate of air by 60 to 90% from 200 to 400 Nm$^3$/hr over 0.3 to 10 hours. Simultaneously, it is preferred to terminate the supply of propane over 0.3 to 10 hours from a rate of 10 to 50 Nm$^3$/hr. Then, once the catalyst temperature has been decreased to the above-described preferred range, the supply rate of ammonia is terminated preferably over 0.3 to 7 hours. Subsequently, while continuing the supply of air, the reactor temperature is decreased preferably over 0.5 to 3.6 hours. Even if the size of the reactor and the catalyst amount are different, although the flow rate per catalyst amount can be set to approximately the same preferred range, it is preferred to appropriately adjust based on the catalyst structure and the magnitude of heat loss.

If for some reason the physical property values of the extracted catalyst deviate from their preferred ranges, the above-described preferred reaction conditions can be appropriately changed to maintain or change the reaction conditions in order to return those physical property values to their preferred ranges.

Next, the composite oxide catalyst that is used in the production method according to the present embodiment will be described.

(a) Composite oxide catalyst

From the perspective of the selectivity of the target product and conducting a long-term flow reaction, the composition of the composite oxide is represented by the following formula:

$$Mo_1V_aNb_bA_cX_dZ_eO_n$$

wherein component A represents Te and/or Sb; component X represents at least one element selected from the group consisting of W, Bi, and Mn; component Z represents at least one element selected from the group consisting of La, Ce, Pr, Yb, Y, Sc, Sr, and Ba; a, b, c, d, e, and n each represent an atomic ratio of the corresponding element per Mo atom; a is in the range of $0.01 \leq a \leq 1$; b is in the range of $0.01 \leq b \leq 1$; c is in the range of $0.01 \leq c \leq 1$; d is in the range of $0 \leq d \leq 1$; e is in the range of $0 \leq e \leq 1$; and n represents the number determined by a valence of component elements.

The atomic ratio a and b of V and Nb per Mo atom is preferably 0.1 to 0.4 and 0.02 to 0.2, respectively.

The atomic ratio c of component A per Mo atom is preferably 0.01 to 0.6, and more preferably 0.1 to 0.4. In an ordinary industrial method for producing an unsaturated nitrile, the composite oxide catalyst preferably can endure long-term use at a temperature of not less than 400°C. However, when component A is Te, Te tends to escape during long-term operation. From this perspective, in the industrial method for producing an unsaturated nitrile, component A is preferably Sb. Based on a diligent research of a/c, which is the atomic ratio of V and component A, although the reasons are not entirely clear, it was learned that when Te is used, a/c is preferably 1 to 10, and when Sb is used, a/c is preferably 0.1 to 1. If a/c is in this range, the target product tends to be obtained at a high yield, and catalyst life also tends to lengthen.

The atomic ratio d of component X per Mo atom is in the range of $0 \leq d \leq 1$, and more preferably $0.001 \leq d \leq 0.3$. From the perspective of long-term industrial use, it is preferred that component X is at least one element selected from the group consisting of W, Bi, and Mn. W is especially preferred, because the yield of the target product tends to be the highest.

If component Z is uniformly dispersed within the composite oxide, an improving effect on the yield of the target product can be obtained. As the component Z element, preferable are La, Ce, Pr, and Yb. From the perspective of an effect for improving the yield of the target product, Ce is especially preferred. However, from the perspective of preventing an undesirable reaction by component Z in a slurry as taught by Japanese Patent Laid-Open No. 11-244702, the atomic ratio e of component Z per Mo atom preferably satisfies $0.001 \leq e < 1$, more preferably satisfies $0.001 \leq e < 0.1$, and still more preferably satisfies $0.002 \leq e < 0.01$.

The above-described composite oxide catalyst may be supported by a carrier. The carrier supporting the composite oxide preferably includes silica as a main component. If the composite oxide is supported by a carrier that includes silica as a main component, since it has a high mechanical strength, such a supported composite oxide catalyst is suitable for a vapor-phase catalytic ammoxidation reaction using a fluidized bed reactor. If the carrier includes silica as a main component, the content of silica in the carrier is preferably 20 to 70 mass%, and more preferably 30 to 60 mass%, in terms of $SiO_2$ based on the total mass of the supported oxide formed from the composite oxide and the carrier.

From the perspectives of strength and prevention of powdering, the content of the silica in the carrier is preferably not less than 20 mass% based on the total mass of the supported oxide formed from the composite oxide and the carrier. If the content of the silica in the carrier is less than 20 mass%, safe operation is difficult in industrial use of the composite oxide catalyst. Moreover, since lost composite oxide catalyst needs to be replenished, such a content is also economically undesirable. On the other hand, from the perspective of obtaining a sufficient activity and properly adjusting the necessary catalyst amount, the content of the silica in the carrier is preferably not more than 70 mass% based on the total mass of the supported oxide formed from the composite oxide and the carrier. In particular, for a fluidized bed reaction, if the silica content is not more than 70 mass% by mass, the specific gravity of the composite oxide catalyst is proper, and it is easy to produce a good flow state.

The normalized UV value of the catalyst before packing into the reactor is preferably 0.6 to 1.0, and more preferably 0.65 to 0.9. The reduction ratio of the catalyst before packing into the reactor is preferably 6 to 11%, and more preferably 7 to 10%.

The normalized UV value according to the present embodiment is a value obtained by converting, when the catalyst includes Mo and V, the balance of the Mo and V valences into a number. However, catalysts having other compositions are not limited to this definition. For example, it is preferred to check in advance the characteristic wavelength based on the constituent elements, define a normalized UV value that focuses on that wavelength, and check the trend between yield and reaction conditions. Similarly, for the reduction ratio, since the amount to be dissolved in potassium permanganate changes depending on the constituent elements and the composition, it is preferred to check in advance the preferred numerical values, and check the trend between yield and reaction conditions.

(b) Production of composite oxide catalyst

The composite oxide catalyst a is produced, for example, by carrying out the following three steps:

(1) Step of blending raw materials to obtain a raw-material blend solution;
(2) Step of drying the raw-material blend solution obtained in step (1) to obtain a catalyst precursor; and
(3) Step of calcining the catalyst precursor obtained in step (2) to obtain a composite oxide catalyst.

Here, the "blending" means to dissolve or disperse the raw materials of the catalyst constituent elements in a solvent. The solvent is preferably an aqueous solvent.

Moreover, the "raw material" means a compound containing an constituent element of the composite oxide catalyst. The raw materials are not especially limited. For example, the following compounds can be used.

Examples of preferably used Mo and V raw materials include ammonium heptamolybdate $[(NH_4)_6Mo_7O_{24} \cdot 4H_2O]$ and ammonium metavanadate $[NH_4VO_3]$, respectively.

Examples of Nb raw materials that can be used include niobic acid, an inorganic niobate, and an organic niobate, and niobic acid is especially preferred. Niobic acid is represented by $Nb_2O_5 \cdot nH_2O$, and is also referred to as niobium hydroxide or niobium oxide hydrate. Further, a Nb raw material solution in which a molar ratio of dicarboxylic acid/niobium is 1 to 4 is preferably used. As the dicarboxylic acid in this case, oxalic acid is preferred.

As the Sb raw material, diantimony trioxide $[Sb_2O3]$ is preferred.

As the Te raw material, telluric acid $[H_6TeO_6]$ is preferred.

The raw materials of component X are not especially limited, as long as the materials contain these elements. A compound containing these elements and a solution in which metal of these elements is solubilized in an appropriate reagent can be used. As the compound containing these elements, an ammonium salt, a nitrate, a carboxylate, an ammonium salt of a carboxylic acid, a peroxocarboxylate, an ammonium salt of a peroxocarboxylic acid, a halogenated ammonium salt, a halide, acetyl acetate, and an alkoxide of these elements can usually be used. Preferably, a water-soluble raw material such as a nitrate, and a carboxylate is used.

The raw materials of component Z are not especially limited, as long as the materials contain these elements. A compound containing these elements and a solution in which the -metal of these elements is solubilized in an appropriate reagent can be used. As the compound containing these elements, a nitrate, a carboxylate, an ammonium salt of a carboxylic acid, a peroxocarboxylate, an ammonium salt of a peroxocarboxylic acid, a halogenated ammonium salt, a halide, acetyl acetate, and an alkoxide of these elements can usually be used. Preferably, a water-soluble raw material such as a nitrate, and a carboxylate is used.

The raw materials of the silica contained in the carrier are not especially limited. Silica sol can be used. However, silica powder can be used either partially or entirely as the silica raw material. The silica powder is preferably produced by a high-temperature method. Using silica powder previously dispersed in water facilitates the addition and mixing of the silica powder to a slurry. A dispersing method is not especially limited. The silica powder can be dispersed by using a general homogenizer, homomixer, and supersonic vibrator or the like either singly or in combination.

Although the catalyst precursor calcining atmosphere and temperature have an influence, the reduction ratio of the catalyst can be adjusted by a known calcining method.

In some cases the thus-produced catalyst includes surface bodies that protrude from the particle surface. The surface bodies are formed in a shape that bulges and/or protrudes out from the surface of the composite oxide catalyst. When used in a fluidized bed reactor, it may be difficult for a composite oxide catalyst having surface bodies to exhibit sufficient fluidity. Moreover, the yield of the target product can also be lower compared with a composite oxide catalyst that does not have surface bodies. Therefore, it is preferred to remove surface bodies from the catalyst, so that the surface body content is 2 mass% or less based on the mass of the oxide catalyst. As a method for removing surface bodies from the catalyst, it is preferred to use a method such as bringing the catalyst into contact with an air flow. In such a case, it is preferred to set the air flow length in the direction that the air is flowing to be 10 mm or more, and the average flow rate to 80 m/s or more to 500 m/s or less based on linear velocity at 15°C under 1 atmosphere.

At least a part of the surface bodies has a different crystal structure from the catalyst surface and/or interior, and also a different redox state. Therefore, if the amount of surface bodies remaining on the catalyst particle surface exceeds 2 mass%, the redox state of the catalyst surface and/or interior cannot be monitored, which can prevent adjustment to the proper reaction conditions. Further, although the reason is not clear, the surface bodies tend to undergo oxidation and reduction more easily than the catalyst, which can adversely impact the catalyst performance if the catalyst surface and/or interior is oxidized or reduced while the surface bodies are closely adhered to the catalyst surface as a result of oxidation or reduction.

Preferred production examples of the composite oxide catalyst including steps (1) to (3) will now be described.

(Step 1: Step of blending raw materials to obtain raw-material blend solution)

In this step, the Mo compound, V compound, component A compound, component X compound, component Z compound, and optionally, a component of other raw material are added to water and, then, heated, thereby preparing an aqueous mixed solution (I). At this point, the inside of the vessel may be a nitrogen atmosphere. The Nb compound and a dicarboxylic acid are then heated in water while stirring to prepare a mixed solution (B0). Further, hydrogen peroxide is added to the mixed solution (B0) to prepare an aqueous mixed solution (II). At this point, the $H_2O_2$/Nb (molar ratio) is preferably 0.5 to 20, and more preferably 1 to 10.

Based on the target composition, the aqueous mixed solution (I) and the aqueous mixed solution (II) are appropriately mixed, to obtain an aqueous mixed solution (III). The obtained aqueous mixed solution (III) is aged under an air atmosphere to obtain a slurry.

Aging of the aqueous mixed solution (III) means to leave standstill or stir the aqueous mixed solution (III) for a predetermined time. When the composite oxide catalyst is industrially produced, a spray dryer usually has a rate-limiting treatment speed. After a portion of the aqueous mixed solution (III) is spray-dried, it takes time to complete the spray drying of the whole mixed solution. In the meantime, the aging of the mixed solution which is not spray-dried is continued. Therefore, an aging time includes not only an aging time before spray drying but also a time from the start to finish of the spray drying.

The aging time is preferably 90 minutes or more to no more than 50 hours, and more preferably 90 minutes or more to no more than 6 hours.

The aging temperature is preferably 25°C or more from the perspective of preventing the condensation of the Mo component and the deposition of V. The aging temperature is preferably 65°C or less from the perspectives of preventing excessive hydrolysis of a complex containing Nb and hydrogen peroxide and forming a slurry in a preferable form. Therefore, the aging temperature is preferably 25°C or more to 65°C or less, and more preferably 30°C or more to 60°C or less.

It is preferred that the atmosphere in the vessel during the aging has a sufficient oxygen concentration. If the oxygen is insufficient, it tends to be more difficult for substantial change of the aqueous mixed solution (III) to occur. Accordingly, the vapor-phase oxygen concentration in the vessel is more preferably 1 vol.% or more.

The vapor-phase oxygen concentration can be measured by an ordinary method, for example, using a zirconia type oxygen meter. The place where the vapor-phase oxygen concentration is measured is preferably near an interface between the aqueous mixed solution (III) and the vapor phase. For example, preferably, the vapor-phase oxygen concentration is measured three times at the same point within 1 minute, and the mean value of the three measurement results is used as the vapor-phase oxygen concentration.

Examples of a dilution gas for reducing the vapor-phase oxygen concentration include, but are not especially limited to, nitrogen, helium, argon, carbon dioxide, and steam. Industrially, nitrogen is preferable. As a gas for increasing the vapor-phase oxygen concentration, pure oxygen or air with a high oxygen concentration is preferable.

Some change is considered to occur in a redox state of the components contained in the aqueous mixed solution (III) by the aging. The occurrence of some change is suggested from the occurrence of a change in color, a change in the redox potential and the like of the aqueous mixed solution (III) during the aging. Consequently, a difference in the performance of the obtained composite oxide catalysts that results from the presence or absence of the aging for 90 minutes or more to 50 hours or less in an atmosphere having an oxygen concentration of 1 to 25 vol.% is manifested. Specifically, it is extremely difficult to correctly identify change in the form of the components in the solution during the aging. However, by producing catalysts having a different aging time, and evaluating their performance, it can be inferred that the aging time which was applied to catalysts having a good performance is preferred, and that a slurry having some preferable form was formed in those cases.

It is considered that the redox potential of the aqueous mixed solution (III) is controlled by the potential (600 mV/AgCl) of the aqueous raw-material solution (II), and that the Nb oxalate peroxide and other metal components contained in the aqueous raw-material solution (II) cause some kind of redox reaction to occur, thereby causing a deterioration in the potential over time. The redox potential of the aqueous mixed solution (III) is preferably 450 to 530 mV/AgCl, and more preferably 470 to 510 mV/AgCl.

The oxygen concentration during the aging is preferably 1 vol.% or more from the perspective of preventing an excessive delay in the progress of the redox reaction, which has an influence on some change in the redox state of the components included in the aqueous mixed solution (III), so that the redox state in the slurry stage tends to become overly oxidative. On the other hand, the oxygen concentration during the aging is preferably 25 vol.% or less from the perspective of preventing the redox reaction from excessively progressing so that the slurry tends to become overly reductive. In whichever case, it is necessary to maintain the oxygen concentration in a proper range since vapor-phase oxygen has an influence on the redox state of the slurry. The range of the oxygen concentration is preferably 5 to 23 vol.%, and more preferably 10 to 20 vol.%.

During the aging, moisture may be vaporized to produce condensation. If aging is performed in an open system, the moisture is naturally vaporized. However, unless the aging is performed in an atmosphere with an oxygen concentration of 1 to 25 vol.%, the performance of the catalyst may not improve.

If the composite oxide is supported by a silica carrier, a raw-material blend solution containing silica sol is prepared. The silica sol can appropriately be added thereto. An aqueous dispersion of the silica powder can be used as a portion of the silica sol. An aqueous dispersion of such silica powder can also appropriately be added.

When Sb (antimony) is used as component A, hydrogen peroxide is preferably added to the aqueous mixed solution (I) or a liquid containing components of the aqueous mixed solution (I) during blending. At this point, $H_2O_2/Sb$ (molar ratio) is preferably 0.01 to 5, and more preferably 0.05 to 4. At this point, stirring is preferably continued at 30°C to 70°C for 30 minutes to 2 hours.

(Step 2: Drying step)

The drying step is a step of drying the raw-material blend solution obtained in step (1) to obtain a dry powder. The drying can be carried out by a known method, such as spray drying or evaporation to dryness, for example. Among these, it is preferred to employ spray drying to obtain a dry powder having a microspherical shape. Spraying in the spray drying method can be performed by a centrifugal system, a two-fluid-nozzle system, or a high-pressure nozzle system. Air heated by steam, an electric heater or the like can be used as a heat source for drying. The inlet temperature of the dryer in a spray drying apparatus is preferably 150 to 300°C. The outlet temperature of the dryer is preferably 100 to 160°C.

(Step 3: Calcining Step)

The calcining step is a step of calcining the dry powder obtained in step (2) to obtain a composite oxide catalyst. A rotary kiln can be used as the calcining apparatus. The shape of the calcining device is not especially limited. If the shape of the calcining device is tubular, continuous calcination can be carried out. The shape of a calcining tube is not especially limited. However, the shape of the calcining tube is preferably cylindrical. The heating system is preferably an external heating system. An electric furnace can suitably be used. The size, material or the like of the calcining tube can be appropriately selected depending on a calcining conditions and production amount. The inner diameter of the calcining tube is preferably 70 to 2000 mm, and more preferably 100 to 1200 mm. The length of the calcining tube is preferably 200 to 10000 mm, and more preferably 800 to 8000 mm. If an impact is applied to the calcining device, the thickness of the calcining device is preferably 2 mm or more, and more preferably 4 mm or more from the perspective that the calcining device has a sufficient thickness not to be broken by the impact. The thickness of the calcining device is preferably 100 mm or less, and more preferably 50 mm or less from the perspective that the impact is sufficiently transmitted into the calcining tube. The material of the calcining device is not especially limited as long as the calcining device has sufficient heat resistance and strength not to be broken by the impact. For example, SUS can be suitably used as the material of the calcining device.

A weir plate having a hole in the center through which powder passes is provided vertically to the flow of the powder in the calcining tube, so that the calcining tube can be partitioned into two or more zones. A holding time in the calcining tube is easily ensured by having the weir plate. The number of the weir plates may be one or more. The material of the weir plate is preferably a metal, and a weir plate made of the same material as that of the calcining tube can suitably be used. The height of the weir plate can be adjusted in accordance with the holding time which should be ensured. For example, when powder is supplied at 250 g/hr using a rotary kiln having a calcining tube having an inner diameter of 150 mm and a length of 1150 mm and made of SUS, the height of the weir plate is preferably 5 to 50 mm, more preferably 10 to 40 mm, and still more preferably 13 to 35 mm. The thickness of the weir plate is not especially limited, and is preferably adjusted in accordance with the size of the calcining tube. For example, in the case of a rotary kiln having a calcining tube having an inner diameter of 150 mm and a length of 1150 mm and made of SUS, the thickness of the weir plate is preferably 0.3 mm or more and 30 mm or less, and more preferably 0.5 mm or more and 15 mm or less.

In order to prevent fissuring, cracking or the like of the dry powder and to uniformly calcine the dry powder, it is preferred to rotate the calcining tube. The rotation speed of the calcining tube is preferably 0.1 to 30 rpm, more preferably 0.5 to 20 rpm, and still more preferably 1 to 10 rpm.

For the calcination of the dry powder, preferably, the heating temperature of the dry powder is continuously or intermittently increased to a temperature in the range of 550 to 800°C from a temperature lower than 400°C.

The calcining atmosphere may be an air atmosphere or an air flow. However, at least a portion of the calcination is preferably carried out while an inert gas which substantially does not contain oxygen, such as nitrogen, flows. The supplied amount of the inert gas is 50 N liters or more per 1 kg of the dry powder, preferably 50 to 5000 N liters, and more preferably 50 to 3000 N liters (N liter means a liter measured under normal temperature and pressure conditions, that is, at 0°C under 1 atmosphere). At this point, the flows of inert gas and dry powder may be in the form of a counter flow or a parallel flow. However, counter flow contact is preferable in consideration of the gas components generated from the dry powder and a trace amount of air entering together with the dry powder.

The calcining step can be carried out in a single stage. However, the calcination preferably includes pre-stage calcination performed in the temperature range of 250 to 400°C and main calcination performed in the temperature range of 550 to 800°C. The pre-stage calcination and the main calcination may be continuously carried out. The main calcination may be carried out anew once the pre-stage calcination has been completed. The pre-stage calcination and the main calcination may each be divided into several stages.

The pre-stage calcination is performed, preferably under an inert gas flow at a heating temperature of 250°C to 400°C, and preferably 300°C to 400°C. The pre-stage calcination is preferably held at a constant temperature within the temperature range of 250°C to 400°C. However, a temperature may fluctuate within the range of 250°C to 400°C, or be gradually increased or lowered. The holding time of the heating temperature is 30 minutes or more, and preferably 3 to 12 hours.

A temperature increasing pattern until the pre-stage calcining temperature is reached may be linearly increased, or a temperature may be increased so that an arc of an upward or downward convex is formed.

A mean rate of increase in temperature until the pre-stage calcining temperature is reached is not especially limited. However, the mean rate of increase in temperature is generally about 0.1 to 15°C/min, preferably 0.5 to 5°C/min, and more preferably 1 to 2°C/min.

The main calcination is carried out, preferably under an inert gas flow, at 550 to 800°C, preferably at 580 to 750°C, more preferably at 600 to 720°C, and still more preferably at 620 to 700°C. The main calcination is preferably held at a constant temperature within the temperature range of 620 to 700°C. However, the temperature may fluctuate within the range of 620 to 700°C, or be gradually increased or decreased. The time of the main calcination is 0.5 to 20 hours, and preferably 1 to 15 hours. When partitioned with a weir plate, the dry powder and/or a composite oxide catalyst continuously passes through at least 2 zones, preferably 2 to 20 zones, and more preferably 4 to 15 zones. The temperature can be controlled using one or more controllers. However, in order to obtain the desired calcining pattern, a heater and a controller are preferably placed in each of the zones partitioned with these weir plates to control the temperature. For example, when seven weir plates are placed so that a length of portion of the calcining tube entering a heating furnace is equally divided into eight, and the calcining tube partitioned into the eight zones is used, the setting temperature of each of the eight zones is preferably controlled by the heater and the controller placed in each of the zones so that the temperature of the dry powder and/or the composite oxide catalyst has the desired calcining temperature pattern. An oxidizing component (for example, oxygen) or a reducing component (for example, ammonia) may be added to the calcining atmosphere under the inert gas flow as necessary.

The temperature increasing pattern until the main calcining temperature is reached may be linearly increased, or a temperature may be increased so that an arc of an upward or downward convex is formed.

The mean rate of increase in temperature until the main calcining temperature is reached is not especially limited. However, the mean rate of increase in temperature is generally about 0.1 to 15°C/min, preferably 0.5 to 10°C/min, and more preferably 1 to 8°C/min.

The mean rate of decrease in temperature after the main calcination is completed is 0.01 to 1000°C/min, preferably 0.05 to 100°C/min, more preferably 0.1 to 50°C/min, and still more preferably 0.5 to 10°C/min. A temperature lower than the main calcining temperature is also preferably held once. The holding temperature is lower than the main calcining temperature by 10°C, preferably 50°C, and more preferably 100°C. The holding time is 0.5 hours or more, preferably 1 hour or more, more preferably 3 hours or more, and especially preferably 10 hours or more.

When the main calcination is carried out anew once the pre-stage calcination has been completed, a low temperature treatment is preferably performed in the main calcination. The time required for the low temperature treatment, that is, the time required for decreasing the temperature of the dry powder and/or the composite oxide catalyst and increasing the temperature to the calcining temperature can appropriately be adjusted based on the size, the thickness, and the material of the calcining device, the catalyst production amount, the series of periods for continuously calcining the dry powder and/or the composite oxide catalyst, the fixing rate and amount and the like. For example, when a calcining tube having an inner diameter of 500 mm, a length of 4500 mm, and a thickness of 20 mm, and made of SUS is used, the time required for the low temperature treatment is preferably within 30 days during the series of periods for continuously calcining a catalyst, more preferably within 15 days, still more preferably within 3 days, and especially preferably within 2 days.

For example, when the dry powder is supplied at a rate of 35 kg/hr while a rotary kiln having a calcining tube having an inner diameter of 500 mm, a length of 4500 mm, and a thickness of 20 mm and made of SUS is rotated at 6 rpm, and the main calcining temperature is set to be 645°C, the step of decreasing the temperature to 400°C and increasing the temperature to 645°C can be performed in about 1 day. When calcination is continuously performed for 1 year, the calcination can be performed by carrying out such low temperature treatment once a month while the temperature of the oxide layer is stably maintained.

Examples

The present invention will now be described in more detail based on the following Examples and Comparative Examples.

In Examples and Comparative Examples, the acrylonitrile yield (AN yield) is based on the following definition.

$$\text{Acrylonitrile yield (\%)} = (\text{number of moles of produced acrylonitrile}) / (\text{number of moles of supplied propane}) \times 100$$

Here, the number of moles of produced acrylonitrile was measured with the thermal conductivity detector (TCD) type gas chromatograph GC2014AT, manufactured by Shimadzu Corporation.

Further, the respective physical property values of the catalyst were measured as follows.

(1) Normalized UV value

The normalized UV value was determined using the following equation (1) based on the absorbance at 400 nm, 580 nm, and 700 nm of the absorption and/or reflection spectrum obtained by setting the extracted catalyst in a sample holder and measuring based on a diffuse reflection method using an ultraviolet-visible spectrophotometer (V-660, manufactured by JASCO Corporation).

$$\text{Normalized UV value} = \{(580 \text{ nm absorbance}) - (400 \text{ nm absorbance})\} / \{(700 \text{ nm absorbance}) - (400 \text{ nm absorbance})\} \quad (1)$$

(2) Reduction ratio

About 200 mg of extracted catalyst was precisely weighed into a beaker. Further, a 1/40 N aqueous solution of $KMnO_4$ was added in excess (usually 24 ml). Then, about 150 mL of purified water and 2 mL of 1:1 sulfuric acid (i.e., an aqueous solution of sulfuric acid obtained by mixing concentrated sulfuric acid and purified water in a 1/1 volumetric ratio) was added to the mixture. The beaker was then covered with a watch glass, and the sample was oxidized while stirring for 1 hour in a hot bath of 70°C $\pm$2°C. At this point, $KMnO_4$ was present in excess, so that unreacted $KMnO_4$ was present in the solution. Therefore, it was confirmed that the solution had a purple to magenta color. After oxidation finished, the solution was filtered with filter paper, and all of the filtrate was recovered. A 1/40 N aqueous solution of sodium oxalate was added in excess (usually 15 ml) based on the $KMnO_4$ present in the filtrate. The solution was heated to a temperature of 70°C, and stirred. It was confirmed that the solution had become colorless and was transparent, and then 2 mL of 1:1 sulfuric acid was added. Stirring was continued while maintaining the solution temperature at 70°C $\pm$2°C, and the solution was then titrated with a 1/40 N aqueous solution of $KMnO_4$. The endpoint was taken as the point at which the solution had a faint pale peach color from the $KMnO_4$ that continued for 30 seconds or more. The $KMnO_4$ amount consumed in the oxidation of the sample was determined based on the total amount of $KMnO_4$ and the total amount of $Na_2C_2O_4$. From this value, $(n_0 - n)$ was calculated, and based on the calculated result the reduction ratio was determined.

The catalyst reduction ratio was calculated based on the following equation (2).

$$\text{Reduction ratio (\%)} = ((n_0 - n) / n_0) \times 100 \quad (2)$$

(wherein n denotes the number of oxygen atoms satisfying the valence of the constituent elements other than oxygen in the catalyst; and $n_0$ denotes the number of oxygen atoms required when the constituent elements other than oxygen in the catalyst each have their maximum oxidation number).

(3) Catalyst constituent element concentration (Mo composition)

A part of the extracted catalyst was pasted for about 2 hours using a pasting machine, and then formed into pellets using a press. The catalyst constituent element concentration was then measured using an X-ray fluorescence analyzer (RIX 1000).

[Example 1]

(Preparation of niobium mixed solution)

A niobium mixed solution was prepared as follows.

76.33 kg of niobic acid containing 80.2 mass% as $Nb_2O_5$ and 29.02 kg of oxalic acid dihydrate [$H_2C_2O_4 \cdot 2H_2O$] were mixed in 500 kg of water. The molar ratio of the charged oxalic acid/niobium was 5.0, and the charged niobium concentration was 0.532 (mol-Nb/Kg-solution). The solution was heated and stirred for 2 hours at 95°C to obtain a mixed solution in which niobium was dissolved. This mixed solution was left to stand, ice-cooled, and then solids were separated by suction filtration to obtain a homogeneous niobium mixed solution. Based on the below-described analysis, the molar ratio of oxalic acid/niobium in this niobium mixed solution was 2.70.

10 g of this niobium mixed solution was precisely weighed into a crucible, dried overnight at 95°C, and then heat treated for 1 hour at 600°C to obtain 0.7868 g of $Nb_2O_5$. Based on this result, the niobium concentration was 0.592 (mol-Nb/Kg-solution).

3 g of this niobium mixed solution was precisely weighed into a 300 mL glass beaker, 200 mL of hot water of about 80°C was added thereto, followed by addition of 10 mL of 1:1 sulfuric acid. The obtained mixed solution was, under stirring while maintaining the temperature of the solution at 70°C on a hot stirrer, titrated using 1/4 N $KMnO_4$. The endpoint was taken as the point at which a faint pale peach color from the $KMnO_4$ continued for about 30 seconds or more. Based on a calculation using the following equation from the titer, the oxalic acid concentration was 1.573 (mol-oxalic acid/Kg).

$$2KMnO_4 + 3H_2SO_4 + 5H_2C_2O_4 \rightarrow K_2SO_4 + 2MnSO_4 + 10CO_2 + 8H_2O$$

The obtained niobium mixed solution was used as the niobium mixed solution ($B_0$) in the below-described catalyst preparation.

(Preparation of composite oxide catalyst)

33.3 kg of ammonium heptamolybdate [$(NH_4)_6Mo_7O_{24} \cdot 4H_2O$], 4.41 kg of ammonium metavanadate [$NH_4VO_3$], and 6.49 kg of antimony trioxide [$Sb_2O_3$] were added to 114.6 kg of water, and the resultant mixture was heated for 1 hour at 95°C under stirring to obtain an aqueous raw-material solution (I) .

To 29.68 kg of the niobium mixed solution ($B_0$), 3.98 kg of hydrogen peroxide water containing 30 wt.% as $H_2O_2$ was added, and the resultant mixture was stirred and mixed for 10 minutes at room temperature to prepare an aqueous raw-material solution (II).

The obtained aqueous raw-material solution (I) was cooled to 70°C, 59.90 kg of silica sol containing 34.0 wt.% as $SiO_2$ was added thereto, then 6.27 kg of hydrogen peroxide water containing 30 wt.% as $H_2O_2$ was further added, and the stirring was continued for another 30 minutes at 55°C. Next, a dispersion in which 2.318 g of a 50.2 wt.% solution of aqueous ammonium metatungstate as $WO_2$ and 14.15 kg of powdered silica were dispersed in 191.0 kg of water, was sequentially added to the aqueous raw-material solution (II) to obtain an aqueous mixed solution (III). The aqueous mixed solution (III) was aged at 50°C for 2 hours 30 minutes after the addition of the aqueous raw-material solution (II) to obtain a slurry.

The obtained slurry was dried by feeding it into a centrifugal spray drier to obtain a dry powder having a microspherical shape. The inlet air temperature of the drier was 210°C, and the outlet air temperature was 120°C. This step was repeated several times. The obtained dry powder was packed into a cylindrical calcining tube made of SUS having an inner diameter of 500 mm, a length of 3500 mm, and a thickness of 20 mm, and while rotating the tube under a nitrogen gas flow of 600 NL/min, calcined for 2 hours at 680°C to obtain a composite oxide catalyst.

(Removal of protrusions)

50 g of the composite oxide catalyst was charged into a perpendicular tube (inner diameter 41.6 mm, length 70 cm) including a perforated disc having three holes with a diameter of 0.3968 mm (1/64 inch) on a bottom portion and provided with a paper filter on an upper portion. The gas flow length in the direction that the gas flowed at this stage was 52 mm, and the gas flow average linear velocity was 310 m/s. No protrusions were present in the composite oxide catalyst that was obtained 24 hours later.

The composition of the composite oxide catalyst was measured by X-ray fluorescence analysis (Rigaku RINT1000, Cr tube, tube voltage 50 kV, tube current 50 mA). The obtained composite oxide catalyst composition was $Mo_{1.0}V_{0.214}Sb_{0.220}b_{0.105}W_{0.030}Ce_{0.005}O_n/50.0$ wt.%-$SiO_2$.

(Propane ammoxidation reaction)

580 kg of the obtained composite oxide catalyst was packed into a carbon steel fluidized bed reactor having an inner diameter of 600 mm. For the packing, the composite oxide catalyst was conveyed using 320°C air. The temperature of the catalyst after conveyance was 210°C. Nozzles for supplying a gas containing propane and ammonia were placed pointing vertically downwards at a position 30 cm above the bottom of the catalyst packed portion of the reactor. The placement positions were the center of the reactor, and at the apexes of a square having 340 mm sides with the center of the reactor as its center (total of 5 locations). Nozzles for supplying a gas containing oxygen were placed pointing vertically upwards at the bottom of the catalyst packed portion of the reactor. The placement positions were set so as to overlap in the vertical direction with the nozzles for supplying a gas containing propane and ammonia (total of 5 locations). For heat removal in the reactor, four cooling coils to be continuously used and two cooling coils for fine adjustments to the temperature were placed in the catalyst dense layer. After conveyance was finished, 450°C air was introduced into the reactor, and the temperature of the catalyst layer in the reactor was increased to 340°C over 12 hours. At this point the supply of ammonia gas was started. The ammonia supply rate was increased to 55 $Nm^3$/hr over 3 hours, and the temperature of the catalyst layer in the reactor was increased further. After increasing the supply rate of ammonia, the supply rate of air was decreased to 280 $Nm^3$/hr. When the temperature of the catalyst layer in the reactor reached 450°C, the supply of propane was started. The propane supply rate was increased to 22.7 $Nm^3$/hr over 6 hours, and the supply rate of air was simultaneously increased to 363 $Nm^3$/hr.

Subsequently, the respective gas amounts and temperatures were adjusted so that at a reactor temperature of 440°C and a reaction pressure of 50 kPa, propane and ammonia from the upper side nozzles and air from the lower side nozzles were supplied at a molar ratio of propane:ammonia:air = 1:1:16 for a contact time of 2.9 sec.g/**cm³**. The AN yield 1 day after the reaction started was 53.0%. Ten days after the reaction started, 500 g of catalyst was extracted from the reactor. The catalyst particles were sieved using sieves having apertures of 32 $\mu$m and 100 $\mu$m, and the reduction ratio and catalyst constituent element concentration of the 32 to 100 $\mu$m catalyst particles were measured. The measurement results of the respective physical property values and the AN yield at this point were as shown in Table 1. Further, the molar ratio of air/propane introduced into the reactor was decreased by 1, and operation was continued. Five days after the change of conditions, the catalyst was similarly extracted from the reactor, and the physical property values and yield were measured. The results were as shown in Table 1.

[Example 2]

A composite oxide catalyst was obtained in the same manner as in Example 1.

(Propane ammoxidation reaction)

A reaction was carried out in the same manner as in Example 1, except that the gases were supplied in a propane:ammonia:air molar ratio of 1:1:14. Ten days after the reaction started, 500 g of catalyst was extracted from the reactor, and measurement of the physical property values shown in Table 1 was carried out. The measurement results of the respective physical property values and the AN yield at this point were as shown in Table 1. Further, the molar ratio of air/propane introduced into the reactor was increased by 0.5, and operation was continued. Five days after the change of conditions, the catalyst was similarly extracted from the reactor, and the physical property values and yield were measured. The results were as shown in Table 1.

[Example 3]

A composite oxide catalyst was obtained in the same manner as in Example 1.

(Propane ammoxidation reaction)

A reaction was carried out in the same manner as in Example 1, except that the gases were supplied in a propane:ammonia:air molar ratio of 1:0.8:15. Ten days after the reaction started, 500 g of catalyst was extracted from the reactor, and measurement of the physical property values shown in Table 1 was carried out. The measurement results of the respective physical property values and the AN yield at this point were as shown in Table 1. Further, the molar ratio of ammonia/propane introduced into the reactor was increased by 0.15, and operation was continued. Five days after the change of conditions, the catalyst was similarly extracted from the reactor, and the physical property values and yield were measured. The results were as shown in Table 1.

[Example 4]

A composite oxide catalyst was obtained in the same manner as in Example 1.

(Propane ammoxidation reaction)

A reaction was carried out in the same manner as in Example 1, except that the gases were supplied at a molar ratio of propane:$NH_3$:air=1:0.9:15.
Ten days after the reaction started, 500 g of catalyst was extracted from the reactor, and measurement of the physical property values shown in Table 1 was carried out. The measurement results of the respective physical property values and the AN yield at this point were as shown in Table 1. Further, the flow rate WWH of propane per amount of catalyst introduced into the reactor was decreased by 0.02, and operation was continued. Five days after the change of conditions, the catalyst was similarly extracted from the reactor, and the physical property values and yield were measured. The results were as shown in Table 1.

[Example 5]

A composite oxide catalyst was obtained in the same manner as in Example 1.

(Propane ammoxidation reaction)

A reaction was carried out in the same condition as "Reaction Conditions After Change" of Example 1. Ten days after the reaction started, 500 g of catalyst was extracted from the reactor, and measurement of the physical property values shown in Table 1 was carried out. The measurement results of the respective physical property values and the AN yield at this point were as shown in Table 1. Further, the molar ratio of ammonia/propane introduced into the reactor was increased by 0.08, and operation was continued. One day after the change of conditions, the catalyst was similarly extracted from the reactor, and the physical property values and yield were measured. The results were as shown in Table 1.

[Example 6]

A composite oxide catalyst was obtained in the same manner as in Example 1.

(Propane ammoxidation reaction)

A reaction was carried out in the same manner as in Example 1, except that the gases were supplied in a propane:ammonia:air molar ratio of 1:1:13. Ten days after the reaction started, 500 g of catalyst was extracted from the reactor, and measurement of the physical property values shown in Table 1 was carried out. The measurement results of the respective physical property values and the AN yield at this point were as shown in Table 1. Further, the molar ratio of air/propane introduced into the reactor was increased by 1, the temperature of the catalyst layer in the reactor was increased by 2°C, and operation was continued. In addition, 1 kg of ammonium heptamolybdate per day was added into the reactor. Five days after the change of conditions, the catalyst was similarly extracted from the reactor, and the physical property values and yield were measured. The results were as shown in Table 1.

[Example 7]

A composite oxide catalyst was obtained in the same manner as in Example 1.

(Propane ammoxidation reaction)

A reaction was carried out in the same manner as in Example 1, except that the gases were supplied in a propane:ammonia:air molar ratio of 1:1.05:13.5. Ten days after the reaction started, 500 g of catalyst was extracted from the reactor, and measurement of the physical property values shown in Table 1 was carried out. The measurement results of the respective physical property values and the AN yield at this point were as shown in Table 1. Further, the molar ratio of air/propane introduced into the reactor was increased by 0.5, and 20 kg of catalyst was newly added into the reactor. One day after the change of conditions, the catalyst was similarly extracted from the reactor, and the physical property values and yield were measured. The results were as shown in Table 1.

[Comparative Example 1]

A composite oxide catalyst was obtained in the same manner as in Example 1.

(Propane ammoxidation reaction)

A reaction was carried out in the same manner as in Example 1, except that the gases were supplied in a propane:ammonia:air molar ratio of 1:0.9:15. Ten days after the reaction started, 500 g of catalyst was extracted from the reactor, and measurement of the physical property values shown in Table 1 was carried out. The measurement results of the respective physical property values and the AN yield at this point were as shown in Table 1. Further, 0.5 kg of ammonium heptamolybdate per day was added into the reactor. One day after the change of conditions, the catalyst was similarly extracted from the reactor, and the physical property values and yield were measured. The concentration of the catalyst constituent elements and the AN yield were as shown in Table 1.

[Example 8]

A composite oxide catalyst was obtained in the same manner as in Example 1.

(Propane ammoxidation reaction)

580 kg of the obtained composite oxide catalyst was packed into a carbon steel fluidized bed reactor having an inner diameter of 600 mm. For the packing, the composite oxide catalyst was conveyed using 320°C air over 6 hours. The temperature of the catalyst after conveyance was 210°C. Nozzles for supplying a gas containing propane and ammonia were placed pointing vertically downwards at a position 30 cm above the bottom of the catalyst packed portion of the reactor. The placement positions were the center of the reactor, and at the apexes of a square having 340 mm sides with the center of the reactor as its center (total of 5 locations). Nozzles for supplying a gas containing oxygen were placed pointing vertically upwards at the bottom of the catalyst packed portion of the reactor. The placement positions were set so as to overlap in the vertical direction with the nozzles for supplying a gas containing propane and ammonia (total of 5 locations). For heat removal in the reactor, four cooling coils to be continuously used and two cooling coils for fine adjustments to the temperature were placed in the catalyst dense layer. After conveyance was finished, 450°C air was introduced into the reactor, and the temperature of the catalyst layer in the reactor was increased to 320°C over 12 hours. At this point the supply of ammonia gas was started. The ammonia supply rate was increased to 70 $Nm^3$/hr over 8 hours, and the temperature of the catalyst layer in the reactor was increased further. After increasing the supply rate of ammonia, the supply rate of air was decreased to 280 $Nm^3$/hr. When the temperature of the catalyst layer in the reactor reached 470°C, the supply of propane was started. The propane supply rate was increased to 22.7 $Nm^3$/hr over 6 hours, and the supply rate of air was simultaneously decreased to 261 $Nm^3$/hr.

Subsequently, the respective gas amounts and temperatures were adjusted to the same conditions as "Reaction Conditions After Change" of Example 1. Immediately after adjustment, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 2 were measured. The measurement results of the respective physical property values and the AN yield at this point were as shown in Table 2. Further, the molar ratio of air/propane introduced into the reactor was increased by 2, the temperature of the catalyst layer in the reactor was increased by 5°C, and operation was continued. Five days after the change of conditions, the catalyst was similarly extracted from the reactor, and the physical property values and yield were measured. The results were as shown in Table 2.

[Example 9]

A composite oxide catalyst was obtained in the same manner as in Example 1.

(Propane ammoxidation reaction)

580 kg of the obtained composite oxide catalyst was packed into a carbon steel fluidized bed reactor having an inner diameter of 600 mm. For the packing, the composite oxide catalyst was conveyed using 360°C air over 12 hours. The temperature of the catalyst after conveyance was 260°C. Nozzles for supplying a gas containing propane and ammonia were placed pointing vertically downwards at a position 30 cm above the bottom of the catalyst packed portion of the reactor. The placement positions were the center of the reactor, and at the apexes of a square having 340 mm sides with the center of the reactor as its center (total of 5 locations). Nozzles for supplying a gas containing oxygen were placed pointing vertically upwards at the bottom of the catalyst packed portion of the reactor. The placement positions were set so as to overlap in the vertical direction with the nozzles for supplying a gas containing propane and ammonia (total of 5 locations). For heat removal in the reactor, four cooling coils to be continuously used and two cooling coils for fine adjustments to the temperature were placed in the catalyst dense layer. After conveyance was finished, 510°C air was introduced into the reactor, and the temperature of the catalyst layer in the reactor was increased to 400°C over 12 hours. At this point the supply of ammonia gas was started. The ammonia supply rate was increased to 25 $Nm^3$/hr over 5 hours, and the temperature of the catalyst layer in the reactor was increased further. After increasing the supply rate of ammonia, the supply rate of air was decreased to 300 $Nm^3$/hr. When the temperature of the catalyst layer in the reactor was at 440°C, the supply of propane was started. The propane supply rate was increased to 22.7 $Nm^3$/hr over 6 hours, and the supply rate of air was simultaneously increased to 386 $Nm^3$/hr.

Subsequently, the respective gas amounts and temperatures were adjusted to the same conditions as "Reaction Conditions After Change" of Example 1. Immediately after adjustment, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 2 were measured. The measurement results of the respective physical property values and the AN yield at this point were as shown in Table 2. Further, the molar ratio of air/propane introduced into the reactor was decreased by 3, and operation was continued. Five days after the change of conditions, the catalyst was similarly extracted from the reactor, and the physical property values and yield were measured. The results were as in Table 2.

[Example 10]

A composite oxide catalyst was obtained in the same manner as in Example 1.

(Propane ammoxidation reaction)

580 kg of the obtained composite oxide catalyst was packed into a carbon steel fluidized bed reactor having an inner diameter of 600 mm. For the packing, the composite oxide catalyst was conveyed using 320°C air over 6 hours. The temperature of the catalyst after conveyance was 210°C. Nozzles for supplying a gas containing propane and ammonia were placed pointing vertically downwards at a position 30 cm above the bottom of the catalyst packed portion of the reactor. The placement positions were the center of the reactor, and at the apexes of a square having 340 mm sides with the center of the reactor as its center (total of 5 locations). Nozzles for supplying a gas containing oxygen were placed pointing vertically upwards at the bottom of the catalyst packed portion of the reactor. The placement positions were set so as to overlap in the vertical direction with the nozzles for supplying a gas containing propane and ammonia (total of 5 locations). For heat removal in the reactor, four cooling coils to be continuously used and two cooling coils for fine adjustments to the temperature were placed in the catalyst dense layer. After conveyance was finished, 450°C air was introduced into the reactor, and the temperature of the catalyst layer in the reactor was increased to 410°C over 36 hours. At this point the supply of ammonia gas was started. The ammonia supply rate was increased to 55 $Nm^3$/hr over 5 hours, and the temperature of the catalyst layer in the reactor was increased further. After increasing the supply rate of ammonia, the supply rate of air was decreased to 280 $Nm^3$/hr. When the temperature of the catalyst layer in the reactor reached 450°C, the supply of propane was started. The propane supply rate was increased to 20 $Nm^3$/hr over 6 hours, and the supply rate of air was simultaneously increased to 329 $Nm^3$/hr.

Subsequently, the respective gas amounts and temperatures were adjusted to the same conditions as "Reaction Conditions After Change" of Example 1. Immediately after adjustment, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 2 were measured. The measurement results of the respective physical property values and the AN yield at this point were as shown in Table 2. The results of the physical property values were confirmed, and operation was continued without changing the conditions. Five days later, the catalyst was similarly extracted from the reactor, and the physical property values and yield were measured. The results were as shown in Table 2.

[Comparative Example 2]

A composite oxide catalyst was obtained in the same manner as in Example 1.

(Propane ammoxidation reaction)

580 kg of the obtained composite oxide catalyst was packed into a carbon steel fluidized bed reactor having an inner diameter of 600 mm. For the packing, the composite oxide catalyst was conveyed using 360°C air over 12 hours. The temperature of the catalyst after conveyance was 260°C. Nozzles for supplying a gas containing propane and ammonia were placed pointing vertically downwards at a position 30 cm above the bottom of the catalyst packed portion of the reactor. The placement positions were the center of the reactor, and at the apexes of a square having 340 mm sides with the center of the reactor as its center (total of 5 locations). Nozzles for supplying a gas containing oxygen were placed pointing vertically upwards at the bottom of the catalyst packed portion of the reactor. The placement positions were set so as to overlap in the vertical direction with the nozzles for supplying a gas containing propane and ammonia (total of 5 locations). For heat removal in the reactor, four cooling coils to be continuously used and two cooling coils for fine adjustments to the temperature were placed in the catalyst dense layer. After conveyance was finished, 500°C air was introduced into the reactor, and the temperature of the catalyst layer in the reactor was increased to 400°C over 24 hours. At this point the supply of ammonia gas was started. The ammonia supply rate was increased to 25 $Nm^3$/hr over 5 hours, and the temperature of the catalyst layer in the reactor was increased further. After increasing the supply rate of ammonia, the supply rate of air was decreased to 300 $Nm^3$/hr. When the temperature of the catalyst layer in the reactor reached 440°C, the supply of propane was started. The propane supply rate was increased to 22.7 $Nm^3$/hr over 6 hours, and the supply rate of air was simultaneously increased to 386 $Nm^3$/hr.

Subsequently, the respective gas amounts and temperatures were adjusted to the same conditions as "Reaction Conditions After Change" of Example 1.

Without having extracted the catalyst from the reactor, and without having measured the physical property values of the catalyst, the AN yield five days after the reaction started was as shown in Table 2.

[Comparative Example 3]

A composite oxide catalyst was obtained in the same manner as in Example 1.

(Propane ammoxidation reaction)

580 kg of the obtained composite oxide catalyst was packed into a carbon steel fluidized bed reactor having an inner diameter of 600 mm. For the packing, the composite oxide catalyst was conveyed using 360°C air over 12 hours. The temperature of the catalyst after conveyance was 260°C. Nozzles for supplying a gas containing propane and ammonia were placed pointing vertically downwards at a position 30 cm above the bottom of the catalyst packed portion of the reactor. The placement positions were the center of the reactor, and at the apexes of a square having 340 mm sides with the center of the reactor as its center (total of 5 locations). Nozzles for supplying a gas containing oxygen were placed pointing vertically upwards at the bottom of the catalyst packed portion of the reactor. The placement positions were set so as to overlap in the vertical direction with the nozzles for supplying a gas containing propane and ammonia (total of 5 locations). For heat removal in the reactor, four cooling coils to be continuously used and two cooling coils for fine adjustments to the temperature were placed in the catalyst dense layer. After conveyance was finished, 480°C air was introduced into the reactor, and the temperature of the catalyst layer in the reactor was increased to 370°C over 12 hours. At this point the supply of ammonia gas was started. The ammonia supply rate was increased to 40 $Nm^3$/hr over 5 hours, and the temperature of the catalyst layer in the reactor was increased further. After increasing the supply rate of ammonia, the supply rate of air was decreased to 280 $Nm^3$/hr. When the temperature of the catalyst layer in the reactor reached 440°C, the supply of propane was started. The propane supply rate was increased to 22.7 $Nm^3$/hr over 6 hours, and the supply rate of air was simultaneously increased to 341 $Nm^3$/hr.

Subsequently, the respective gas amounts and temperatures were adjusted to the same conditions as "Reaction Conditions After Change" of Example 1. Immediately after adjustment, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 2 were measured. The measurement results of the respective physical property values and the AN yield at this point were as shown in Table 2. Further, the molar ratio of air/propane introduced into the reactor was increased by 2, and operation was continued. Five days after the change of conditions, the catalyst was similarly extracted from the reactor, and the physical property values and yield were measured. The normalized UV value, reduction ratio, catalyst constituent element concentration, and AN yield were as shown in Table 2.

[Example 11]

A composite oxide catalyst was obtained in the same manner as in Example 1.

(Propane ammoxidation reaction)

580 kg of the obtained composite oxide catalyst was packed into a carbon steel fluidized bed reactor having an inner diameter of 600 mm. For the packing, the composite oxide catalyst was conveyed using 360°C air over 12 hours. The temperature of the catalyst after conveyance was 260°C. Nozzles for supplying a gas containing propane and ammonia were placed pointing vertically downwards at a position 30 cm above the bottom of the catalyst packed portion of the reactor. The placement positions were the center of the reactor, and at the apexes of a square having 340 mm sides with the center of the reactor as its center (total of 5 locations). Nozzles for supplying a gas containing oxygen were placed pointing vertically upwards at the bottom of the catalyst packed portion of the reactor. The placement positions were set so as to overlap in the vertical direction with the nozzles for supplying a gas containing propane and ammonia (total of 5 locations). For heat removal in the reactor, four cooling coils to be continuously used and two cooling coils for fine adjustments to the temperature were placed in the catalyst dense layer. After conveyance was finished, 480°C air was introduced into the reactor, and the temperature of the catalyst layer in the reactor was increased to 370°C over 12 hours. At this point the supply of ammonia gas was started. The ammonia supply rate was increased to 40 $Nm^3$/hr over 5 hours, and the temperature of the catalyst layer in the reactor was increased further. After increasing the supply rate of ammonia, the supply rate of air was decreased to 280 $Nm^3$/hr. When the temperature of the catalyst layer in the reactor was at 440°C, the supply of propane was started. The propane supply rate was increased to 22.7 $Nm^3$/hr over 6 hours, and the supply rate of air was simultaneously increased to 350 $Nm^3$/hr.

Subsequently, the respective gas amounts and temperatures were adjusted to the same conditions as "Reaction Conditions After Change" of Example 1. Immediately after adjustment, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 2 were measured. The measurement results of the respective physical property values and the AN yield at this point were as shown in Table 2. Further, the molar ratio of air/propane introduced into the reactor was decreased by 0.5, and operation was continued. Five days after the change of conditions, the catalyst was similarly extracted from the reactor, and the physical property values and yield were measured. The normalized UV value, reduction ratio, AN yield, and catalyst constituent element concentration were as shown in Table 2.

[Example 12]

A composite oxide catalyst was obtained in the same manner as in Example 1.

(Propane ammoxidation reaction)

580 kg of the obtained composite oxide catalyst was packed into a carbon steel fluidized bed reactor having an inner diameter of 600 mm. For the packing, the composite oxide catalyst was conveyed using 360°C air over 12 hours. The temperature of the catalyst after conveyance was 260°C. Nozzles for supplying a gas containing propane and ammonia were placed pointing vertically downwards at a position 30 cm above the bottom of the catalyst packed portion of the reactor. The placement positions were the center of the reactor, and at the apexes of a square having 340 mm sides with the center of the reactor as its center (total of 5 locations). Nozzles for supplying a gas containing oxygen were placed pointing vertically upwards at the bottom of the catalyst packed portion of the reactor. The placement positions were set so as to overlap in the vertical direction with the nozzles for supplying a gas containing propane and ammonia (total of 5 locations). For heat removal in the reactor, four cooling coils to be continuously used and two cooling coils for fine adjustments to the temperature were placed in the catalyst dense layer. After conveyance was finished, 440°C air was introduced into the reactor, and the temperature of the catalyst layer in the reactor was increased to 330°C over 12 hours. At this point the supply of ammonia gas was started. The ammonia supply rate was increased to 60 $Nm^3$/hr over 5 hours, and the temperature of the catalyst layer in the reactor was increased further. After increasing the supply rate of ammonia, the supply rate of air was decreased to 260 $Nm^3$/hr. When the temperature of the catalyst layer in the reactor was at 460°C, the supply of propane was started. The propane supply rate was increased to 22.7 $Nm^3$/hr over 6 hours, and the supply rate of air was simultaneously increased to 318 $Nm^3$/hr.

Subsequently, the respective gas amounts and temperatures were adjusted to the same conditions as "Reaction Conditions After Change" of Example 1. Immediately after adjustment, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 2 were measured. The measurement results of the respective physical property values and the AN yield at this point were as shown in Table 2. Further, the molar ratio of ammonia/propane introduced into the reactor was decreased by 0.05, and operation was continued. Five days after the change of conditions, the catalyst was similarly extracted from the reactor, and the physical property values and yield were measured. The results were as shown in Table 2.

[Example 13]

A composite oxide catalyst was obtained in the same manner as in Example 1.

(Propane ammoxidation reaction)

580 kg of the obtained composite oxide catalyst was packed into a carbon steel fluidized bed reactor having an inner diameter of 600 mm. For the packing, the composite oxide catalyst was conveyed using 330°C air over 24 hours. The temperature of the catalyst after conveyance was 240°C. Nozzles for supplying a gas containing propane and ammonia were placed pointing vertically downwards at a position 30 cm above the bottom of the catalyst packed portion of the reactor. The placement positions were the center of the reactor, and at the apexes of a square having 340 mm sides with the center of the reactor as its center (total of 5 locations). Nozzles for supplying a gas containing oxygen were placed pointing vertically upwards at the bottom of the catalyst packed portion of the reactor. The placement positions were set so as to overlap in the vertical direction with the nozzles for supplying a gas containing propane and ammonia (total of 5 locations). For heat removal in the reactor, four cooling coils to be continuously used and two cooling coils for fine adjustments to the temperature were placed in the catalyst dense layer. After conveyance was finished, 500°C air started to be introduced into the reactor. Two hours later, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 3 were measured. The temperature of the introduced air was decreased to 450°C, and the temperature of the catalyst layer in the reactor was increased to 320°C over 8 hours. Then, a reaction was started under the same conditions as in Example 10.
Subsequently, the respective gas amounts and temperatures were adjusted to the same conditions as in Example 1. One day after adjustment was finished, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 3 were measured. The measurement results of the respective physical property values and the AN yield at this point were as shown in Table 3.

[Example 14]

A composite oxide catalyst was obtained in the same manner as in Example 1.

(Propane ammoxidation reaction)

580 kg of the obtained composite oxide catalyst was packed into a carbon steel fluidized bed reactor having an inner diameter of 600 mm. For the packing, the composite oxide catalyst was conveyed using 320°C air over 6 hours. The temperature of the catalyst after conveyance was 210°C. Nozzles for supplying a gas containing propane and ammonia were placed pointing vertically downwards at a position 30 cm above the bottom of the catalyst packed portion of the reactor. The placement positions were the center of the reactor, and at the apexes of a square having 340 mm sides with the center of the reactor as its center (total of 5 locations). Nozzles for supplying a gas containing oxygen were placed pointing vertically upwards at the bottom of the catalyst packed portion of the reactor. The placement positions were set so as to overlap in the vertical direction with the nozzles for supplying a gas containing propane and ammonia (total of 5 locations). For heat removal in the reactor, four cooling coils to be continuously used and two cooling coils for fine adjustments to the temperature were placed in the catalyst dense layer. After conveyance was finished, 450°C air started to be introduced into the reactor, and the temperature of the catalyst layer in the reactor was increased to 320°C over 8 hours. At this point the supply of ammonia gas was started. The ammonia supply rate was increased to 80 Nm$^3$/hr over 20 minutes. Three hours later, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 3 were measured. The ammonia supply rate was decreased to 50 Nm$^3$/hr, and the temperature of the catalyst layer in the reactor was further increased over 2 hours. Then, a reaction was started under the same conditions as in Example 10.
Subsequently, the respective gas amounts and temperatures were adjusted to the same conditions as in Example 1. One day after adjustment was finished, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 3 were measured. The measurement results of the respective physical property values and the AN yield at this point were as shown in Table 3.

[Example 15]

A composite oxide catalyst was obtained in the same manner as in Example 1.

(Propane ammoxidation reaction)

580 kg of the obtained composite oxide catalyst was packed into a carbon steel fluidized bed reactor having an inner diameter of 600 mm. For the packing, the composite oxide catalyst was conveyed using 320°C air over 6 hours. The temperature of the catalyst after conveyance was 210°C. Nozzles for supplying a gas containing propane and ammonia were placed pointing vertically downwards at a position 30 cm above the bottom of the catalyst packed portion of the reactor. The placement positions were the center of the reactor, and at the apexes of a square having 340 mm sides with the center of the reactor as its center (total of 5 locations). Nozzles for supplying a gas containing oxygen were placed pointing vertically upwards at the bottom of the catalyst packed portion of the reactor. The placement positions were set so as to overlap in the vertical direction with the nozzles for supplying a gas containing propane and ammonia (total of 5 locations). For heat removal in the reactor, four cooling coils to be continuously used and two cooling coils for fine adjustments to the temperature were placed in the catalyst dense layer. After conveyance was finished, 450°C air started to be introduced into the reactor, and the temperature of the catalyst layer in the reactor was increased to 340°C over 12 hours. At this point the supply of ammonia gas was started. The ammonia supply rate was increased to 55 Nm$^3$/hr over 3 hours, and the temperature of the catalyst layer in the reactor was increased further. After increasing the supply rate of ammonia, the supply rate of air was decreased to 280 m$^3$/hr. When the temperature of the catalyst layer in the reactor reached 460°C, the supply of propane was started. The propane supply rate was increased to 22.7 Nm$^3$/hr over 6 hours, and the supply rate of air was simultaneously increased to 290 Nm$^3$/hr. Three hours later, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 3 were measured. The propane supply rate was decreased to 20 Nm$^3$/hr, and the temperature of the catalyst layer in the reactor was increased further over 3 hours. Then, a reaction was started as in Example 10.
Subsequently, the respective gas amounts and temperatures were adjusted to the same conditions as in Example 1. One day after adjustment was finished, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 3 were measured. The measurement results of the respective physical property values and the AN yield at this point were as shown in Table 3.

[Comparative Example 4]

A composite oxide catalyst was obtained in the same manner as in Example 1.

(Propane ammoxidation reaction)

A reaction was started as in Example 13, except that during the reaction the steps of extracting the catalyst, measuring the physical property values, and changing the reaction conditions were not carried out. The AN yield at this point was as shown in Table 3.

[Comparative Example 5]

A composite oxide catalyst was obtained in the same manner as in Example 1.

(Propane ammoxidation reaction)

A reaction was started in the same manner as in Example 14, except that during the reaction the steps of extracting the catalyst, measuring the physical property values, and changing the reaction conditions were not carried out. The AN yield at this point was as shown in Table 3.

[Comparative Example 6]

A composite oxide catalyst was obtained in the same manner as in Example 1.

(Propane ammoxidation reaction)

A reaction was started in the same manner as in Example 15, except that during the reaction the steps of extracting the catalyst, measuring the physical property values, and changing the reaction conditions were not carried out. The AN yield at this point was as shown in Table 3.

[Comparative Example 7]

A composite oxide catalyst was obtained in the same manner as in Example 1.

(Propane ammoxidation reaction)

35 g of the obtained composite oxide catalyst was packed into a glass fluidized bed reactor having an inner diameter 1B. 460°C air was introduced into the reactor, and the temperature of the catalyst layer in the reactor was increased to 320°C over 36 hours. At this point the supply of ammonia gas was started. The ammonia supply rate was increased to 30 Ncc/min over 5 hours, and the temperature of the catalyst layer in the reactor was increased further. After increasing the supply rate of ammonia, the supply rate of air was decreased to 180 Ncc/min. When the temperature of the catalyst layer in the reactor reached 450°C, the supply of propane was started. The propane supply rate was increased to 20 Ncc/min over 6 hours, and the reaction was started. Subsequently, the respective gas amounts and temperatures were adjusted so that at a reactor temperature of 440°C and a reaction pressure of 50 kPa, propane, ammonia, and air were supplied at a molar ratio of propane:ammonia:air = 1:1:15 for a contact time of 2.9 sec·g/cc. The AN yield 1 day after the reaction started was 53.0%.

560 kg of composite oxide catalyst obtained in the same manner was packed into a carbon steel fluidized bed reactor having an inner diameter of 600 mm. Then, with the same conditions and gas flow rates per catalyst amount as used for the above-described glass fluidized bed reactor having an inner diameter 1B, and the other conditions being similar, the reaction was started. Catalyst extraction and physical property value measurement during the reaction were not carried out. The AN yield 1 day after the reaction started was 52.5%.

[Example 16]

A composite oxide catalyst was obtained in the same manner as in Example 1.

(Propane ammoxidation reaction)

A reaction was started under the same conditions as in Example 10.

(Reaction termination)

While decreasing the supply rate of propane into the reactor to 0 $Nm^3$/hr over 6 hours, the supply rate of ammonia was increased to 80 $Nm^3$/hr. The supply rate of air at this point was 350 $Nm^3$/hr. Immediately before the supply rate of propane reached 0 $Nm^3$/hr, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 4 were measured. The supply rate of air was increased to 390 $Nm^3$/hr, and the supply rate of ammonia was decreased over 1 hour. Then, the supply rate of air was decreased to 0 $Nm^3$/hr over 8 hours while decreasing the temperature of the catalyst layer in the reactor.

(Reaction re-start)

The reaction was re-started under the same conditions as in Example 1. Ten days after re-starting, the physical property values of the 32 to 100 $\mu$m catalyst particles shown in Table 4 were measured. The measurement results of the respective physical property values and the AN yield are shown in Table 4.

[Example 17]

A composite oxide catalyst was obtained in the same manner as in Example 1.

(Propane ammoxidation reaction)

A reaction was started under the same conditions as in Example 10.

(Reaction termination)

While decreasing the supply rate of propane into the reactor to 0 Nm$^3$/hr over 6 hours, the supply rate of ammonia was increased to 60 Nm$^3$/hr. The supply rate of air at this point was 360 Nm$^3$/hr. The supply rate of ammonia started to be decreased at a rate of 20 Nm$^3$/hr over 10 minutes, and then the 10 minutes later, 500 g of catalyst was extracted from the reactor. The catalyst particles were sieved using sieves having apertures of 32 μm and 100 μm, and the physical property values of the 32 to 100 μm catalyst particles shown in Table 4 were measured. The supply rate of air was decreased to 250 Nm$^3$/hr, and the supply rate of ammonia was decreased to 0 Nm$^3$/hr over 1.5 hours. Then, the supply rate of air was decreased to 0 Nm$^3$/hr over 8 hours while decreasing the temperature of the catalyst layer in the reactor.

(Reaction re-start)

The reaction was re-started under the same conditions as in Example 1. Ten days after re-starting, the physical property values of the 32 to 100 μm catalyst particles shown in Table 4 were measured. The measurement results of the respective physical property values and the AN yield are shown in Table 4.

[Comparative Example 8]

A composite oxide catalyst was obtained in the same manner as in Example 1.

(Propane ammoxidation reaction)

A reaction was started under the same conditions as in Example 10.

(Reaction termination)

The reaction was terminated in the same manner as in Example 16, except that during the reaction the steps of extracting the catalyst, measuring the physical property values, and changing the reaction conditions were not carried out.

(Reaction re-start)

The reaction was re-started under the same conditions as in Example 1. The AN yield 10 days after re-starting is shown in Table 4.

[Comparative Example 9]

A composite oxide catalyst was obtained in the same manner as in Example 1.

(Propane ammoxidation reaction)

A reaction was started under the same conditions as in Example 10.

(Reaction termination)

The reaction was terminated in the same manner as in Example 17, except that during the reaction the steps of extracting the catalyst, measuring the physical property values, and changing the reaction conditions were not carried out.

(Reaction re-start)

The reaction was re-started under the same conditions as in Example 1. The AN yield 10 days after re-starting is shown in Table 4.

[Example 18]

A composite oxide catalyst was obtained in the same manner as in Example 1.

(Propane ammoxidation reaction)

35 g of the obtained composite oxide catalyst was packed into a glass fluidized bed reactor having an inner diameter 1B. Then, with the same flow rates of raw-material gases per catalyst as in Example 10, and the other conditions also the same, the reaction was started.

Subsequently, the respective gas amounts and temperatures were adjusted so that at a reactor temperature of 440°C and a reaction pressure of 50 kPa, propane, ammonia, and air were supplied at a molar ratio of propane:ammonia:air = 1:1:15 for a contact time of 2.9 sec·g/cc. One day after the reaction started, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 5 were measured. The measurement results of the respective physical property values and the AN yield at this point were as shown in Table 5. The molar ratio of air/propane introduced into the reactor was decreased by 2, and operation was continued. One day after the change of conditions, the catalyst was similarly extracted from the reactor, and the physical property values and yield were measured. The results were as shown in Table 5.

[Example 19]

(Preparation of composite oxide catalyst)

A silica-supported catalyst represented by the composition formula $Mo_1V_{0.214}Nb_{0.105}Sb_{0.220}$/50 mass%-$SiO_2$ was produced as follows.

(Preparation of raw-material blend solution)

34.4 kg of ammonium heptamolybdate [$(NH_4)_6Mo_7O_{24}\cdot4H_2O$] , 4.55 kg of ammonium metavanadate [$NH_4VO_3$], and 6.71 kg of antimony trioxide [$Sb_2O_3$] were added to 118.4 kg of water, and the resultant mixture was heated for 1 hour at 95°C under stirring to obtain an aqueous mixed solution A-1.

To 30.7 kg of the above-described niobium raw-material solution, 4.12 kg of hydrogen peroxide water containing 30 mass% as $H_2O_2$ was added, and the resultant mixture was stirred and mixed while maintaining the temperature at about 20°C to obtain an aqueous solution B-1.

The aqueous mixed solution A-1 was cooled to 70°C, 59.9 kg of silica sol containing 34.0 mass% as $SiO_2$ was added thereto, then 7.79 kg of hydrogen peroxide water containing 30 mass% as $H_2O_2$ was further added. The resultant mixture was stirred and mixed for 1 hour at 50°C, and then the aqueous solution B-1 was added. Further, a solution in which 14.1 kg of fumed silica was dispersed in 191.0 kg of water was added to the mixture, which was then stirred for 2.5 hours at 50°C to obtain a raw-material blend solution. A composite oxide catalyst was obtained by carrying out the subsequent steps in the same manner as in Example 1.

(Propane ammoxidation reaction)

35 g of the obtained composite oxide catalyst was packed into a glass fluidized bed reactor having an inner diameter 1B. Then, with the same flow rates of raw-material gasses per catalyst as in Example 10, and the other conditions also the same, the reaction was started.

Subsequently, the respective gas amounts and temperatures were adjusted so that at a reactor temperature of 440°C and a reaction pressure of 50 kPa, propane, ammonia, and air were supplied at a molar ratio of propane:ammonia:air = 1:1:15 for a contact time of 2.9 sec·g/cc. One day after the reaction started, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 5 were measured. The measurement results of the respective physical property values and the AN yield at this point were as shown in Table 5. Further, the molar ratio of air/propane introduced into the reactor was decreased by 2, and operation was continued. One day after the change of conditions, the catalyst was similarly extracted from the reactor, and the physical property values were measured. The results were as shown in Table 5.

[Example 20]

A silica-supported catalyst represented by the composition formula $Mo_1V_{0.214}Nb_{0.105}Sb_{0.220}W_{0.03}Mn_{0.002}/50$ mass%-$SiO_2$ was produced as follows.

(Preparation of raw-material blend solution)

33.4 kg of ammonium heptamolybdate [$(NH_4)_6Mo_7O_{24}\cdot4H_2O$] , 4.42 kg of ammonium metavanadate [$NH_4VO_3$], and 6.51 kg of antimony trioxide [$Sb_2O_3$] were added to 132.6 kg of water, and the resultant mixture was heated for 1 hour at 95°C under stirring to obtain an aqueous mixed solution A-1.

To 29.8 kg of the above-described niobium raw-material solution, 4.00 kg of hydrogen peroxide water containing 30 mass% as $H_2O_2$ was added, and the resultant mixture was stirred and mixed while maintaining the temperature at about 20°C to obtain an aqueous solution B-1.

The aqueous mixed solution A-1 was cooled to 70°C, 66.96 kg of silica sol containing 34.0 mass% as $SiO_2$ was added thereto, then 7.56 kg of hydrogen peroxide water containing 30 mass% as $H_2O_2$ was added. The resultant mixture was stirred and mixed for 1 hour at 50°C, and then the aqueous solution B-1 was added. Further, a solution in which 14.1 kg of fumed silica was dispersed in 198.0 kg of water, 2.33 kg of ammonium metatungstate containing 50 mass% of $WO_3$, and 0.096 kg of manganese nitrate [$Mn(NO_3)_2\cdot6H_2O$] were sequentially added to the mixture, which was then stirred for 2.5 hours at 50°C to obtain a raw-material blend solution. A composite oxide catalyst was obtained by carrying out the subsequent steps in the same manner as in Example 1.

(Propane ammoxidation reaction)

35 g of the obtained composite oxide catalyst was packed into a glass fluidized bed reactor having an inner diameter 1B. Then, with the same flow rates of raw-material gasses per catalyst as in Example 10, and the other conditions also the same, the reaction was started.

Subsequently, the respective gas amounts and temperatures were adjusted so that at a reactor temperature of 440°C and a reaction pressure of 50 kPa, propane, ammonia, and air were supplied at a molar ratio of propane:ammonia:air = 1:1:15 for a contact time of 2.9 sec·g/cc. One day after the reaction started, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 5 were measured. The measurement results of the respective physical property values and the AN yield at this point were as shown in Table 5. The molar ratio of air/propane introduced into the reactor was decreased by 2, and operation was continued. One day after the change of conditions, the catalyst was similarly extracted from the reactor, and the physical property values and yield were measured. The results were as shown in Table 5.

[Example 21]

A silica-supported catalyst represented by the composition formula $Mo_1V_{0.214}Nb_{0.105}Sb_{0.220}B_{0.05}/50$ mass%-$SiO_2$ was produced as follows.

(Preparation of raw-material blend solution)

34.12 kg of ammonium heptamolybdate [$(NH_4)_6Mo_7O_{24}\cdot4H_2O$], 4.52 kg of ammonium metavanadate [$NH_4VO_3$], and 6.66 kg of antimony trioxide [$Sb_2O_3$] were added to 140.1 kg of water, and the resultant mixture was heated for 1 hour at 95°C under stirring to obtain an aqueous mixed solution A-1.

To 30.44 kg of the above-described niobium raw-material solution, 4.09 kg of hydrogen peroxide water containing 30 mass% as $H_2O_2$ was added, and the resultant mixture was stirred and mixed while maintaining the temperature at about 20°C to obtain an aqueous solution B-1.

The aqueous mixed solution A-1 was cooled to 70°C, 66.96 kg of silica sol containing 34.0 mass% as $SiO_2$ was added thereto, then 7.73 kg of hydrogen peroxide water containing 30 mass% as $H_2O_2$ was added. The resultant mixture was stirred and mixed for 1 hour at 50°C, and then the aqueous solution B-1 was added. Further, a solution in which 14.1 kg of fumed silica was dispersed in 198.0 kg of water and 0.535 kg of boric acid [$H_3BO_3$] were sequentially added to the mixture, which was then stirred for 2.5 hours at 50°C to obtain a raw-material blend solution. A composite oxide catalyst was obtained by carrying out the subsequent steps in the same manner as in Example 1.

(Propane ammoxidation reaction)

35 g of the obtained composite oxide catalyst was packed into a glass fluidized bed reactor having an inner diameter 1B. Then, with the same flow rates of raw-material gasses per catalyst as in Example 10, and the other conditions also the same, the reaction was started.

Subsequently, the respective gas amounts and temperatures were adjusted so that at a reactor temperature of 440°C and a reaction pressure of 50 kPa, propane, ammonia, and air were supplied at a molar ratio of propane:ammonia:air = 1:1:15 for a contact time of 2.9 sec·g/cc. One day after the reaction started, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 5 were measured. The measurement results of the respective physical property values and the AN yield at this point were as shown in Table 5. The molar ratio of air/propane introduced into the reactor was decreased by 2, and operation was continued. One day after the change of conditions, the catalyst was similarly extracted from the reactor, and the physical property values were measured. The results were as shown in Table 5.

[Example 22]

A silica-supported catalyst represented by the composition formula $Mo_1V_{0.214}Nb_{0.105}Sb_{0.220}Al_{0.01}$/50 mass%-$SiO_2$ was produced as follows.

(Preparation of raw-material blend solution)

34.4 kg of ammonium heptamolybdate [$(NH_4)_6Mo_7O_{24}·4H_2O$], 4.54 kg of ammonium metavanadate [$NH_4VO_3$], and 6.70 kg of antimony trioxide [$Sb_2O_3$] were added to 140.8 kg of water, and the resultant mixture was heated for 1 hour at 95°C under stirring to obtain an aqueous mixed solution A-1.

To 30.6 kg of the above-described niobium raw-material solution, 4.11 kg of hydrogen peroxide water containing 30 mass% as $H_2O_2$ was added, and the resultant mixture was stirred and mixed while maintaining the temperature at about 20°C to obtain an aqueous solution B-1.

The aqueous mixed solution A-1 was cooled to 70°C, 66.96 kg of silica sol containing 34.0 mass% as $SiO_2$ was added thereto, then 7.78 kg of hydrogen peroxide water containing 30 mass% as $H_2O_2$ was added. The resultant mixture was stirred and mixed for 1 hour at 50°C, and then the aqueous solution B-1 was added. Further, a solution in which 14.15 kg of fumed silica was dispersed in 198.0 kg of water and 0.088 kg of aluminum oxide [$Al_2O_3$] were sequentially added to the mixture, which was then stirred for 2.5 hours at 50°C to obtain a raw-material blend solution. A composite oxide catalyst was obtained by carrying out the subsequent steps in the same manner as in Example 1.

(Propane ammoxidation reaction)

35 g of the obtained composite oxide catalyst was packed into a glass fluidized bed reactor having an inner diameter 1B. Then, with the same flow rates of raw-material gasses per catalyst as in Example 10, and the other conditions also the same, the reaction was started.

Subsequently, the respective gas amounts and temperatures were adjusted so that at a reactor temperature of 440°C and a reaction pressure of 50 kPa, propane, ammonia, and air were supplied at a molar ratio of propane:ammonia:air = 1:1:15 for a contact time of 2.9 sec·g/cc. One day after the reaction started, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 5 were measured. The measurement results of the respective physical property values and the AN yield at this point were as shown in Table 5. The molar ratio of air/propane introduced into the reactor was decreased by 2, and operation was continued. One day after the change of conditions, the catalyst was similarly extracted from the reactor, and the physical property values were measured. The results were as shown in Table 5.

[Example 23]

A silica-supported catalyst represented by the composition formula $Mo_1V_{0.214}Nb_{0.105}Sb_{0.220}Ti_{0.008}$/50 mass%-$SiO_2$ was produced as follows.

(Preparation of raw-material blend solution)

34.3 kg of ammonium heptamolybdate [$(NH_4)_6Mo_7O_{24}·4H_2O$], 4.54 kg of ammonium metavanadate [$NH_4VO_3$],

and 6.69 kg of antimony trioxide [$Sb_2O_3$] were added to 140.7 kg of water, and the resultant mixture was heated for 1 hour at 95°C under stirring to obtain an aqueous mixed solution A-1.

To 30.6 kg of the above-described niobium raw-material solution, 4.11 kg of hydrogen peroxide water containing 30 mass% as $H_2O_2$ was added, and the resultant mixture was stirred and mixed while maintaining the temperature at about 20°C to obtain an aqueous solution B-1.

The aqueous mixed solution A-1 was cooled to 70°C, 66.96 kg of silica sol containing 34.0 mass% as $SiO_2$ was added thereto, then 7.77 kg of hydrogen peroxide water containing 30 mass% as $H_2O_2$ was added. The resultant mixture was stirred and mixed for 1 hour at 50°C, and then the aqueous solution B-1 was added. Further, a solution in which 14.15 kg of fumed silica was dispersed in 198.0 kg of water and 0.110 kg of titanium oxide [$TiO_2$] were sequentially added to the mixture, which was then stirred for 2.5 hours at 50°C to obtain a raw-material blend solution. A composite oxide catalyst was obtained by carrying out the subsequent steps in the same manner as in Example 1.

(Propane ammoxidation reaction)

35 g of the obtained composite oxide catalyst was packed into a glass fluidized bed reactor having an inner diameter 1B. Then, with the same flow rates of raw-material gasses per catalyst as in Example 10, and the other conditions also the same, the reaction was started.

Subsequently, the respective gas amounts and temperatures were adjusted so that at a reactor temperature of 440°C and a reaction pressure of 50 kPa, propane, ammonia, and air were supplied at a molar ratio of propane:ammonia:air = 1:1:15 for a contact time of 2.9 sec·g/cc. One day after the reaction started, 500 g of catalyst was extracted from the reactor. The catalyst particles were sieved using sieves having apertures of 32 μm and 100 μm, and the physical property values of the 32 to 100 μm catalyst particles shown in Table 5 were measured. The measurement results of the respective physical property values and the AN yield at this point were as shown in Table 5. The molar ratio of air/propane introduced into the reactor was decreased by 2, and operation was continued. One day after the change of conditions, the catalyst was similarly extracted from the reactor, and the physical property values and yield were measured. The results were as shown in Table 5.

[Example 24]

A silica-supported catalyst represented by the composition formula $Mo_1V_{0.214}Nb_{0.105}Sb_{0.220}Ta_{0.01}$/50 mass%-$SiO_2$ was produced as follows.

(Preparation of raw-material blend solution)

34.1 kg of ammonium heptamolybdate [$(NH_4)_6Mo_7O_{24}\cdot4H_2O$], 4.51 kg of ammonium metavanadate [$NH_4VO_3$], and 6.64 kg of antimony trioxide [$Sb_2O_3$] were added to 135.3 kg of water, and the resultant mixture was heated for 1 hour at 95°C under stirring to obtain an aqueous mixed solution A-1.

To 30.4 kg of the above-described niobium raw-material solution, 4.08 kg of hydrogen peroxide water containing 30 mass% as $H_2O_2$ was added, and the resultant mixture was stirred and mixed while maintaining the temperature at about 20°C to obtain an aqueous solution B-1.

The aqueous mixed solution A-1 was cooled to 70°C, 66.96 kg of silica sol containing 34.0 mass% as $SiO_2$ was added thereto, then 7.72 kg of hydrogen peroxide water containing 30 mass% as $H_2O_2$ was added. The resultant mixture was stirred and mixed for 1 hour at 50°C, and then the aqueous solution B-1 was added. Further, a solution in which 14.15 kg of fumed silica was dispersed in 198.0 kg of water and 0.432 kg of tantalic acid were sequentially added to the mixture, which was then stirred for 2.5 hours at 50°C to obtain a raw-material blend solution. A composite oxide catalyst was obtained by carrying out the subsequent steps in the same manner as in Example 1.

(Propane ammoxidation reaction)

35 g of the obtained composite oxide catalyst was packed into a glass fluidized bed reactor having an inner diameter 1B. Then, with the same flow rates of raw-material gasses per catalyst as in Example 10, and the other conditions also the same, the reaction was started.

Subsequently, the respective gas amounts and temperatures were adjusted so that at a reactor temperature of 440°C and a reaction pressure of 50 kPa, propane, ammonia, and air were supplied at a molar ratio of propane:ammonia:air = 1:1:15 for a contact time of 2.9 sec·g/cc. One day after the reaction started, 500 g of catalyst was extracted from the reactor. The catalyst particles were sieved using sieves having apertures of 32 μm and 100 μm, and the physical property values of the 32 to 100 μm catalyst particles shown in Table 5 were measured. The measurement results of the physical property values and the AN yield at this point were as shown in Table 5. The molar ratio of air/propane introduced into the reactor was decreased by 2, and operation was continued. One day after the change of conditions, the catalyst was similarly extracted from the reactor, and the physical property values and yield were measured. The results were as shown in Table 5.

[Example 25]

A silica-supported catalyst represented by the composition formula $Mo_1V_{0.214}Nb_{0.105}Sb_{0.22}Ce_{0.004}Bi_{0.02}$/50 mass%-$SiO_2$ was produced as follows.

(Preparation of raw-material blend solution)

33.7 kg of ammonium heptamolybdate $[(NH_4)_6Mo_7O_{24}\cdot4H_2O]$, 4.45 kg of ammonium metavanadate $[NH_4VO_2]$, 6.56 kg of antimony trioxide $[Sb_2O_3]$, and 0.299 kg of cerium nitrate $[Ce(NO_3)_3\cdot6H_2O]$ were added to 138.0 kg of water, and the resultant mixture was heated for 1 hour at 95°C under stirring to obtain an aqueous mixed solution A-1.
To 30.0 kg of the above-described niobium raw-material solution, 4.03 kg of hydrogen peroxide water containing 30 mass% as $H_2O_2$ was added, and the resultant mixture was stirred and mixed while maintaining the temperature at about 20°C to obtain an aqueous solution B-1.
The aqueous mixed solution A-1 was cooled to 70°C, 66.96 kg of silica sol containing 34.0 mass% as $SiO_2$ was added thereto, then 7.62 kg of hydrogen peroxide water containing 30 mass% as $H_2O_2$ was added. The resultant mixture was stirred and mixed for 1 hour at 50°C, and then the aqueous solution B-1 was added. Further, a solution in which 14.15 kg of fumed silica was dispersed in 198.0 kg of water and 1.346 kg of bismuth nitrate $[Bi(NO_3)_2\cdot6H_2O]$ were sequentially added to the mixture, which was then stirred for 2.5 hours at 50°C to obtain a raw-material blend solution. A composite oxide catalyst was obtained by carrying out the subsequent steps in the same manner as in Example 1.

(Propane ammoxidation reaction)

35 g of the obtained composite oxide catalyst was packed into a glass fluidized bed reactor having an inner diameter 1B. Then, with the same flow rates of raw-material gasses per catalyst as in Example 10, and the other conditions also the same, the reaction was started.
Subsequently, the respective gas amounts and temperatures were adjusted so that at a reactor temperature of 440°C and a reaction pressure of 50 kPa, propane, ammonia, and air were supplied at a molar ratio of propane:ammonia:air = 1:1:15 for a contact time of 2.9 sec·g/cc. One day after the reaction started, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 5 were measured. The measurement results of the respective physical property values and the AN yield at this point were as shown in Table 5. The molar ratio of air/propane introduced into the reactor was decreased by 2, and operation was continued. One day after the change of conditions, the catalyst was similarly extracted from the reactor, and the physical property values were measured. The results were as shown in Table 5.

[Example 26]

A silica-supported catalyst represented by the composition formula $Mo_1V_{0.214}Nb_{0.105}Sb_{0.220}Yb_{0.008}$/50 mass%-$SiO_2$ was produced as follows.

(Preparation of raw-material blend solution)

34.2 kg of ammonium heptamolybdate $[(NH_4)_6Mo_7O_{24}\cdot4H_2O]$, 4.52 kg of ammonium metavanadate $[NH_4VO_3]$, 6.67 kg of antimony trioxide $[Sb_2O_3]$, and 0.592 kg of ytterbium nitrate $[Yb(NO_3)_3\cdot4H_2O]$ were added to 162.8 kg of water, and the resultant mixture was heated for 1 hour at 95°C under stirring to obtain an aqueous mixed solution A-1.
To 30.5 kg of the above-described niobium raw-material solution, 4.09 kg of hydrogen peroxide water containing 30 mass% as $H_2O_2$ was added, and the resultant mixture was stirred and mixed while maintaining the temperature at about 20°C to obtain an aqueous solution B-1.
The aqueous mixed solution A-1 was cooled to 70°C, 67.4 kg of silica sol containing 34.0 mass% as $SiO_2$ was added, then 7.74 kg of hydrogen peroxide water containing 30 mass% as $H_2O_2$ was added. The resultant mixture was stirred and mixed for 1 hour at 50°C, and then the aqueous solution B-1 was added. Further, a solution in which 14.15 kg of fumed silica was dispersed in 198.0 kg of water was added to the mixture, which was then stirred for 2.5 hours at 50°C to obtain a raw-material blend solution. A composite oxide catalyst was obtained by carrying out the subsequent steps in the same manner as in Example 1.

(Propane ammoxidation reaction)

35 g of the obtained composite oxide catalyst was packed into a glass fluidized bed reactor having an inner diameter 1B. Then, with the same flow rates of raw-material gasses per catalyst as in Example 10, and the other conditions also the same, the reaction was started.

Subsequently, the respective gas amounts and temperatures were adjusted so that at a reactor temperature of 440°C and a reaction pressure of 50 kPa, propane, ammonia, and air were supplied at a molar ratio of propane:ammonia:air = 1:1:15 for a contact time of 2.9 sec·g/cc. One day after the reaction started, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 5 were measured. The measurement results of the respective physical property values and the AN yield at this point were as shown in Table 5. The molar ratio of air/propane introduced into the reactor was decreased by 2, and operation was continued. One day after the change of conditions, the catalyst was similarly extracted from the reactor, and the physical property values and yield were measured. The results were as shown in Table 5.

[Example 27]

A silica-supported catalyst represented by the composition formula $Mo_1V_{0.231}Nb_{0.105}Sb_{0.199}$/50 mass%-$SiO_2$ was produced as follows.

(Preparation of raw-material blend solution)

34.5 kg of ammonium heptamolybdate [$(NH_4)_6Mo_7O_{24}·4H_2O$], 5.27 kg of ammonium metavanadate [$NH_4VO_3$], and 6.09 kg of antimony trioxide [$Sb_2O_3$] were added to 189.8 kg of water, and the resultant mixture was heated for 1 hour at 95°C under stirring to obtain an aqueous mixed solution A-1.

To 30.7 kg of the above-described niobium raw-material solution, 4.12 kg of hydrogen peroxide water containing 30 mass% as $H_2O_2$ was added, and the resultant mixture was stirred and mixed while maintaining the temperature at about 20°C to obtain an aqueous solution B-1.

The aqueous mixed solution A-1 was cooled to 70°C, 67.4 kg of silica sol containing 34.0 mass% as $SiO_2$ was added thereto, then 7.07 kg of hydrogen peroxide water containing 30 mass% as $H_2O_2$ was added. The resultant mixture was stirred and mixed for 1 hour at 50°C, and then the aqueous solution B-1 was added. Further, a solution in which 14.15 kg of fumed silica was dispersed in 198.0 kg of water was added to the mixture, which was then stirred for 2.5 hours at 50°C to obtain a raw-material blend solution. A composite oxide catalyst was obtained by carrying out the subsequent steps in the same manner as in Example 1.

(Propane ammoxidation reaction)

35 g of the obtained composite oxide catalyst was packed into a glass fluidized bed reactor having an inner diameter 1B. Then, with the same flow rates of raw-material gasses per catalyst as in Example 8, and the other conditions also the same, the reaction was started. Subsequently, the respective gas amounts and temperatures were adjusted so that at a reactor temperature of 440°C and a reaction pressure of 50 kPa, propane, ammonia, and air were supplied at a molar ratio of propane:ammonia:air = 1:1:15 for a contact time of 2.9 sec·g/**cm³**). One day after the reaction started, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 5 were measured. The measurement results of the respective physical property values and the AN yield at this point were as shown in Table 5. The molar ratio of air/propane introduced into the reactor was decreased by 2, and operation was continued. One day after the change of conditions, the catalyst was similarly extracted from the reactor, and the physical property values and yield were measured. The results were as shown in Table 5.

[Example 28]

A silica-supported catalyst represented by the composition formula $Mo_1V_{0.214}Nb_{0.105}Sb_{0.220}$/55 mass%-$SiO_2$ was produced as follows.

(Preparation of raw-material blend solution)

31.2 kg of ammonium heptamolybdate [$(NH_4)_6Mo_7O_{24}·4H_2O$], 4.13 kg of ammonium metavanadate [$NH_4VO_3$], and 6.09 kg of antimony trioxide [$Sb_2O_3$] were added to 148.7 kg of water, and the resultant mixture was heated for 1 hour at 95°C under stirring to obtain an aqueous mixed solution A-1.

To 27.8 kg of the above-described niobium raw-material solution, 3.74 kg of hydrogen peroxide water containing 30 mass% as $H_2O_2$ was added, and the resultant mixture was stirred and mixed while maintaining the temperature at about 20°C to obtain an aqueous solution B-1.

The aqueous mixed solution A-1 was cooled to 70°C, 69.7 kg of silica sol containing 34.0 mass% as $SiO_2$ was added thereto, then 7.07 kg of hydrogen peroxide water containing 30 mass% as $H_2O_2$ was added. The resultant mixture was stirred and mixed for 1 hour at 50°C, and then the aqueous solution B-1 was added. Further, a solution in which 17.21 kg of fumed silica was dispersed in 241.0 kg of water was added to the mixture, which was then stirred for 2.5 hours at 50°C to obtain a raw-material blend solution. A composite oxide catalyst was obtained by carrying out the subsequent steps in the same manner as in Example 1.

(Propane ammoxidation reaction)

35 g of the obtained composite oxide catalyst was packed into a glass fluidized bed reactor having an inner diameter 1B. Then, with the same flow rates of raw-material gasses per catalyst as in Example 10, and the other conditions also the same, the reaction was started.

Subsequently, the respective gas amounts and temperatures were adjusted so that at a reactor temperature of 440°C and a reaction pressure of 50 kPa, propane, ammonia, and air were supplied at a molar ratio of propane:ammonia:air = 1:1:15 for a contact time of 2.9 sec·g/cm³). One day after the reaction started, 500 g of catalyst was extracted from the reactor. The catalyst particles were sieved using sieves having apertures of 32 $\mu$m and 100 $\mu$m, and the physical property values of the 32 to 100 $\mu$m catalyst particles shown in Table 5 were measured. The measurement results of the respective physical property values and the AN yield at this point were as shown in Table 5. The molar ratio of air/propane introduced into the reactor was decreased by 2, and operation was continued. One day after the change of conditions, the catalyst was similarly extracted from the reactor, and the physical property values and yield were measured. The results were as shown in Table 5.

[Table 1]

| | Catalyst Extraction | Reaction Conditions | | | Physical Property Values 10 Days After Reaction Start | | | Reaction Conditions After Change | | | Physical Property Values 5 Days After Change | | | AN Yield (%) | |
|---|---|---|---|---|---|---|---|---|---|---|---|---|---|---|---|
| | | Gas Com-position (molar ra-tio) Pro-pane: Ammonia : Air | Tempera-ture °C | Cata-lyst Amount k g | Normal-ized UV Value | Reduc-tion ratio % | Mo Com-position | Gas Com-position (molar ra-tio) Pro-pane:Am-monia: Air | Tempera-ture °C | Cata-lyst Amount kg | Normal-ized UV Value | Reduc-tion ratio % | Mo Com-position | Before Change | 5 Days After Change |
| Example 1 | Yes | 1:1:16 | 440 | 580 | - | 8.3 | 0.98 | 1:1:15 | 440 | 580 | - | 8.5 | 0.98 | 52.7 | 53.2 |
| Example 2 | Yes | 1:1:14 | 440 | 580 | - | 8.8 | 0.98 | 1:1:14.5 | 440 | 580 | - | 8.6 | 0.98 | 52.3 | 53.3 |
| Example 3 | Yes | 1:0.8:15 | 440 | 580 | - | 8.2 | - | 1:0.95:15 | 440 | 580 | - | 8.5 | - | 52.2 | 53.1 |
| Example 4 | Yes | 1:0.9:15 | 440 | 580 | - | 8.8 | - | 1:0.9:15 | 440 | 580 | - | 8.4 | - | 52 | 52.9 |
| Example 5 | Yes | 1:1:15 | 440 | 580 | 0.73 | - | 0.97 | 1:1.08:15 | 440 | 580 | 0.76 | - | 0.97 | 52.4 | 52.9 |
| Example 6 | Yes | 1:1:13 | 440 | 580 | - | 8.9 | 0.95 | 1:1:14 | 442 | 580 | - | 8.6 | 0.97 | 52.3 | 53.3 |
| Example 7 | Yes | 1:1.05:13.5 | 440 | 560 | 0.79 | - | 0.97 | 1:1.05:14 | 440 | 600 | 0.76 | - | 0.98 | 52.4 | 53.0 |
| Compara-tive Exam-ple 1 | Yes | 1:0.9:15 | 440 | 580 | - | - | 0.97 | 1:0.9:15 | 440 | 580 | - | - | 0.98 | 52.5 | 52.5 |

[Table 2]

| | Catalyst Extraction | Physical Property Values Immediately After Reaction Start | | | Reaction Conditions After Change | | Physical Property Values 5 Days After Change | | | AN Yield (%) | |
|---|---|---|---|---|---|---|---|---|---|---|---|
| | | Normalized UV Value | Reduction ratio % | Mo Composi-tion | Gas Composi-tion (molar ra-tio) Propane: Ammonia: Air | Temperature °C | Normalized UV Value | Reduction ratio % | Mo Composition | Before Change | 5 Days After Change |
| Example 8 | Yes | 1 | 11.5 | - | 1:1:17 | 445 | 0.9 | 10.2 | - | 49.1 | 50.3 |
| Example 9 | Yes | 0.5 | 5.6 | - | 1:1:12 | 440 | 0.6 | 6.8 | - | 48.9 | 49.8 |
| Example 10 | Yes | - | 8.5 | - | 1:1:15 | 440 | - | 8.5 | - | 53.1 | 53.1 |
| Comparative Example 2 | No | - | - | - | | - | - | - | - | | 47.5(*) |
| Comparative Example 3 | Yes | 0.71 | 8 | 0.98 | 1:1:17 | 440 | 0.7 | 7.7 | 0.98 | 52.4 | 48.8 |
| Example 11 | Yes | 0.7 | 7.9 | 0.98 | 1:1:14.5 | 440 | 0.73 | 8.3 | 0.98 | 51.8 | 52.7 |
| Example 12 | Yes | 0.81 | 8.8 | - | 1:0.095:15 | 440 | 0.77 | 8.7 | - | 52.2 | 52.6 |

* Comparative Example 2 was the AN yield 5 days after the reaction started.

[Table 3]

| | Catalyst Extraction | Physical Property Values at Reaction Start and During Reaction | | | Physical Property Values 1 Day After Reaction Start | | | AN Yield 1 Day After Reaction Start % |
|---|---|---|---|---|---|---|---|---|
| | | Normalized UV Value | Reduction ratio % | Mo Composition | Normalized UV Value | Reduction ratio % | Mo Composition | |
| Example 13 | Yes | 0.65 | 7.3 | 0.98 | 0.73 | 8.3 | 0.98 | 52.9 |
| Example 14 | Yes | 0.9 | 10.2 | 0.98 | 0.78 | 8.4 | 0.98 | 53 |
| Example 15 | Yes | - | 9.6 | 0.98 | - | 8.6 | 0.98 | 53.1 |
| Comparative Example 4 | No | | | | | | | 52.2 |
| Comparative Example 5 | No | | | | | | | 52.1 |
| Comparative Example 6 | No | | | | | | | 52.3 |
| Comparative Example 7 | No | | | | | | | 52.5 |

[Table 4]

| | Catalyst Extraction | Physical Property Values When Reaction Terminated | | | Physical Property Values 10 Days After Reaction Re-Start | | | AN Yield 10 Days After Reaction Re-Start |
|---|---|---|---|---|---|---|---|---|
| | | Normalized UV Value | Reduction ratio % | Mo Composition | Normalized UV Value | Reduction ratio % | Mo Composition | |
| Example 16 | Yes | 0.84 | 9.4 | - | 0.77 | 8.6 | - | 53 |
| Example 17 | Yes | 0.67 | 7.5 | - | 0.73 | 8.2 | - | 53.1 |
| Comparative Example 8 | No | | | | | | | 52.1 |
| Comparative Example 9 | No | | | | | | | 51.9 |

[Table 5]

| | Catalyst Extraction | Physical Property Values | | Physical Property Values 1 Day After Change | | AN Yield (%) | | Catalyst Composition |
|---|---|---|---|---|---|---|---|---|
| | | Normalized UV Value | Reduction ratio % | Normalized UV Value | Reduction ratio % | Before Change | 5 Days After Change | |
| Example 18 | Yes | - | 8.4 | - | 8.5 | 52.6 | 53.4 | Mo1.0V0.214Sb0.220Nb0.105W0.030Ce0.005On/50.0 mass%-SiO2 |
| Example 19 | Yes | - | 8.5 | - | 8.6 | 51.7 | 52.7 | Mo1.0V0.214Sb0.220Nb0.105/50 mass%-SiO2 |
| Example 20 | Yes | - | 8.4 | - | 8.5 | 52.1 | 52.9 | Mo1.0V0.214Sb0.220Nb0.105W0.03Mn0.002/50 mass%-SiO2 |
| Example 21 | Yes | 0.72 | 8.1 | 0.73 | 8.2 | 52.5 | 53 | Mo1.0V0.214Sb0.220Nb0.105B0.05/50 mass%-SiO2 |
| Example 22 | Yes | - | 8.4 | - | 8.5 | 51.7 | 52.4 | Mo1.0V0.214Sb0.220Nb0.105Al0.01/50 mass%-SiO2 |
| Example 23 | Yes | - | 8.2 | - | 8.4 | 52 | 52.6 | Mo1.0V0.214Sb0.220Nb0.105W0.03Ti0.008/50 mass%-SiO2 |
| Example 24 | Yes | - | 8.5 | - | 8.6 | 51.8 | 52.6 | Mo1.0V0.214Sb0.220Nb0.105Ta0.01/50 mass%-SiO2 |
| Example 25 | Yes | - | 8.4 | - | 8.5 | 52 | 52.8 | Mo1.0V0.214Sb0.220Nb0.105Ce0.004Bi0.02/50 mass%-SiO2 |
| Example 26 | Yes | - | 8.5 | - | 8.6 | 52.1 | 52.9 | Mo1.0V0.214Sb0.220Nb0.105Yb0.008/50 mass%-SiO2 |
| Example 27 | Yes | 0.76 | 8.59 | 0.77 | 8.7 | 51.5 | 52.4 | Mo1.0V0.231Sb0.199Nb0.105/50 mass%-SiO2 |
| Example 28 | Yes | - | 8.36 | - | 8.5 | 52.8 | 53.4 | Mo1.0V0.214Sb0.220Nb0.105/55 mass%-SiO2 |

49

Industrial Applicability

According to the production method of the present invention, the yield of acrylonitrile can be maintained.

**Claims**

1. A method for producing acrylonitrile by a propane ammoxidation reaction, using a composite oxide catalyst represented by the following formula:

$$Mo_1V_aNb_bA_cX_dZ_eO_n$$

wherein component A represents Sb; component X represents at least one element selected from the group consisting of W, Bi and Mn; component Z represents at least one element selected from the group consisting of La, Ce, Pr, Yb, Y, Sc, Sr, and Ba; a, b, c, d, e, and n each represent an atomic ratio of the corresponding element per Mo atom; a is in the range of $0.01 \leqq a \leqq 1$; b is in the range of $0.01 \leqq b \leqq 1$; c is in the range of $0.01 \leqq c \leqq 1$; d is in the range of $0 \leqq d \leqq 1$; e is in the range of $0 \leqq e \leqq 1$; and n represents the number determined by a valence of component elements,
said method comprising:

(1) a step of extracting a part of the catalyst from the reactor in a non-steady state of the ammoxidation reaction, wherein a steady-state refers to a state in which, when no change has been made to the temperature of the catalyst layer in the reactor or the composition of the raw material gas supplied to the reactor for 1 or more days, temperature fluctuations of the catalyst layer in the reactor over 1 day are no greater than 10 °C;
(2) a step of measuring at least one catalyst physical property value selected from the group consisting of the normalized UV value of the catalyst and the reduction ratio of the catalyst contained in a reactor; and
(3) a step of maintaining or changing a reaction condition based on the measured physical property value so that fluctuation in the physical property value of the catalyst is maintained within $\pm$ 30% of the physical property value of the catalyst prior to packing into the reactor,
wherein in the step (3) of maintaining or changing the reaction condition comprises performing at least one selected from the group consisting of maintaining or changing of the temperature of the catalyst layer in the reactor, and maintaining or changing the composition of raw material gases supplied to the reactor, and maintaining or changing a contact time between the catalyst and the raw material gases, and
wherein the normalized UV value acts as an index of the catalyst redox state which is determined based on the absorbance at 400 nm, 580 nm, and 700 nm of the absorption and/or reflection spectrum obtained by measuring by a diffuse reflection method using a visible-ultraviolet spectrophotometer and calculated based on the following equation (1):

$$\text{Normalized UV value} = \{(580 \text{ nm absorbance}) - (400 \text{ nm absorbance})\} / \{(700 \text{ nm absorbance}) - (400 \text{ nm absorbance})\} \qquad (1)$$

and
wherein the reduction ratio of the catalyst is represented by the following equation (2):

$$\text{Reduction ratio (\%)} = ((n_0 - n) / n_0 \times 100 \qquad (2)$$

wherein n denotes the number of oxygen atoms satisfying the valence of the constituent elements other than oxygen in the catalyst; and no denotes the number of oxygen atoms required when the constituent elements other than oxygen in the catalyst each have their maximum oxidation number.

2. The method for producing acrylonitrile according to claim 1, wherein in the step of maintaining or changing the reaction condition, so that an oxygen concentration in a production gas at an outlet of the reactor during the reaction when the ammoxidation reaction is in a steady state is at a target concentration set to be between 1.5 and 6.0 vol.%, at least one condition selected from the group consisting of (1) a molar ratio of oxygen to propane in the raw material gases, (2) a temperature of the reactor, and (3) a contact time between the catalyst and the raw material gases is adjusted, and

when adjusting the condition (1) and/or (3), change in the temperature of the reactor is set to be within ±5 °C of the temperature prior to adjusting the condition, and when adjusting the condition (2), change in the temperature of the reactor is set to be within ±5 °C of a target temperature.

3. The method for producing acrylonitrile according to claim 1, wherein in the step of maintaining or changing the reaction condition, so that an outlet ammonia concentration calculated based on a propane concentration in a raw material gases when the ammoxidation reaction is in a steady state is at a target concentration set to be more than 0 vol.% to 18 vol.% or less depending on change in an outlet ammonia amount obtained by measuring the outlet ammonia amount of the reactor, at least one condition selected from the group consisting of (1) a molar ratio of ammonia to propane in the raw material gases, (2) a temperature of the reactor, and (3) a contact time between the catalyst and the raw material gases is adjusted, and

when adjusting the condition (1) and/or (3), change in the temperature of the reactor is set to be within ±5 °C of the temperature prior to adjusting the condition, and when adjusting the condition (2), change in the temperature of the reactor is set to be within ±5 °C of a target temperature.

4. The method for producing acrylonitrile according to claim 2, wherein when the oxygen concentration is higher than the target concentration, the molar ratio of oxygen to propane in the raw material gases is decreased, and when the oxygen concentration is lower than the target concentration, the molar ratio of oxygen to propane in the raw material gases is increased.

5. The method for producing acrylonitrile according to claim 4, wherein the rate of increasing or decreasing the oxygen amount in the raw material gases is 10 % or less based on the oxygen amount included in the raw material gases per minute.

6. The method for producing acrylonitrile according to claim 3, wherein when the outlet ammonia concentration is higher than the target concentration, the molar ratio of ammonia to propane in the raw material gases is decreased, and when the outlet ammonia concentration is lower than the target concentration, the molar ratio of ammonia to propane in the raw material gases is increased.

7. The method for producing acrylonitrile according to claim 6, wherein the rate of increasing or decreasing the ammonia amount in the raw material gases is 15% or less based on the ammonia amount included in the raw material gases per minute.

8. The method for producing acrylonitrile according to any one of claims 2 to 7, wherein the rate of change in the temperature of the reactor is 10°C or less per hour.

9. The method for producing acrylonitrile according to any one of claims 2 to 8, wherein the rate of change in the contact time between the composite oxide catalyst and the raw material gases is 1.0 sec or less per hour.

**Patentansprüche**

1. Verfahren zur Herstellung von Acrylnitril durch eine Propan-Ammoxidationsreaktion unter Verwendung eines Mischoxidkatalysators, der durch die folgende Formel dargestellt wird:

$$Mo_1V_aNb_bA_cX_dZ_eO_n,$$

wobei Komponente A für Sb steht, Komponente X für wenigstens ein Element steht, das aus der Gruppe ausgewählt ist, die aus W, Bi und Mn besteht, Komponente Z für wenigstens ein Element steht, das aus der Gruppe ausgewählt ist, die aus La, Ce, Pr, Yb, Y, Sc, Sr und Ba besteht, a, b, c, d, e und n jeweils für ein Atomverhältnis des jeweiligen Elements auf der Basis eines Mo-Atoms stehen, a in einem Bereich von $0{,}01 \leq a \leq 1$ liegt, b in einem Bereich von $0{,}01 \leq b \leq 1$ liegt, c in einem Bereich von $0{,}01 \leq c \leq 1$ liegt, d in einem Bereich von $0 \leq d \leq 1$ liegt, e in einem Bereich von $0 \leq e \leq 1$ liegt und n für eine Zahl steht, die von der Wertigkeit der Komponentenelemente bestimmt wird;
wobei das Verfahren umfasst:

(1) einen Schritt des Extrahierens eines Teils des Katalysators aus dem Reaktor in einem nichtstationären Zustand der Ammoxidationsreaktion;

wobei sich "stationärer Zustand" auf einen Zustand bezieht, in dem, wenn über 1 oder mehr Tage keine Veränderung an der Temperatur der Katalysatorschicht in dem Reaktor oder der Zusammensetzung des dem Reaktor zugeführten Rohstoffgases vorgenommen wurde, die Temperaturfluktuationen der Katalysatorschicht in dem Reaktor über 1 Tag hinweg nicht größer als 10 °C sind;

(2) einen Schritt des Messens wenigstens eines Werts einer physikalischen Eigenschaft des Katalysators, der aus der Gruppe ausgewählt ist, die aus dem normierten UV-Wert des Katalysators und dem Reduktionsverhältnis des in einem Reaktor enthaltenen Katalysators besteht; und

(3) einen Schritt des Aufrechterhaltens oder Veränderns einer Reaktionsbedingung auf der Basis des gemessenen Werts der physikalischen Eigenschaft, so dass eine Fluktuation in dem Wert der physikalischen Eigenschaft des Katalysators innerhalb von ±30% des Werts der physikalischen Eigenschaft des Katalysators vor dem Befüllen des Reaktors gehalten wird;

wobei Schritt (3) des Aufrechterhaltens oder Veränderns der Reaktionsbedingung das Durchführen wenigstens einer Maßnahme umfasst, die aus der Gruppe ausgewählt ist, die aus dem Aufrechterhalten oder Verändern der Temperatur der Katalysatorschicht in dem Reaktor und dem Aufrechterhalten oder Verändern der Zusammensetzung von dem Reaktor zugeführten Rohstoffgasen und dem Aufrechterhalten oder Verändern der Kontaktzeit zwischen dem Katalysator und den Rohstoffgasen besteht; und

wobei der normierte UV-Wert als Index des Katalysatorredoxzustands wirkt, der auf der Basis der Extinktion bei 400 nm, 580 nm und 700 nm im Absorptions- und/oder Reflexionsspektrum bestimmt wird, das man dadurch erhält, dass man durch ein Verfahren der diffusen Reflektion mit Hilfe eines Sichtbar-UV-Spektrophotometers misst, und auf der Basis der folgenden Gleichung (1) berechnet wird:

$$\text{normierter UV-Wert} = \{(\text{580-nm-Extinktion}) - (\text{400-nm-Extinktion})\}/\{(\text{700-nm-Extinktion}) - (\text{400-nm-Extinktion})\} \qquad (1)$$

und wobei das Reduktionsverhältnis des Katalysators durch die folgende Gleichung (2) dargestellt wird:

$$\text{Reduktionsverhältnis (\%)} = (n_0 - n)/n_0) \times 100 \qquad (2),$$

wobei n die Anzahl der Sauerstoffatome bezeichnet, die die Wertigkeit der anderen konstituierenden Elemente als Sauerstoff im Katalysator absättigen, und $n_0$ die Anzahl der Sauerstoffatome bezeichnet, die erforderlich sind, wenn die anderen konstituierenden Elemente als Sauerstoff im Katalysator jeweils ihre maximale Oxidationszahl aufweisen.

2. Verfahren zur Herstellung von Acrylnitril gemäß Anspruch 1, wobei im Schritt des Aufrechterhaltens oder Veränderns der Reaktionsbedingung, so dass die Sauerstoffkonzentration im Produktionsgas an einem Auslass des Reaktors während der Reaktion, wenn sich die Ammoxidationsreaktion in einem stationären Zustand befindet, bei einer Zielkonzentration liegt, die auf 1,5 bis 6,0 Vol.-% eingestellt wurde, wenigstens ein Zustand, der aus der Gruppe ausgewählt wird, die aus (1) dem Stoffmengenverhältnis von Sauerstoff zu Propan in den Rohstoffgasen, (2) der Temperatur des Reaktors und (3) der Kontaktzeit zwischen dem Katalysator und den Rohstoffgasen besteht, eingestellt wird; und

beim Einstellen des Zustands (1) und/oder (3) die Änderung der Temperatur des Reaktors so eingestellt wird, dass sie innerhalb von ± 5°C der Temperatur vor dem Einstellen des Zustands liegt, und beim Einstellen des Zustands (2) die Änderung der Temperatur des Reaktors so eingestellt wird, dass sie innerhalb von ± 5 °C einer Zieltemperatur liegt.

3. Verfahren zur Herstellung von Acrylnitril gemäß Anspruch 1, wobei im Schritt des Aufrechterhaltens oder Veränderns der Reaktionsbedingung, so dass die Auslassammoniakkonzentration, die auf der Basis der Propankonzentration in Rohstoffgasen berechnet wird, wenn sich die Ammoxidationsreaktion in einem stationären Zustand befindet, bei einer Zielkonzentration, die je nach Veränderung der Auslassammoniakmenge, die man durch Messen der Auslassammoniakmenge des Reaktors erhält, auf mehr als 0 Vol.-% bis 18 Vol.-% oder weniger eingestellt wurde, liegt, wenigstens eine Bedingung, die aus der Gruppe ausgewählt ist, die aus (1) dem Stoffmengenverhältnis von Sauerstoff zu Propan in den Rohstoffgasen, (2) der Temperatur des Reaktors und (3) der Kontaktzeit zwischen dem Katalysator und den Rohstoffgasen besteht, eingestellt wird; und

beim Einstellen des Zustands (1) und/oder (3) die Änderung der Temperatur des Reaktors so eingestellt wird, dass sie innerhalb von ± 5 °C der Temperatur vor dem Einstellen des Zustands liegt, und beim Einstellen des Zustands (2) die Änderung der Temperatur des Reaktors so eingestellt wird, dass sie innerhalb von ± 5 °C einer Zieltemperatur liegt.

4. Verfahren zur Herstellung von Acrylnitril gemäß Anspruch 2, wobei, wenn die Sauerstoffkonzentration höher ist als die Zielkonzentration, das Stoffmengenverhältnis von Sauerstoff zu Propan in den Rohstoffgasen gesenkt wird und, wenn die Sauerstoffkonzentration kleiner ist als die Zielkonzentration, das Stoffmengenverhältnis von Sauerstoff zu Propan in den Rohstoffgasen erhöht wird.

5. Verfahren zur Herstellung von Acrylnitril gemäß Anspruch 4, wobei die Geschwindigkeit der Zunahme oder Abnahme der Sauerstoffmenge in den Rohstoffgasen 10% oder weniger beträgt, bezogen auf die Sauerstoffmenge, die in den Rohstoffgasen pro Minute enthalten ist.

6. Verfahren zur Herstellung von Acrylnitril gemäß Anspruch 3, wobei, wenn die Auslassammoniakkonzentration höher ist als die Zielkonzentration, das Stoffmengenverhältnis von Ammoniak zu Propan in den Rohstoffgasen gesenkt wird und, wenn die Auslassammoniakkonzentration kleiner ist als die Zielkonzentration, das Stoffmengenverhältnis von Ammoniak zu Propan in den Rohstoffgasen erhöht wird.

7. Verfahren zur Herstellung von Acrylnitril gemäß Anspruch 6, wobei die Geschwindigkeit der Zunahme oder Abnahme der Ammoniakmenge in den Rohstoffgasen 15% oder weniger beträgt, bezogen auf die Ammoniakmenge, die in den Rohstoffgasen pro Minute enthalten ist.

8. Verfahren zur Herstellung von Acrylnitril gemäß einem der Ansprüche 2 bis 7, wobei die Geschwindigkeit der Veränderung der Temperatur des Reaktors 10 °C oder weniger pro Stunde beträgt.

9. Verfahren zur Herstellung von Acrylnitril gemäß einem der Ansprüche 2 bis 8, wobei die Geschwindigkeit der Veränderung der Kontaktzeit zwischen dem Mischoxidkatalysator und den Rohstoffgasen 1,0 s oder weniger pro Stunde beträgt.

**Revendications**

1. Procédé pour produire de l'acrylonitrile par une réaction d'ammoxydation du propane, utilisant un catalyseur de type oxyde composite représenté par la formule suivante :

$$Mo_1V_aNb_bA_cX_dZ_eO_n$$

dans laquelle le composant A représente Sb ; le composant X représente au moins un élément choisi dans l'ensemble constitué par W, Bi et Mn ; le composant Z représente au moins un élément choisi dans l'ensemble constitué par La, Ce, Pr, Yb, Y, Sc, Sr, et Ba ; chacun de a, b, c, d, e et n représente le rapport atomique de l'élément correspondant par atome Mo ; a est situé dans la plage $0,01 \leq a \leq 1$ ; b est situé dans la plage $0,01 \leq b \leq 1$ ; c est situé dans la plage $0,01 \leq c \leq 1$ ; d est situé dans la plage $0 \leq d \leq 1$ ; e est situé dans la plage $0 \leq e \leq 1$ ; et n représente le nombre déterminé par la valence des éléments constitutifs,
ledit procédé comprenant :

(1) une étape d'extraction d'une partie du catalyseur hors du réacteur dans un état non à l'équilibre de la réaction d'ammoxydation,
dans laquelle un état d'équilibre se réfère à un état dans lequel, quand aucun changement n'a été apporté à la température de la couche de catalyseur dans le réacteur ou à la composition du gaz matière première fourni au réacteur pendant 1 jour ou plus, les fluctuations de température de la couche de catalyseur dans le réacteur sur 1 jour ne sont pas supérieures à 10°C ;
(2) une étape de mesure d'au moins une valeur de propriété physique du catalyseur, choisie dans l'ensemble constitué par la valeur UV normalisée du catalyseur et le taux de réduction du catalyseur contenu dans le réacteur ; et
(3) une étape de maintien ou de changement d'une condition réactionnelle sur la base de la valeur de propriété physique mesurée de façon qu'une fluctuation de la valeur de propriété physique du catalyseur soit maintenue dans les ± 30 % de la valeur de propriété physique du catalyseur avant tassement dans le réacteur,
dans lequel l'étape (3) de maintien ou de changement de la condition réactionnelle comprend la mise en oeuvre d'au moins l'un choisi dans l'ensemble constitué par le maintien ou le changement de la température de la couche de catalyseur dans le réacteur, et le maintien ou le changement de la composition des gaz matières premières fournis au réacteur, et le maintien ou le changement du temps de contact entre le catalyseur et les gaz matières premières, et

dans lequel la valeur UV normalisée agit comme un indice de l'état redox du catalyseur qui est déterminé sur la base de l'absorbance à 400 nm, 580 nm et 700 nm du spectre d'absorption et/ou de réflexion obtenu par mesure au moyen d'un procédé de réflexion diffuse utilisant un spectrophotomètre visible-ultraviolet, et calculé sur la base de l'équation (1) suivante :

$$\text{Valeur UV normalisée} = \{(\text{absorbance à 580 nm}) - (\text{absorbance à 400 nm})\} / \{(\text{absorbance à 700 nm}) - (\text{absorbance à 400 nm})\} \qquad (1)$$

et dans lequel le taux de réduction du catalyseur est représenté par l'équation (2) suivante :

$$\text{Taux de réduction (\%)} = ((n_0 - n) / n_0) \times 100 \qquad (2)$$

où $n$ représente le nombre d'atomes d'oxygène satisfaisant à la valence des éléments constitutifs autres que l'oxygène dans le catalyseur ; et $n_0$ représente le nombre d'atomes d'oxygène requis quand chacun des éléments constitutifs autres que l'oxygène dans le catalyseur a son nombre d'oxydation maximal.

**2.** Procédé pour produire de l'acrylonitrile selon la revendication 1, dans lequel, dans l'étape de maintien ou de changement de la condition réactionnelle, de façon que la concentration d'oxygène dans le gaz de production à la sortie du réacteur durant la réaction quand la réaction d'ammoxydation est dans un état d'équilibre soit à une concentration cible établie de façon à être comprise entre 1,5 et 6,0 % en volume, au moins une condition choisie dans l'ensemble constitué par (1) le rapport molaire de l'oxygène au propane dans les gaz matières premières, (2) la température du réacteur, et (3) le temps de contact entre le catalyseur et les gaz matières premières, est ajustée, et

lors de l'ajustement des conditions (1) et/ou (3), le changement de température du réacteur est établi de façon à être de ± 5°C de la température avant l'ajustement de la condition, et lors de l'ajustement de la condition (2), le changement de température du réacteur est établi de façon à être de ± 5°C d'une température cible.

**3.** Procédé pour produire de l'acrylonitrile selon la revendication 1, dans lequel, dans l'étape de maintien ou de changement de la condition réactionnelle, de façon que la concentration d'ammoniac en sortie, calculée sur la base de la concentration de propane dans les gaz matières premières quand la réaction d'ammoxydation est dans un état d'équilibre, soit à une concentration cible établie de façon à être de plus de 0 % en volume à 18 % en volume ou moins en fonction d'un changement de la quantité d'ammoniac en sortie obtenue par mesure de la quantité d'ammoniac en sortie du réacteur, au moins une condition choisie dans l'ensemble constitué par (1) le rapport molaire de l'ammoniac au propane dans les gaz matières premières, (2) la température du réacteur, et (3) le temps de contact entre le catalyseur et les gaz matières premières, est ajustée, et

lors de l'ajustement des conditions (1) et/ou (3), le changement de température du réacteur est établi de façon à être de ± 5°C de la température avant l'ajustement de la condition, et lors de l'ajustement de la condition (2), le changement de température du réacteur est établi de façon à être de ± 5°C d'une température cible.

**4.** Procédé pour produire de l'acrylonitrile selon la revendication 2, dans lequel, lorsque la concentration d'oxygène est supérieure à la concentration cible, le rapport molaire de l'oxygène au propane dans les gaz matières premières est réduit, et lorsque la concentration d'oxygène est inférieure à la concentration cible, le rapport molaire de l'oxygène au propane dans les gaz matières premières est augmenté.

**5.** Procédé pour produire de l'acrylonitrile selon la revendication 4, dans lequel la vitesse d'augmentation ou de réduction de la quantité d'oxygène dans les gaz matières premières est de 10 % ou moins sur la base de la quantité d'oxygène se trouvant dans les gaz matières premières par minute.

**6.** Procédé pour produire de l'acrylonitrile selon la revendication 3, dans lequel, lorsque la concentration d'ammoniac en sortie est supérieure à la concentration cible, le rapport molaire de l'ammoniac au propane dans les gaz matières premières est réduit, et lorsque la concentration d'ammoniac en sortie est inférieure à la concentration cible, le rapport molaire de l'ammoniac au propane dans les gaz matières premières est augmenté.

7. Procédé pour produire de l'acrylonitrile selon la revendication 6, dans lequel la vitesse d'augmentation ou de réduction de la quantité d'ammoniac dans les gaz matières premières est de 15 % ou moins sur la base de la quantité d'ammoniac se trouvant dans les gaz matières premières par minute.

8. Procédé pour produire de l'acrylonitrile selon l'une quelconque des revendications 2 à 7, dans lequel la vitesse de changement de la température du réacteur est de 10°C ou moins par heure.

9. Procédé pour produire de l'acrylonitrile selon l'une quelconque des revendications 2 à 8, dans lequel la vitesse de changement du temps de contact entre le catalyseur de type oxyde composite et les gaz matières premières est de 1,0 s ou moins par heure.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060235238 A
- JP 2007308423 A
- JP 11244702 A